(19) Europäisches Patentamt
European Patent Office
Office européen des brevets

(11) EP 3 204 412 B1

(12) EUROPEAN PATENT SPECIFICATION

(45) Date of publication and mention of the grant of the patent:
**19.08.2020 Bulletin 2020/34**

(21) Application number: **15774630.6**

(22) Date of filing: **06.10.2015**

(51) Int Cl.:
***C07K 16/22*** *(2006.01)* ***C07K 16/28*** *(2006.01)*
***C07K 16/40*** *(2006.01)*

(86) International application number:
**PCT/EP2015/072974**

(87) International publication number:
**WO 2016/055432 (14.04.2016 Gazette 2016/15)**

(54) **COMBINATION THERAPY OF BISPECIFIC ANTIBODIES SPECIFIC FOR FAP AND DR5 AND CHEMOTHERAPEUTIC AGENTS**

KOMBINATIONSTHERAPIE VON BISPEZIFISCHEN ANTIKÖRPERN SPEZIFISCH FÜR FAP UND DR5 UND CHEMOTHERAPEUTIKA

TRAITEMENT COMBINÉ D'ANTICORPS BISPÉCIFIQUES SPÉCIFIQUES DE FAP ET DR5 ET AGENTS CHIMIOTHÉRAPEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.10.2014 EP 14188176**
**15.01.2015 EP 15151221**
**23.03.2015 EP 15160231**
**30.07.2015 EP 15179117**

(43) Date of publication of application:
**16.08.2017 Bulletin 2017/33**

(73) Proprietor: **F. Hoffmann-La Roche AG**
**4070 Basel (CH)**

(72) Inventors:
- **FRIESS, Thomas**
**86911 Diessen-Dettenhofen (DE)**
- **KRIETER, Oliver**
**81379 Muenchen (DE)**
- **MAJETY, Meher**
**81241 Muenchen (DE)**
- **WARTHA, Katharina**
**CH-4054 Basel (CH)**

(74) Representative: **Townsend, Carolin**
**F. Hoffmann-La Roche AG**
**Patent Department**
**Grenzacherstrasse 124**
**4070 Basel (CH)**

(56) References cited:
**WO-A1-2011/039126 WO-A1-2014/161845**

- **OLIVER PATSY G ET AL: "Treatment of human colon cancer xenografts with TRA-8 anti-death receptor 5 antibody alone or in combination with CPT-11.", 1 April 2008 (2008-04-01), CLINICAL CANCER RESEARCH : AN OFFICIAL JOURNAL OF THE AMERICAN ASSOCIATION FOR CANCER RESEARCH 1 APR 2008, VOL. 14, NR. 7, PAGE(S) 2180 - 2189, XP002752434, ISSN: 1078-0432 the whole document**
- **PUKAC L ET AL: "HGS-ETR1, a fully human TRAIL-receptor 1 monoclonal antibody, induces cell death in multiple tumour types in vitro and in vivo", BRITISH JOURNAL OF CANCER, NATURE PUBLISHING GROUP, GB, vol. 92, no. 8, 1 April 2005 (2005-04-01), pages 1430-1441, XP002357744, ISSN: 0007-0920, DOI: 10.1038/SJ.BJC.6602487**
- **RAY SUBRATA ET AL: "Apoptosis induction in prostate cancer cells and xenografts by combined treatment with Apo2 ligand/tumor necrosis factor-related apoptosis-inducing ligand and CPT-11.", CANCER RESEARCH 1 AUG 2003, vol. 63, no. 15, 1 August 2003 (2003-08-01), pages 4713-4723, XP002752435, ISSN: 0008-5472**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 204 412 B1

- GANTEN TOM M ET AL: "Preclinical differentiation between apparently safe and potentially hepatotoxic applications of TRAIL either alone or in combination with chemotherapeutic drugs.", 15 April 2006 (2006-04-15), CLINICAL CANCER RESEARCH : AN OFFICIAL JOURNAL OF THE AMERICAN ASSOCIATION FOR CANCER RESEARCH 15 APR 2006, VOL. 12, NR. 8, PAGE(S) 2640 - 2646, XP002752436, ISSN: 1078-0432 the whole document

SEQUENCE LISTING

<110> F. Hoffmann - La Roche AG

<120> COMBINATION THERAPY OF BISPECIFIC ANTIBODIES SPECIFIC FOR FAP AND DR5 AND CHEMOTHERAPEUTIC AGENTS

<130> 32336 WO

<160> 20

<170> PatentIn version 3.5

<210> 1
<211> 5
<212> PRT

<213> Artificial sequence

<220>
<223> DR5 (5E11)_CDRH1

<400> 1

Ser Tyr Ala Met Ser
1 5

<210> 2
<211> 17
<212> PRT
<213> Artificial sequence

<220>
<223> DR5 (5E11)_CDRH2

<400> 2

Ala Ile Ser Gly Ser Gly Gly Ser Thr Tyr Tyr Ala Asp Ser Val Lys
1 5 10 15

Gly

<210> 3
<211> 8
<212> PRT
<213> Artificial sequence

<220>
<223> DR5 (5E11)_CDRH3

<400> 3

Gly Val Arg Val Ser Phe Asp Tyr
1 5

<210> 4
<211> 12
<212> PRT
<213> Artificial sequence

<220>
<223> DR5 (5E11)_CDRHL1

<400> 4

Arg Ala Ser Gln Ser Val Ser Ser Ser Tyr Leu Ala
1 5 10

<210> 5
<211> 7
<212> PRT
<213> Artificial sequence

<220>

<223> DR5 (5E11)_CDRL2

<400> 5

Gly Ala Ser Ser Arg Ala Thr
1               5

<210> 6
<211> 9
<212> PRT
<213> Artificial sequence

<220>
<223> DR5 (5E11)_CDRL3

<400> 6

Gln Gln Gly Thr Thr His Pro Ile Thr
1               5

<210> 7
<211> 117
<212> PRT
<213> Artificial sequence

<220>
<223> DR5 (5E11)_VH

<400> 7

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Val Arg Val Ser Phe Asp Tyr Trp Gly Gln Gly Thr Leu
100 105 110

Val Thr Val Ser Ser
115

<210> 8
<211> 108
<212> PRT
<213> Artificial sequence

<220>
<223> DR5 (5E11)_VL

<400> 8

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Gly Thr Thr His Pro
85 90 95

Ile Thr Phe Gly Gln Gly Thr Lys Val Glu Ile Lys
100 105

<210> 9
<211> 5
<212> PRT
<213> Artificial sequence

<220>
<223> FAP (28H1)_CDRH1

<400> 9

Ser His Ala Met Ser
1 5

<210> 10
<211> 16
<212> PRT
<213> Artificial sequence

<220>
<223> FAP (28H1)_CDRH2

<400> 10

Ala Ile Trp Ala Ser Gly Glu Gln Tyr Tyr Ala Asp Ser Val Lys Gly
1 5 10 15

<210> 11
<211> 8
<212> PRT
<213> Artificial sequence

<220>
<223> FAP (28H1)_CDRH3

<400> 11

Gly Trp Leu Gly Asn Phe Asp Tyr
1 5

<210> 12
<211> 12
<212> PRT
<213> Artificial sequence

<220>
<223> FAP (28H1)_CDRL1

<400> 12

Arg Ala Ser Gln Ser Val Ser Arg Ser Tyr Leu Ala
1 5 10

<210> 13
<211> 7
<212> PRT
<213> Artificial sequence

<220>
<223> FAP (28H1)_CDRL2

<400> 13

Gly Ala Ser Thr Arg Ala Thr
1 5

<210> 14

<211> 9
<212> PRT
<213> Artificial sequence

<220>
<223> FAP (28H1)_CDRL3

<400> 14

```
Gln Gln Gly Gln Val Ile Pro Pro Thr
1               5
```

<210> 15
<211> 116
<212> PRT
<213> Artificial sequence

<220>
<223> FAP (28H1)_VH

<400> 15

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val
        35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser
        115
```

<210> 16
<211> 108
<212> PRT
<213> Artificial sequence

<220>

<223> FAP (28H1)_VL

<400> 16

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Arg Ser
20 25 30

Tyr Leu Ala Trp Tyr Gln Gln Lys Pro Gly Gln Ala Pro Arg Leu Leu
35 40 45

Ile Ile Gly Ala Ser Thr Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Gly Gln Val Ile Pro
85 90 95

Pro Thr Phe Gly Gln Gly Thr Lys Val Glu Ile Lys
100 105

<210> 17
<211> 691
<212> PRT
<213> Artificial sequence

<220>
<223> DR5(5E11)-FAP (28H1) VHCL

<400> 17

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
```

```
Ala Lys Gly Val Arg Val Ser Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350
```

```
Pro Ser Arg Asp Glu Leu Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys Ser
        435                 440                 445

Gly Gly Gly Gly Ser Gly Gly Gly Gly Ser Gly Gly Gly Gly Ser Gly
    450                 455                 460

Gly Gly Gly Ser Glu Val Gln Leu Leu Glu Ser Gly Gly Gly Leu Val
465                 470                 475                 480

Gln Pro Gly Gly Ser Leu Arg Leu Ser Cys Ala Ala Ser Gly Phe Thr
                485                 490                 495

Phe Ser Ser His Ala Met Ser Trp Val Arg Gln Ala Pro Gly Lys Gly
            500                 505                 510

Leu Glu Trp Val Ser Ala Ile Trp Ala Ser Gly Glu Gln Tyr Tyr Ala
        515                 520                 525

Asp Ser Val Lys Gly Arg Phe Thr Ile Ser Arg Asp Asn Ser Lys Asn
    530                 535                 540

Thr Leu Tyr Leu Gln Met Asn Ser Leu Arg Ala Glu Asp Thr Ala Val
545                 550                 555                 560

Tyr Tyr Cys Ala Lys Gly Trp Leu Gly Asn Phe Asp Tyr Trp Gly Gln
                565                 570                 575

Gly Thr Leu Val Thr Val Ser Ser Ala Ser Val Ala Ala Pro Ser Val
            580                 585                 590

Phe Ile Phe Pro Pro Ser Asp Glu Gln Leu Lys Ser Gly Thr Ala Ser
```

595 600 605

Val Val Cys Leu Leu Asn Asn Phe Tyr Pro Arg Glu Ala Lys Val Gln
610 615 620

Trp Lys Val Asp Asn Ala Leu Gln Ser Gly Asn Ser Gln Glu Ser Val
625 630 635 640

Thr Glu Gln Asp Ser Lys Asp Ser Thr Tyr Ser Leu Ser Ser Thr Leu
645 650 655

Thr Leu Ser Lys Ala Asp Tyr Glu Lys His Lys Val Tyr Ala Cys Glu
660 665 670

Val Thr His Gln Gly Leu Ser Ser Pro Val Thr Lys Ser Phe Asn Arg
675 680 685

Gly Glu Cys
690

<210> 18
<211> 689
<212> PRT
<213> Artificial sequence

<220>
<223> DR5(5E11)-FAP (28H1) VHCL Removal of C-term. Lysine in Fc P329G/LALA mut.

<400> 18

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Val Arg Val Ser Phe Asp Tyr Trp Gly Gln Gly Thr Leu
```

```
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Ala Ala Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Gly Ala Pro Ile Glu Lys Thr Ile
                325                 330                 335

Ser Lys Ala Lys Gly Gln Pro Arg Glu Pro Gln Val Tyr Thr Leu Pro
            340                 345                 350
```

Pro Ser Arg Asp Glu Leu Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Gly Gly
435 440 445

Gly Gly Ser Gly Gly Gly Gly Ser Gly Gly Gly Gly Ser Gly Gly Gly
450 455 460

Gly Ser Glu Val Gln Leu Leu Glu Ser Gly Gly Gly Leu Val Gln Pro
465 470 475 480

Gly Gly Ser Leu Arg Leu Ser Cys Ala Ala Ser Gly Phe Thr Phe Ser
485 490 495

Ser His Ala Met Ser Trp Val Arg Gln Ala Pro Gly Lys Gly Leu Glu
500 505 510

Trp Val Ser Ala Ile Trp Ala Ser Gly Glu Gln Tyr Tyr Ala Asp Ser
515 520 525

Val Lys Gly Arg Phe Thr Ile Ser Arg Asp Asn Ser Lys Asn Thr Leu
530 535 540

Tyr Leu Gln Met Asn Ser Leu Arg Ala Glu Asp Thr Ala Val Tyr Tyr
545 550 555 560

Cys Ala Lys Gly Trp Leu Gly Asn Phe Asp Tyr Trp Gly Gln Gly Thr
565 570 575

Leu Val Thr Val Ser Ser Ala Ser Val Ala Ala Pro Ser Val Phe Ile
580 585 590

Phe Pro Pro Ser Asp Glu Gln Leu Lys Ser Gly Thr Ala Ser Val Val
595 600 605

Cys Leu Leu Asn Asn Phe Tyr Pro Arg Glu Ala Lys Val Gln Trp Lys
610 615 620

Val Asp Asn Ala Leu Gln Ser Gly Asn Ser Gln Glu Ser Val Thr Glu
625 630 635 640

Gln Asp Ser Lys Asp Ser Thr Tyr Ser Leu Ser Ser Thr Leu Thr Leu
645 650 655

Ser Lys Ala Asp Tyr Glu Lys His Lys Val Tyr Ala Cys Glu Val Thr
660 665 670

His Gln Gly Leu Ser Ser Pro Val Thr Lys Ser Phe Asn Arg Gly Glu
675 680 685

Cys

<210> 19
<211> 215
<212> PRT
<213> Artificial sequence

<220>
<223> DR5(5E11) LC

<400> 19

Glu Ile Val Leu Thr Gln Ser Pro Gly Thr Leu Ser Leu Ser Pro Gly
1 5 10 15

Glu Arg Ala Thr Leu Ser Cys Arg Ala Ser Gln Ser Val Ser Ser Ser
20 25 30

Tyr Leu Ala Trp Tyr Gln Gln Lys Pro Gly Gln Ala Pro Arg Leu Leu
35 40 45

Ile Tyr Gly Ala Ser Ser Arg Ala Thr Gly Ile Pro Asp Arg Phe Ser
50 55 60

Gly Ser Gly Ser Gly Thr Asp Phe Thr Leu Thr Ile Ser Arg Leu Glu
65 70 75 80

Pro Glu Asp Phe Ala Val Tyr Tyr Cys Gln Gln Gly Thr Thr His Pro
85 90 95

Ile Thr Phe Gly Gln Gly Thr Lys Val Glu Ile Lys Arg Thr Val Ala
100 105 110

```
Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215
```

<210> 20
<211> 214
<212> PRT
<213> Artificial sequence

<220>
<223> FAP (28H1) _VLCH1

<400> 20

```
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Arg Ser
            20                  25                  30

Tyr Leu Ala Trp Tyr Gln Gln Lys Pro Gly Gln Ala Pro Arg Leu Leu
        35                  40                  45

Ile Ile Gly Ala Ser Thr Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Gly Gln Val Ile Pro
                85                  90                  95

Pro Thr Phe Gly Gln Gly Thr Lys Val Glu Ile Lys Ser Ser Ala Ser

            100                 105                 110

Thr Lys Gly Pro Ser Val Phe Pro Leu Ala Pro Ser Ser Lys Ser Thr
        115                 120                 125

Ser Gly Gly Thr Ala Ala Leu Gly Cys Leu Val Lys Asp Tyr Phe Pro
    130                 135                 140

Glu Pro Val Thr Val Ser Trp Asn Ser Gly Ala Leu Thr Ser Gly Val
145                 150                 155                 160

His Thr Phe Pro Ala Val Leu Gln Ser Ser Gly Leu Tyr Ser Leu Ser
                165                 170                 175

Ser Val Val Thr Val Pro Ser Ser Ser Leu Gly Thr Gln Thr Tyr Ile
            180                 185                 190

Cys Asn Val Asn His Lys Pro Ser Asn Thr Lys Val Asp Lys Lys Val
        195                 200                 205

Glu Pro Lys Ser Cys Asp
    210
```

## Description

### FIELD OF THE INVENTION

The present invention relates to combination therapies employing bispecific antibodies comprising a first antigen binding site specific for Death Receptor 5 (DR5) and a second antigen binding site specific for Fibroblast Activation Protein (FAP) and a chemotherapeutic agent, and the use of these combination therapies for the treatment of cancer.

### BACKGROUND

Monoclonal antibodies are powerful therapeutic agents in the treatment of cancer since they selectively target antigens which are differentially expressed on cancer cells. Targeting of the TRAIL (TNF related apoptosis inducing ligand) death receptors on cancer cells with agonistic monoclonal antibodies represents a new generation of monoclonal antibody therapy, as they are able to directly induce apoptosis of targeted cells.

Upon binding of TRAIL, death receptors of the TNFR-SF family such as DR4 and DR5 become trimerized. The trimerization induces the extrinsic apoptotic pathway and a complex cascade of events including Caspase activation, which finally result in the killing of the target cells. Apoptosis induction is further enhanced if hyperclustering of DR5 (i.e. the clustering of multiple trimers) takes place. Although death receptors are widely expressed on a variety of cell types, induction of apoptosis via the extrinsic pathway is restricted to tumor cells. Since agonistic DR4 or DR5 binding antibodies are able to cross-link death receptors and hence induce apoptosis, these receptors are interesting targets in cancer therapy. At least eight death receptor targeting molecules entered clinical development and have been assessed in clinical trials for possible treatment of different indications such as advanced solid tumors like colorectal or lung cancers. In addition there have been attempts to treat other indications such as lymphoma and multiple myeloma.

Drozitumab, a fully human DR5 agonistic antibody described in US2007/0031414 and WO 2006/083971, shows some *in vitro* apoptotic activity in the absence of cross-linking at high concentrations. However, *in vivo* data revealed a different mode of action: In FcyR mutant mice (or when antibody variants were used in which FcyR binding was inhibited) Drozitumab was inactive indicating that the *in vivo* activity of this molecule is mainly dependent on FcyR mediated cross-linking. This molecule was tested up to clinical phase II, seemed to be safe (no MTD up to 20 mg/kg was reached) but did not demonstrate any significant efficacy.

Conatumumab (described in EP1922337A), is another fully human DR5 agonistic antibody. The activity of Conatumumab is strictly dependent on cross-linking via Fc receptors. In contrast to Drozitumab this antibody is non-ligand blocking. Also this molecule only showed very limited efficacy in clinical trials.

LBY-135, a chimeric DR5 antibody, exhibits similar characteristics as Conatumumab with respect to cross-linking dependent activity and non-ligand blocking property and did not demonstrate any significant efficacy in monotherapy. In addition, LBY-135 showed signs of immunogenicity in part of the enrolled patients of a phase I trial.

Dulanermin, a recombinantly produced natural ligand of DR4 and DR5 (TRAIL), only showed limited objective responses in clinical trials. The use of the natural ligand has somehow disadvantageous: TRAIL targets multiple receptors including both the death receptors and decoy receptors and, therefore, selectivity is a concern. In addition, TRAIL has a much shorter blood half-life compared with monoclonal anti- DR antibodies, a factor which affects dose and schedule parameters. The very short blood half-life of TRAIL requires large and frequent doses compared with monoclonal anti-DR antibodies. In addition recombinant TRAIL is very difficult and tedious to produce.

The development of all three DR5 agonistic antibodies and the ligand described above was discontinued.

Two additional fully human antibodies, Mapatumumab (anti-DR4) and Lexatumumab (anti-DR5) are still in development although also these molecules did not exhibit promising efficacy in monotherapy.

Tigatuzumab is a humanized DR5 agonistic antibody which is described as being active *in vitro* (already at low concentrations) in the absence of secondary cross-linking which of course bears the risk of systemic toxicity issues. However, as all the other described agonistic DR5 antibodies, also this molecule has not demonstrated convincing efficacy in Ph I / Ph II studies so far and the maximally tolerated dose MTD only was demonstrated up to 8 mg/kg.

A different approach to induce apoptosis by targeting a death receptor is pursued with the molecule TAS266, a tetrameric DR5 binding nanobody (WO 2011/098520). Due to the tetravalent configuration of DR5 binding moieties, it is thought that DR5 cross-linking is increased compared to standard bivalent antibodies, which may result in increased activity. However, due to their small size, these molecules have the disadvantage of a rather short half-life (compared to antibodies). In addition there is an increased risk of systemic toxicity since this tetrameric molecule is not targeted to the tumor.

Combining the DR5 antibody Drozitumab with a tumor antigen binding moiety or an antigen present in the stroma surrounding the tumor in a bispecific antibody platform has been described by the inventors of the present application as a new approach to achieve two effects: firstly the DR5 binding antibody can be targeted to the tumor site which could avoid potential systemic toxicity issues (especially when using a DR5 antibody exhibiting cross-linking

independent activity). Secondly, this tumor or tumor stroma targeting moiety then also serves as the cross-linking unit to induce DR5 hyperclustering and subsequently tumor site specific apoptosis. The basic concept has been demonstrated using Drozitumab_scFv fusion molecules targeting different tumor types (see WO 2011/039126).

Bispecific antibodies capable of binding to DR5 and Human Fibroblast Activation Protein (FAP; GenBank Accession Number AAC51668) have been proposed. Human FAP was originally identified in cultured fibroblasts using the monoclonal antibody (mAb) F19 (described in WO 93/05804, ATCC Number HB 8269). Homologues of the protein were found in several species, including mice (Niedermeyer et al., Int J Cancer 71, 383-389 (1997), Niedermeyer et al., Eur J Biochem 254, 650-654 (1998); GenBank Accession Number AAH19190). FAP has a unique tissue distribution: its expression was found to be highly upregulated on reactive stromal fibroblasts of more than 90% of all primary and metastatic epithelial tumors, including lung, colorectal, bladder, ovarian and breast carcinomas, while it is generally absent from normal adult tissues (Rettig et al., Proc Natl Acad Sci USA 85, 3110-3114 (1988); Garin-Chesa et al., Proc Natl Acad Sci USA 87, 7235-7239 (1990)). Subsequent reports showed that FAP is not only expressed in stromal fibroblasts but also in some types of malignant cells of epithelial origin, and that FAP expression directly correlates with the malignant phenotype (Jin et al., Anticancer Res 23, 3195-3198 (2003)).

The activity of conventional DR5 targeting molecules as described above is dependent on Fc Receptor (FcR) mediated hyperclustering, and is influenced by the immune infiltration and activation status in the tumor (Li and Ravetch, PNAS 2012; Wilson, Cancer Cell 2011; WO 2011/098520). The Fc/FcR interactions can be impaired by physiological human IgG levels. Thus, the activity of conventional DR5 targeting molecules is often limited to a few infiltrating cells (Moessner, Blood 2010). By using a bispecific antibody targeting both DR5 and FAP, the percentage of sensitive tumor cells can be significantly increased by hypercrosslinking via FAP and the risk of an intrinsic resistance to DR5 agonists is decreased. The novel DR5 binding moieties are only active after crosslinking with FAP, which could result in an improved safety and toxicology profile compared to the DR5 binders Drozitumab and Tigatuzumab. The DR5 agonists that have been tested so far were safe in the clinic; however, these clinical programs have been impeded by a low efficacy of the DR5 targeting molecules.

It remains a problem in the art to find ways of effective therapies for targeting cancer using DR5 agonist antibodies.

## SUMMARY

Broadly, the present invention relates to bispecific antibodies combining a Death Receptor 5 (DR5) targeting antigen binding site with a second antigen binding site that targets Fibroblast Activation Protein (FAP) and their use in combination with a further chemotherapeutic agent. The bispecific antibodies employed in accordance with the present invention enable the death receptors become cross linked and induce apoptosis of the targeted tumor cell. The advantage over conventional death receptor targeting antibodies is the specificity of induction of apoptosis only at the site where FAP is expressed as well as the higher potency of these bispecific antibodies due to the induction of DR5 hyperclustering. The detailed technical disclosure set out below may in some respects go beyond the disclosure of the invention per se, and may also provide technical background for related technical developments. It will be appreciated that this technical disclosure is not intended to define the invention as such (which is defined exclusively by the appended claims), but rather to place it in a broader technical context. Accordingly, it will be appreciated that the term "embodiments" reflects a specific detail of the disclosure and is not intended to define as part of the invention aspects that do not fall within the scope of the appended claims.

Accordingly, in one aspect, the present invention provides a bispecific antibody that binds to Death Receptor 5 (DR5) and Fibroblast Activation Protein (FAP) comprising at least one antigen binding site specific for DR5 and at least one antigen binding site specific for FAP for use as a combination therapy in a method of treating cancer, wherein the bispecific antibody is used in combination with a chemotherapeutic agent selected from Irinotecan, Doxorubicin, Oxaliplatin, 5-FU, a MDM2 inhibitor, a Bcl-2 inhibitor, Abraxane, Paclitaxel, Gemcitabine, Bortezomib, Cyclopamine, a PARP inhibitor, Ifosfamide or an anti-VEGF antibody.

Preferably, the chemotherapeutic agent is selected from Irinotecan, Doxorubicin, Oxaliplatin, Ifosfamide or an anti-VEGF antibody. In one embodiment, the bispecific antibody is used in combination with Irinotecan. In one such embodiment the cancer to be treated is colorectal cancer. In one embodiment, the bispecific antibody is used in combination with an anti-VEGF antibody. In one such embodiment the cancer to be treated is colorectal cancer. In one embodiment, the bispecific antibody is used in combination with doxorubicin. In one such embodiment the cancer to be treated is a sarcoma. In one embodiment, the bispecific antibody is used in combination with doxorubicin Ifosfamide . In one such embodiment the cancer to be treated is a sarcoma.

In one embodiment, the bispecific antibody is used in combination with Gemcitabine and Abraxane. In one such embodiment the cancer to be treated is pancreatic cancer.

In one embodiment the MDM2 inhibitor is RG7388.

In one embodiment the Bcl-2 inhibitor is ABT199.

In one embodiment the PARP inhibitor is PJ34.

In one aspect the PARP inhibitor is Olaparip.

In one embodiment the present invention provides a bispecific antibody that binds to Death Receptor 5 (DR5) and Fibroblast Activation Protein (FAP) comprising at least one antigen binding site specific for DR5 and at least one antigen binding site specific for FAP for use as a combination therapy in a method of treating cancer, wherein the cancer is colorectal cancer, sarcoma, head and neck cancer, squamous cell carcinoma, breast cancer, pancreatic cancer, gastric cancer, non-small-cell lung carcinoma, small-cell lung cancer and mesothelioma.

In one embodiment the cancer is colorectal cancer.

In one embodiment the sarcoma is chondrosarcoma, leiomyosarcoma, gastrointestinal stromal tumours, fibrosarcoma, osteosarcoma, liposarcoma or maligant fibrous histiocytoma.

In one embodiment the present invention provides a bispecific antibody that binds to Death Receptor 5 (DR5) and Fibroblast Activation Protein (FAP) comprising at least one antigen binding site specific for DR5 and at least one antigen binding site specific for FAP for use as a combination therapy in a method of treating cancer, wherein the bispecific antibody and the chemotherapeutic agent are administered together, optionally as a combined formulation.

In one embodiment the bispecific antibody and the chemotherapeutic agent are administered by alternation.

In one embodiment the chemotherapeutic agent is administered before the bispecific antibody.

In one embodiment the chemotherapeutic agent is administered after the bispecific antibody.

In one embodiment the combination is administered at intervals from about one week to three weeks.

In some embodiments, the present invention employs a bispecific antibody in which the antigen binding site specific for DR5 comprises:

(a) a heavy chain CDR1 of SEQ ID NO.:1;
(b) a heavy chain CDR2 of SEQ ID NO.:2;
(c) a heavy chain CDR3 of SEQ ID NO.:3;
(d) a light chain CDR1 of SEQ ID NO.:4;
(e) a light chain CDR2 of SEQ ID NO.:5; and
(f) a light chain CDR3 of SEQ ID NO.:6.

In some embodiments, the present invention employs a bispecific antibody in which the antigen binding site specific for FAP comprises

(a) a heavy chain CDR1 of SEQ ID NO.:9;
(b) a heavy chain CDR2 of SEQ ID NO.:10;
(c) a heavy chain CDR3 of SEQ ID NO.:11;
(d) a light chain CDR1 of SEQ ID NO.:12;
(e) a light chain CDR2 of SEQ ID NO.:13; and
(f) a light chain CDR3 of SEQ ID NO.:14.

In one embodiment, the bispecific antibody comprises at least one antigen binding site specific for DR5 comprising a variable heavy chain and a variable light chain comprising an amino acid sequence of: SEQ ID NO.:7 and SEQ ID NO.:8.

In some embodiments, the present invention, the bispecific antibody comprises the antigen binding site specific for FAP comprises a variable heavy chain and a variable light chain comprising an amino acid sequence selected from the group of SEQ ID NO.:15 and SEQ ID NO.:16.

In some embodiments, the present invention, the bispecific antibody comprises the antigen binding site specific for DR5 comprises: (a) a heavy chain CDR1 of SEQ ID NO.:1;(b) a heavy chain CDR2 of SEQ ID NO.:2; (c) a heavy chain CDR3 of SEQ ID NO.:3; (d) a light chain CDR1 of SEQ ID NO.:4; (e) a light chain CDR2 of SEQ ID NO.:5; (f) a light chain CDR3 of SEQ ID NO.:6 and the antigen binding site specific for FAP comprises: (a) a heavy chain CDR1 of SEQ ID NO.:9; (b) a heavy chain CDR2 of SEQ ID NO.:10; (c) a heavy chain CDR3 of SEQ ID NO.:1 1; (d) a light chain CDR1 of SEQ ID NO.:12; (e) a light chain CDR2 of SEQ ID NO.:13; (f) a light chain CDR3 of SEQ ID NO.:14.

In one embodiment, the bispecific antibody comprises at least one antigen binding site specific for DR5 comprising a variable heavy chain comprising an amino acid sequence of SEQ ID NO.:7 and a variable light chain comprising an amino acid sequence of SEQ ID NO.:8; and at least one antigen binding site specific for FAP comprising a heavy chain variable region comprising an amino acid sequence of SEQ ID NO.:15 and a light chain variable region comprising an amino acid sequence of SEQ ID NO.:16.

In some embodiments, the present invention, the bispecific antibody comprises the bispecific antibody comprises amino acid sequences of SEQ ID NO: 18, 19 and 20 or the bispecific antibody comprises amino acid sequences SEQ ID NO: 17, 19 and 20.

In all aspects of the present invention, advantageously said bispecific antibody is human or humanized.

In some embodiments, the bispecific antibody comprises an Fc domain, at least one Fab fragment comprising the antigen binding site specific for DR5, and at least one Fab fragment comprising the antigen binding site specific for FAP.

In some embodiments, the bispecific antibody comprises an Fc domain, two Fab fragments comprising each an antigen binding site specific for DR5, and two Fab fragments comprising each an antigen binding site specific for FAP.

In some embodiments, the bispecific antibody is bivalent both for DR5 and FAP.

In some embodiments, the bispecific antibody comprises one or more Fab fragment(s) comprising an antigen binding site specific for FAP, wherein the variable regions or the constant regions of the heavy and light chain are exchanged.

In some embodiments, the bispecific antibody comprises an Fc domain, at least one Fab fragment comprising the antigen binding site specific for DR5, and at least one Fab fragment comprising the antigen binding site specific for FAP wherein either the variable regions or the constant regions of the heavy and light chain of at least one Fab fragment are exchanged.

In some aspects, the bispecific antibody comprises:

a) an Fc domain,
b) two Fab fragments comprising an antigen binding site specific for DR5,
wherein said Fab fragments are connected at the C-terminus of the constant heavy chain (CH1) to the first or second subunit of the Fc domain,
c) two Fab fragments comprising the antigen binding site specific for FAP,

wherein the two Fab fragments are connected at the N-terminus of the variable heavy chain (VH) to the first or second subunit of the Fc domain.

In one embodiment at least one of said Fab fragments is connected to the Fc domain via a peptide linker.

In one embodiment said bispecific antibody comprises an Fc domain, which comprises one or more amino acid substitution that reduces binding to Fc receptors and/or effector function. In one embodiment said one or more amino acid substitution is at one or more positions selected from the group of L234, L235, and P329. In one embodiment each subunit of the Fc domain comprises three amino acid substitutions that abolish binding to an activating or inhibitory Fc receptor and/or effector function wherein said amino acid substitutions are L234A, L235A and P329G.

In a further aspect, the present invention provides a pharmaceutical composition comprising a bispecific antibody as described herein and a chemotherapeutic agent selected from Irinotecan, Doxorubicin, Oxaliplatin, 5-FU, a MDM2 inhibitor, a Bcl-2 inhibitor, Abraxane, Paclitaxel, Gemcitabine, Bortezomib, Cyclopamine, a PARP inhibitor, Ifosfamide or an anti-VEGF antibody. In one embodiment, the pharmaceutical composition comprises a bispecific antibody as described herein, Gemcitabine and Paclitaxel or Abraxane.

In one embodiment the MDM2 inhibitor is RG7388.

In one embodiment the Bcl-2 inhibitor is ABT199.

In one embodiment the PARP inhibitor is PJ34.

In one aspect the PARP inhibitor is Olaparip.

In a further aspect, the present invention provides a pharmaceutical composition comprising a bispecific antibody as described herein and a chemotherapeutic agent selected from Irinotecan, Doxorubicin, Oxaliplatin, Ifosfamide or an anti-VEGF antibody.

In a further aspect, the present invention provides a pharmaceutical composition comprising a bispecific antibody as described herein and Irinotecan.

In a further aspect, the present invention provides a pharmaceutical composition comprising a bispecific antibody as described herein, Paclitaxel and Gemcitabine or Abraxane.

In a further aspect, the present invention provides the use of a combination of a bispecific antibody that binds to Death Receptor 5 (DR5) and Fibroblast Activation Protein (FAP) and a chemotherapeutic agent in the manufacture of a medicament for the treatment of cancer, wherein the bispecific antibody is used in combination with a chemotherapeutic agent selected from Irinotecan, Doxorubicin, Oxaliplatin, 5-FU, a MDM2 inhibitor , a Bcl-2 inhibitor ABT199, Abraxane, Paclitaxel, Gemcitabine, Bortezomib, Cyclopamine, a PARP inhibitor, Ifosfamide or an anti-VEGF antibody. In one aspect the present invention provides the use of a combination of a bispecific antibody that binds to Death Receptor 5 (DR5) and Fibroblast Activation Protein (FAP), Paclitaxel and Abraxane in the manufacture of a medicament for the treatment of cancer.

In a further aspect, the present invention provides the use of a combination of a bispecific antibody that binds to Death Receptor 5 (DR5) and Fibroblast Activation Protein (FAP) and a chemotherapeutic agent in the manufacture of a medicament for the treatment of cancer, wherein the bispecific antibody is used in combination with a chemotherapeutic agent selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody.

In a further aspect, the present invention provides a kit comprising:

(i) a first container comprising a composition which comprises a bispecific antibody as described herein; and
(ii) a second container comprising a composition comprising a chemotherapeutic agent selected from Irinotecan, Doxorubicin, Oxaliplatin, 5-FU, a MDM2 inhibitor , a Bcl-2 inhibitor, Abraxane, Paclitaxel, Gemcitabine, Bortezomib, Cyclopamine, aPARP inhibitor, Ifosfamide or an anti-VEGF antibody.

In a further aspect, the present invention provides a kit comprising:

(i) a first container comprising a composition which comprises a bispecific antibody as described herein; and
(ii) a second container comprising a composition comprising a chemotherapeutic agent selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody.

In a further aspect, the present invention provides a kit comprising:

(i) a first container comprising a composition which comprises a bispecific antibody as described herein; and
(ii) a second container comprising Abraxane, and
(iii) a third container comprising Gemcitabine.

In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one antigen binding site specific for FAP and a therapeutically effective amount of a chemotherapeutic agent selected from Irinotecan, Doxorubicin, Oxaliplatin, 5-FU, a MDM2 inhibitor, a Bcl-2 inhibitor, Abraxane, Paclitaxel, Gemcitabine, Bortezomib, Cyclopamine, a PARP inhibitor, Ifosfamide or an anti-VEGF antibody.

In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one antigen binding site specific for FAP and a therapeutically effective amount of a chemotherapeutic agent selected from selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody.

In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one antigen binding site specific for FAP and a therapeutically effective amount of Abraxane and a therapeutically effective amount of Gemcitabine.

In some embodiments, the cancer is colorectal cancer, sarcoma, head and neck cancers, squamous cell carcinomas, breast cancer, pancreatic cancer, gastric cancer, non-small-cell lung carcinoma, small-cell lung cancer, desmoplastic melanoma and mesothelioma. In one embodiment, the cancer is colorectal cancer. In other embodiments, the sarcoma is chondrosarcoma, leiomyosarcoma, gastrointestinal stromal tumours, fibrosarcoma, osteosarcoma. liposarcoma or maligant fibrous histiocytoma.

In some embodiments, the bispecific antibody and the chemotherapeutic agent are administered together, optionally as a combined formulation. Alternatively, the bispecific antibody and the chemotherapeutic agent may be administered by alternation, with either the chemotherapeutic agent administered before the bispecific antibody, or the chemotherapeutic agent administered after the bispecific antibody. The combination may be administered in accordance with clinical practice, for example being administered at intervals from about one week to three weeks.

Embodiments of the present invention will now be described by way of example and not limitation with reference to the accompanying figures. However various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited

to any particular aspect or embodiment of the invention and apply equally to all aspects and embodiments which are described.

## BRIEF DESCRIPTION OF THE FIGURES

**Figure 1.** Schematic representation of the FAP-DR5 bispecific antibody molecule design and mode of action.

**Figure 2.** Plot of % inhibition (cell viability assay) of HT29 CRC cells at 3 days for multiple concentrations of DR5 Ab + FC alone and in combination with 10 μM Irinotecan.

**Figure 3.** Plot of % inhibition (cell viability assay) of BxPC3 cells at 3 days for multiple concentrations of DR5 Ab + FC alone and in combination with 20 nM Abraxane (Nab- Paclitaxel).

**Figure 4.** Plot of % inhibition (cell viability assay) of Capan2 PDAC cells at 3 days for multiple concentrations of DR5 Ab + FC alone and in combination with 20 nM Bortezomib.

**Figure 5.** Plot of the *in vivo* median tumor volume change over time in DLD-1 CRC xenograft bearing mice treated with either vehicle, DR5-FAP bispecific antibody (1 and 10 mg/kg) or Drozitumab LALA (10 mg/kg). Animals were treated from day 9 to 20 for 4 times with DR5-FAP bispecific antibody and once weekly with Drozitumab LALA. The Tumor Growth Inhibition for treatment with DR5-FAP was calculated at 89% (10 mg/kg) and 79% (1.0 mg/kg), respectively.

**Figure 6.** Plot of the *in vivo* median tumor volume change over time in DLD-1 CRC xenograft bearing mice treated with either vehicle, DR5-FAP bispecific antibody (10 mg/kg), Irinotecan (15 mg/kg) or a combination of DR5-FAP bispecific antibody and Irinotecan. Animals were treated once weekly for 5 times with DR5-FAP bispecific antibody and every 5 days for a total of 3 times with Irinotecan. The Tumor Regression for treatment with the combination of DR5-FAP and Irinotecan was calculated at 72%.

**Figure 7.** Plot of the *in vivo* median tumor volume change over time in DLD-1 CRC xenograft bearing mice treated with either vehicle, DR5-FAP bispecific antibody (10 mg/kg), Irinotecan (15 mg/kg) or a combination of DR5-FAP bispecific antibody and Irinotecan applied in parallel or sequentially. Animals were treated from day 7 once weekly i.v. for 5 times with DR5-FAP bispecific antibody (days 7, 14, 21, 28, 35) and with irinotecan i.p. daily on 5 days in total for 3 cycles (days 7-11, 19-23, 33-36)

**Figure 8.** Plot of the *in vivo* median tumor volume change over time in HCT116 CRC xenograft bearing mice treated with either vehicle, DR5-FAP bispecific antibody (10 mg/kg), Irinotecan (15 mg/kg) or a combination of DR5-FAP bispecific antibody and Irinotecan. Animals were treated from day 7 once weekly for 3 times with DR5-FAP bispecific antibody and every 5 days for a total of 2 times with Irinotecan. The Tumor Growth Inhibition for treatment with DR5-FAP was calculated at 46% and tumor regression was induced in combination with Irinotecan (TGI>100%).

**Figure 9.** Plot of the *in vivo* median tumor volume change over time in HCT116 CRC xenograft bearing mice treated with either vehicle, DR5-FAP bispecific antibody (10 mg/kg), Oxaliplatin (5 mg/kg) or a combination of DR5-FAP bispecific antibody and Oxaliplatin. Animals were treated from day 7 once weekly for 3 times with DR5-FAP bispecific antibody (days 7, 14, 21) and i.p. for a total of 2 cycles with Oxaliplatin (days 7-10, 21-23). The Tumor Growth Inhibition for treatment with the combination of DR5-FAP and Oxaliplatin was calculated at 67%.

**Figure 10.** Plot of the *in vivo* median tumor volume change over time in LOX-IMVI desmoplastic melanoma xenograft bearing mice treated with either vehicle, DR5-FAP bispecific antibody (10 mg/kg) or Drozitumab LALA (10 mg/kg). Animals were treated from day 13 to 20 twice with DR5-FAP bispecific antibody or Drozitumab LALA. The Tumor Growth Inhibition for treatment with DR5-FAP was calculated at over 100%, whilst treatment with Drozitumab LALA was calculated at 65%.

**Figure 11.** Plot of the *in vivo* median tumor volume change over time in patient-derived Co5896 CRC xenograft bearing mice treated with either vehicle or DR5-FAP bispecific antibody (30 mg/kg). Animals were treated from day 18 to 34 for 6 times with DR5-FAP bispecific antibody. The Tumor Growth Inhibition for treatment with DR5-FAP was calculated at 76%.

**Figure 12.** Plot of the *in vivo* median tumor volume change over time in patient-derived Co5896 CRC xenograft bearing mice treated with either vehicle, DR5-FAP bispecific antibody (30 mg/kg), Irinotecan (15 mg/kg) or a combination of DR5-FAP bispecific antibody and Irinotecan. Animals were treated once weekly for 4 times with DR5-FAP bispecific antibody from days 15-19 with Irinotecan. Treatment with the combination of DR5-FAP and Irinotecan resulted in complete tumor regression in all animals (10/10).

**Figure 13.** IHC image showing FAP+ stroma in patient-derived Co5896 CRC xenograft bearing mice.

**Figure 14.** Plot of the *in vivo* median tumor volume change over time in patient-derived Sarc4605 sarcoma xenograft bearing mice treated with either vehicle or DR5-FAP bispecific antibody (10 mg/kg). Animals were treated from day 10 to 31 for 4 times with DR5-FAP bispecific antibody. The Tumor Growth Inhibition for treatment with DR5-FAP was calculated at over 100%.

**Figure 15.** Plot of the *in vivo* median tumor volume change over time in DLD-1 CRC xenograft bearing mice treated with either vehicle, DR5-FAP bispecific antibody (10 mg/kg), Ang2/VEGF bispecific antibody (10 mg/kg) or combination of DR5-FAP bispecific antibody (10 mg/kg) and Ang2/VEGF bispecific antibody (10 mg/kg). Both antibodies were given on once weekly on days 8, 15 and 22. While treatment with the DR5-FAP antibody as single agent resulted in significant tumor growth inhibition (TGI 87%) the combination with anti-Ang/VEGF Mab was additive efficacious and increased tumor growth inhibition to 94%. Treatment with Ang2/VEGF antibody alone inhibited tumor growth at 75%.

**Figure 16.** Plot of the *in vivo* median tumor volume change over time in LOX-IMVI desmoplastic melanoma xenograft bearing mice treated with either vehicle, DR5-FAP angbispecific antibody (10 mg/kg) or Doxorubicin (5 mg/kg) or the combination of DR5-FAP bispecific antibody (10 mg/kg) and Doxorubicin (5 mg/kg). Animals were treated from day 8 to 15 twice with DR5-FAP bispecific antibody or the combination. While treatment with the DR5-FAP antibody (10mg/kg, days 8 and 15) as single agent resulted in tumor stasis (TGI 97%) the combination with doxorubicin was more than additive efficacious and caused distinct tumor regression (93%). Treatment with doxorubicin alone inhibited tumor growth at 77%.

**Figure 17.** Plot of the *in vivo* median tumor volume change over time in patient-derived Sarc4605 sarcoma xenograft bearing mice treated with either vehicle, DR5-FAP bispecific antibody (10 mg/kg), Doxorubicin (5 mg/kg), or a combination of DR5-FAP bispecific antibody (10 mg/kg) and Doxorubicin (5 mg/kg). Animals were treated from day 20 to 41 for 4 times with DR5-FAP bispecific antibody or the combination. While treatment with the DR5-FAP antibody (10mg/kg, days 20, 27, 34 and 41) as single agent resulted in tumor stasis (TGI 99%) the combination with doxorubicin was more than additive efficacious and caused distinct tumor regression (83%). Treatment with doxorubicin alone inhibited tumor growth at 77%.

**Figure 18.** Plot of the *in vivo* median tumor volume change over time in patient-derived PA1178 PDAC xenograft bearing mice treated with either vehicle, DR5-FAP bispecific antibody (10 mg/kg), gemcitabine (40 mg/kg), gemcitabine (40 mg/kg) and nab-paclitaxel (6 mg/kg), or a combination of DR5-FAP bispecific antibody (10 mg/kg) and gemcitabine (40 mg/kg) and nab-paclitaxel (6 mg/kg). Animals were treated from day 32 to 81 for 7 times with DR5-FAP bispecific antibody or the combination. While treatment with the DR5-FAP antibody as single agent resulted in tumor growth inhibition of 96 % the triple combination was efficacious with complete tumor remission.

**Figure 19.** Plot of the *in vivo* median tumor volume change over time in patient-derived Sarc4605 sarcoma xenograft bearing mice treated with either vehicle, DR5-FAP bispecific antibody (10 mg/kg), Ifosfamid (100 mg/kg), or a combination of DR5-FAP bispecific antibody (10 mg/kg) and Ifosfamid (100 mg/kg),. Animals were treated from day 20 to 41 for 4 times with DR5-FAP bispecific antibody or the combination. (10mg/kg) in combination with alkylating drug ifosfamide (100mg/kg, q7dx2). Treatment with DR5-FAP antibody (10mg/kg, q7dx8) alone resulted in strong tumor regression (96% with 80% tumor free) whereas combination with ifosfamid was slightly more efficacious (86% tumor free). Additionally, the kinetic of tumor regression was faster in combination.

**Figure 20** Luminex Data after treatment with single agent bispecific DR5-FAP antibody (10 mg/kg; DR5 binder: VH SEQ ID NO.:7, VL SEQ ID NO.: 8, FAP binder: VH SEQ ID N0.:15, VL SEQ ID NO.: 16). The bispecific DR5-FAP antibody induced strong time-related tumor cells apoptosis against DLD-1/3T3 xenografts. Strong effects were observed shortly after antibody treatment (6h).

**Figure 21** Luminex Data after treatment with single agent bispecific DR5-FAP antibody (10 mg/kg; DR5 binder: VH SEQ ID NO.:7, VL SEQ ID NO.: 8, FAP binder: VH SEQ ID NO.:15, VL SEQ ID NO.: 16) or in combination with doxorubicin (10 mg/kg). The bispecific DR5-FAP antibody strongly induces tumor cell apoptosis in a time-related fashion. The tumor cell apoptosis induction is superior with the combination treatment of the bispecific DR5-FAP antibody together with doxorubicin. A: Analysis of induction of cleaved Caspase-3 (effector Caspase). B: Analysis of induction of activated Caspase-8 (extrinsic apoptosis pathway). C: Analysis of induction of activated Caspase-9 (intrinsic apoptosis pathway).

**Figure 22** Luminex Data after treatment with single agent bispecific DR5-FAP antibody (10 mg/kg; DR5 binder: VH SEQ ID NO.:7, VL SEQ ID NO.: 8, FAP binder: VH SEQ ID NO.:15, VL SEQ ID NO.: 16) or in combination with irinotecan (15 mg/kg) or oxaliplatin (5 mg/kg). The bispecific DR5-FAP antibody strongly induces tumor cell apoptosis in a time-related fashion. The tumor cell apoptosis induction is superior with the combination treatment of the bispecific DR5-FAP antibody together with irinotecan or oxaliplatin. A: Analysis of induction of cleaved PARP B: Analysis of induction of Caspase-3 (effector Caspase) C: Analysis of induction of activated Caspase-9 (intrinsic apoptosis pathway) D: Analysis of induction of activated Caspase-8 (extrinsic apoptosis pathway).

## DETAILED DESCRIPTION OF EMBODIMENTS OF THE INVENTION

### I. DEFINITIONS

An "acceptor human framework" for the purposes herein is a framework comprising the amino acid sequence

of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The term "A bispecific antibody that specifically binds death receptor 5 (DR5) and Fibroblast Activation Protein (FAP)" refers to a bispecific antibody that is capable of binding DR5 and FAP with sufficient affinity such that the antibody is useful as a diagnostic and/or therapeutic agent in targeting cells expressing DR5 and FAP). Specifically "A bispecific antibody that specifically binds death receptor 5 (DR5) and Fibroblast Activation Protein (FAP)" refers to a bispecific antibody targeting DR5 on a tumor cell and FAP in the stroma surrounding said tumor. In one embodiment, the extent of binding of a bispecific antibody that specifically binds death receptor 5 (DR5) and Fibroblast Activation Protein (FAP) to an unrelated, non-FAP or non-DR5 protein is less than about 10% of the binding of the antibody to DR5 or FAP as measured, e.g., by a Enzyme-linked immunosorbent assay (ELISA), surface plasmon resonance (SPR) based assays (e.g. Biacore) or flow cytometry (FACS). In certain embodiments, a bispecific antibody that specifically binds death receptor 5 (DR5) and Fibroblast Activation Protein (FAP) has a dissociation constant (Kd) of $\leq 1\mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g., from $10^{-9}$ M to $10^{-13}$ M). In certain embodiments, a bispecific antibody that specifically binds death receptor 5 (DR5) and Fibroblast Activation Protein (FAP) binds to an epitope of DR5 or FAP that is conserved among DR5 or FAP from different species. Preferably said bispecific antibody binds to human and cynomolgous monkey DR5 and to human, cynomolgous monkey and mouse FAP.

The terms "An antibody that specifically binds death receptor 5 (DR5)" refers to an antibody that is capable of binding DR5 with sufficient affinity such that the antibody is useful as a diagnostic and/or therapeutic agent in targeting cells expressing DR5. In one embodiment, the extent of binding of an antibody that specifically binds death receptor 5 (DR5) to an unrelated non-DR5 protein is less than about 10% of the binding of the antibody to DR5 as measured, e.g., by a radioimmunoassay (RIA) or flow cytometry (FACS). In certain embodiments, an antibody that specifically binds death receptor 5 (DR5) has a dissociation constant (Kd) of $\leq 1\mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g., from $10^{-9}$ M to $10^{-13}$ M). In certain embodiments, an antibody that specifically binds death receptor 5 (DR5) binds to an epitope of DR5 that is conserved among DR5 from different species. Preferably said antibody binds to human and cynomolgous monkey DR5. The term "An antibody that specifically binds death receptor 5 (DR5)" also encompasses bispecific antibodies that are capable of binding DR5 and a second antigen.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies, cross-Fab fragments; linear antibodies; single-chain antibody molecules (e.g. scFv); and multispecific antibodies formed from antibody fragments. scFv antibodies are, e.g. described in Houston, J.S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

As used herein, "Fab fragment" refers to an antibody fragment comprising a light chain fragment comprising a VL domain and a constant domain of a light chain (CL), and a VH domain and a first constant domain (CH1) of a heavy chain. In one embodiment the bispecific antibodies of the invention comprise at least one Fab fragment, wherein either the variable regions or the constant regions of the heavy and light chain are exchanged. Due to the exchange of either the variable regions or the constant regions, said Fab fragment is also referred to as "cross-Fab fragment" or "xFab

fragment" or "crossover Fab fragment". Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as $CrossFab_{(VLVH)}$. On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as $CrossFab_{(CLCH1)}$. Bispecific antibody formats comprising crossover Fab fragments have been described, for example, in WO 2009/080252, WO 2009/080253, WO 2009/080251, WO 2009/080254, WO 2010/136172, WO 2010/145792 and WO 2013/026831.

A "single chain Fab fragment" or "scFab" is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction:
a) VH-CH1-linker-VL-CL, b) VL-CL-linker-VH-CH1, c) VH-CL-linker-VL-CH1 or d) VL-CH1-linker-VH-CL; and wherein said linker is a polypeptide of at least 30 amino acids, preferably between 32 and 50 amino acids. Said single chain Fab fragments a) VH-CH1-linker-VL-CL, b) VL-CL-linker-VH-CH1, c) VH-CL-linker-VL-CH1 and d) VL-CH1-linker-VH-CL, are stabilized via the natural disulfide bond between the CL domain and the CH1 domain. In addition, these single chain Fab molecules might be further stabilized by generation of interchain disulfide bonds via insertion of cysteine residues (e.g. position 44 in the variable heavy chain and positionn 100 in the variable light chain according to Kabat numbering). The term "N-terminus denotes the last amino acid of the N-terminus. The term "C-terminus denotes the last amino acid of the C-terminus. By "fused" or "connected" is meant that the components (e.g. a Fab molecule and an Fc domain subunit) are linked by peptide bonds, either directly or via one or more peptide linkers.

The term "linker" as used herein refers to a peptide linker and is preferably a peptide with an amino acid sequence with a length of at least 5 amino acids, preferably with a length of 5 to 100, more preferably of 10 to 50 amino acids. In one embodiment said peptide linker is $(G_xS)_n$ or $(G_xS)_nG_m$ with G = glycine, S = serine, and (x = 3, n= 3, 4, 5 or 6, and m= 0, 1, 2 or 3) or (x = 4,n= 2, 3, 4 or 5 and m= 0, 1, 2 or 3), preferably x = 4 and n= 2 or 3, more preferably with x = 4, n= 2. In one embodiment said peptide linker is $(G_4S)_2$.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CHI, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. The heavy chain of an immunoglobulin may be assigned to one of five types, called $\alpha$ (IgA), $\delta$ (IgD), $\varepsilon$ (IgE), $\gamma$ (IgG), or $\mu$ (IgM), some of which may be further divided into subtypes, e.g. $\gamma_1$ ($IgG_1$), $\gamma_2$ ($IgG_2$), $\gamma_3$ ($IgG_3$), $\gamma_4$ ($IgG_4$), $\alpha_1$ ($IgA_1$) and $\alpha_2$ ($IgA_2$). The light chain of an immunoglobulin may be assigned to one of two types, called kappa ($\kappa$) and lambda ($\lambda$), based on the amino acid sequence of its constant domain. An immunoglobulin essentially consists of two Fab molecules and an Fc domain, linked via the immunoglobulin hinge region.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "antigen binding domain" refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more antibody variable domains (also called antibody variable regions). Preferably, an antigen binding domain comprises an antibody light chain variable region (VL) and an antibody heavy chain variable region (VH).

The term "chimeric" antibody refers to an antibody in which a portion of the heavy and/or light chain is derived from a particular source or species, while the remainder of the heavy and/or light chain is derived from a different source or species, usually prepared by recombinant DNA techniques. Chimeric antibodies comprising a rabbit variable region and a human constant region are preferred. Other preferred forms of "chimeric antibodies" encompassed by the present invention are those in which the constant region has been modified or changed from that of the original antibody to generate the properties according to the invention, especially in regard to C1q binding and/or Fc receptor (FcR) binding. Such chimeric antibodies are also referred to as "class-switched antibodies". Chimeric antibodies are the product of

expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art. See e.g. Morrison, S.L., et al., Proc. Natl. Acad. Sci. USA 81 (1984) 6851-6855; US Patent Nos. 5,202,238 and 5,204,244.

The term "cytotoxic agent" as used herein refers to a substance that inhibits or prevents a cellular function and/or causes cell death or destruction. Cytotoxic agents include, but are not limited to, radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); antibody-dependent cellular phagocytosis (ADCP), cytokine secretion, immune complex-mediated antigen uptake by antigen presenting cells; down regulation of cell surface receptors (e.g. B cell receptor); and B cell activation.

As used herein, the terms "engineer, engineered, engineering", are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to an Fc receptor, or increased association with another peptide. Amino acid sequence deletions and insertions include amino- and/or carboxy-terminal deletions and insertions of amino acids. Particular amino acid mutations are amino acid substitutions. For the purpose of altering e.g. the binding characteristics of an Fc region, non-conservative amino acid substitutions, i.e. replacing one amino acid with another amino acid having different structural and/or chemical properties, are particularly preferred. Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc domain to glycine can be indicated as 329G, G329, $G_{329}$, P329G, or Pro329Gly.

An "effective amount" of an agent, e.g., a pharmaceutical formulation, refers to an amount effective, at dosages and for periods of time necessary, to achieve the desired therapeutic or prophylactic result.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991. A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be

non-identical in the sense that further components fused to each of the subunits (e.g. antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. As also mentioned for chimeric and humanized antibodies according to the invention the term "human antibody" as used herein also comprises such antibodies which are modified in the constant region to generate the properties according to the invention, especially in regard to C1q binding and/or FcR binding, e.g. by "class switching" i.e. change or mutation of Fc parts (e.g. from IgG1 to IgG4 and/or IgG1/IgG4 mutation.)

The term "recombinant human antibody", as used herein, is intended to include all human antibodies that are prepared, expressed, created or isolated by recombinant means, such as antibodies isolated from a host cell such as a NS0 or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to *in vivo* somatic hypermutation. Thus, the amino acid sequences of the VH and VL regions of the recombinant antibodies are sequences that, while derived from and related to human germ line VH and VL sequences, may not naturally exist within the human antibody germ line repertoire *in vivo.*

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

The term "hypervariable region" or "HVR," as used herein refers to each of the regions of an antibody variable domain which are hypervariable in sequence and/or form structurally defined loops ("hypervariable loops"). Generally, native four-chain antibodies comprise six HVRs; three in the VH (HI, H2, H3), and three in the VL (LI, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) Hypervariable regions (HVRs) are also referred to as complementarity

determining regions (CDRs), and these terms are used herein interchangeably in reference to portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table A as a comparison. The exact residue numbers which encompass a particular CDR will vary depending on the sequence and size of the CDR. Those skilled in the art can routinely determine which residues comprise a particular CDR given the variable region amino acid sequence of the antibody.

TABLE A. CDR Definitions[1]

| CDR | Kabat | Chothia | AbM[2] |
|---|---|---|---|
| $V_H$ CDR1 | 31-35 | 26-32 | 26-35 |
| $V_H$ CDR2 | 50-65 | 52-58 | 50-58 |
| $V_H$ CDR3 | 95-102 | 95-102 | 95-102 |
| $V_L$CDR1 | 24-34 | 26-32 | 24-34 |
| $V_L$ CDR2 | 50-56 | 50-52 | 50-56 |
| $V_L$ CDR3 | 89-97 | 91-96 | 89-97 |

[1] Numbering of all CDR definitions in Table A is according to the numbering conventions set forth by Kabat et al. (see below).
[2] "AbM" with a lowercase "b" as used in Table A refers to the CDRs as defined by Oxford Molecular's "AbM" antibody modeling software.

Kabat *et al.* also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra.*

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding a bispecific antibody that specifically binds DR5 and FAP antibody" refers to one or more nucleic acid molecules encoding antibody heavy and light chains (or fragments thereof), including such nucleic acid molecule(s) in a single vector or separate vectors, and such nucleic acid molecule(s) present at one or more

locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"No substantial cross-reactivity" means that a molecule (*e.g.,* an antibody) does not recognize or specifically bind an antigen different from the actual target antigen of the molecule (e.g. an antigen closely related to the target antigen), particularly when compared to that target antigen. For example, an antibody may bind less than about 10% to less than about 5% to an antigen different from the actual target antigen, or may bind said antigen different from the actual target antigen at an amount consisting of less than about 10%, 9%, 8% 7%, 6%, 5%, 4%, 3%, 2%, 1%, 0.5%, 0.2%, or 0.1%, preferably less than about 2%, 1%, or 0.5%, and most preferably less than about 0.2% or 0.1% antigen different from the actual target antigen.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid

sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "death receptor 5 (DR5)", as used herein, refers to any native DR5 from any vertebrate source, including mammals such as primates (e.g. humans) and rodents (e.g., mice and rats), unless otherwise indicated. The term encompasses "full-length," unprocessed DR5 as well as any form of DR5 that results from processing in the cell. The term also encompasses naturally occurring variants of DR5, e.g., splice variants or allelic variants. The amino acid sequence of an exemplary human DR5 is disclosed in WO 2011/039126.

The term "Fibroblast activation protein (FAP)", as used herein, refers to any native FAP from any vertebrate source, including mammals such as primates (e.g. humans) and rodents (e.g., mice and rats), unless otherwise indicated. The term encompasses "full-length," unprocessed FAP as well as any form of FAP that results from processing in the cell. The term also encompasses naturally occurring variants of FAP, e.g., splice variants or allelic variants. Preferably, an anti-FAP antibody of the invention binds to the extracellular domain of FAP. The amino acid sequence of exemplary FAP polypeptide sequences, including the sequence of human FAP, are disclosed in WO 2012/020006.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease. The term cancer as used herein refers to proliferative diseases, such as the cancer is colorectal cancer, sarcoma, head and neck cancer, squamous cell carcinoma, breast cancer, pancreatic cancer, gastric cancer, non-small-cell lung carcinoma, small-cell lung cancer and mesothelioma, including refractory versions of any of the above cancers, or a combination of one or more of the above cancers. In one embodiment, the cancer is colorectal cancer and optionally the chemotherapeutic agent is Irinotecan. "Sarcoma" as used herein refers to a cancer type that grows in connective tissue. Sarcomas include Gastro-intestinal stromal tumours (a type of soft tissue sarcoma found in the stomach and intestines commonly known as GIST), soft tissue sarcomas (e.g. Leiomyosarcoma, Fibroblastic sarcoma, Liposarcoma, Kaposi's sarcoma (KS), Angiosarcoma, Malignant peripheral nerve sheath tumour (MPNST), Synovial sarcoma, Rhabdomyosarcoma) and bone sarcomas (e.g. Chondrosarcoma, Osteosarcoma, Ewing's sarcoma, Chordoma)

In embodiments in which the cancer is sarcoma, optionally the sarcoma is chondrosarcoma, leiomyosarcoma, gastrointestinal stromal tumours, fibrosarcoma, osteosarcoma. liposarcoma or maligant fibrous histiocytoma.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "antigen-binding site of an antibody" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

Antibody specificity refers to selective recognition of the antibody for a particular epitope of an antigen. Natural antibodies, for example, are monospecific. "Bispecific antibodies" according to the invention are antibodies which have two different antigen-binding specificities. Antibodies of the present invention are specific for two different antigens, i.e.

DR5 as first antigen and FAP as second antigen.

The term "monospecific" antibody as used herein denotes an antibody that has one or more binding sites each of which bind to the same epitope of the same antigen.

The term "bispecific" antibody as used herein denotes an antibody that has at least two binding sites each of which bind to different epitopes of the same antigen or a different antigen.

The antibody provided herein is a multispecific antibody, e.g. a bispecific antibody. Multispecific antibodies are monoclonal antibodies that have binding specificities for at least two different sites. Provided herein is a bispecific antibody, with binding specificities for FAP and DR5. In certain embodiments, bispecific antibodies may bind to two different epitopes of DR5. Bispecific antibodies may also be used to localize cytotoxic agents to cells which express DR5. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising at least one antigen binding site that binds to FAP or DR5 as well as another, different antigen (see, US 2008/0069820, for example).

The term "valent" as used within the current application denotes the presence of a specified number of binding sites in an antibody molecule. As such, the terms "bivalent", "tetravalent", and "hexavalent" denote the presence of two binding sites, four binding sites, and six binding sites, respectively, in an antibody molecule. The bispecific antibodies according to the invention are at least "bivalent" and may be "trivalent" or "multivalent" (e.g."tetravalent" or "hexavalent").

Antibodies of the present invention have two or more binding sites and are bispecific. That is, the antibodies may be bispecific even in cases where there are more than two binding sites (i.e. that the antibody is trivalent or multivalent). Bispecific antibodies of the invention include, for example, multivalent single chain antibodies, diabodies and triabodies, as well as antibodies having the constant domain structure of full length antibodies to which further antigen-binding sites (e.g., single chain Fv, a VH domain and/or a VL domain, Fab, or (Fab)2) are linked via one or more peptide-linkers. The antibodies can be full length from a single species, or be chimerized or humanized.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The term "amino acid" as used within this application denotes the group of naturally occurring carboxy $\alpha$-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

As used herein, the expressions "cell", "cell line", and "cell culture" are used interchangeably and all such designations include progeny. Thus, the words "transfectants" and "transfected cells" include the primary subject cell and cultures derived there from without regard for the number of transfers. It is also understood that all progeny may not be precisely identical in DNA content, due to deliberate or inadvertent mutations. Variant progeny that have the same function or biological activity as screened for in the originally transformed cell are included.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

As used herein, the term "binding" or "specifically binding" refers to the binding of the antibody to an epitope of the antigen in an in-vitro assay, preferably in a surface plasmon resonance assay (SPR, BIAcore, GE-Healthcare Uppsala, Sweden). The affinity of the binding is defined by the terms ka (rate constant for the association of the antibody

from the antibody/antigen complex), kD (dissociation constant), and KD (kD/ka). Binding or specifically binding means a binding affinity (KD) of $10^{-8}$ mol/l or less, preferably $10^{-9}$ M to $10^{-13}$ mol/l.

Binding of the antibody to the death receptor can be investigated by a BIAcore assay (GE-Healthcare Uppsala, Sweden). The affinity of the binding is defined by the terms ka (rate constant for the association of the antibody from the antibody/antigen complex), kD (dissociation constant), and KD (kD/ka)

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of an antigen that is bound by an antibody.

As used herein, the terms "engineer, engineered, engineering," particularly with the prefix "glyco-," as well as the term "glycosylation engineering" are considered to include any manipulation of the glycosylation pattern of a naturally occurring or recombinant polypeptide or fragment thereof. Glycosylation engineering includes metabolic engineering of the glycosylation machinery of a cell, including genetic manipulations of the oligosaccharide synthesis pathways to achieve altered glycosylation of glycoproteins expressed in cells. Furthermore, glycosylation engineering includes the effects of mutations and cell environment on glycosylation. In one embodiment, the glycosylation engineering is an alteration in glycosyltransferase activity. In a particular embodiment, the engineering results in altered glucosaminyltransferase activity and/or fucosyltransferase activity.

## II. COMPOSITIONS AND METHODS

In one aspect, the invention is based on the use of a therapeutic combination of bispecific antibodies comprising a first antigen binding site specific for TRAIL death receptor 5 (DR5) and a second antigen binding site specific for Fibroblast Activation Protein (FAP) and a further chemotherapeutic agent, e.g., for the treatment of cancer.

### A. Combination therapies of bispecific antibodies specific for FAP and DR5

Broadly, the present invention relates to bispecific antibodies combining a Death Receptor 5 (DR5) targeting antigen binding site with a second antigen binding site that targets Fibroblast Activation Protein (FAP) and their use in combination with a further chemotherapeutic agent. The bispecific antibodies employed in accordance with the present invention enable the death receptors become cross linked and induce apoptosis of the targeted tumor cell. The advantage over conventional death receptor targeting antibodies is the specificity of induction of apoptosis only at the site where FAP is expressed as well as the higher potency of these bispecific antibodies due to the induction of DR5 hyperclustering.

Accordingly, in one aspect, the present invention provides a bispecific antibody that binds to Death Receptor 5 (DR5) and Fibroblast Activation Protein (FAP) comprising at least one antigen binding site specific for DR5 and at least one antigen binding site specific for FAP for use as a combination therapy in a method of treating cancer, wherein the bispecific antibody is used in combination with a chemotherapeutic agent selected from Irinotecan, Doxorubicin, Oxaliplatin, 5-FU, a MDM2 inhibitor, a Bcl-2 inhibitor, Abraxane, Paclitaxel, Gemcitabine, Bortezomib, Cyclopamine, a PARP inhibitor, Ifosfamide or an anti-VEGF antibody.

Preferably, the chemotherapeutic agent is selected from Irinotecan, Doxorubicin, Oxaliplatin, Ifosfamide or an anti-VEGF antibody. In one embodiment, the chemotherapeutic agent is selected from Irinotecan, Doxorubicin or Oxaliplatin. In one embodiment, the bispecific antibody is used in combination with Irinotecan. In one embodiment, the bispecific antibody is used in combination with Paclitaxel and Gemcitabine. In one embodiment, the bispecific antibody is used in combination with Ifosfamide.

Irinotecan (Camptosar®) is a drug used for the treatment of cancer. Irinotecan prevents DNA from unwinding by inhibition of topoisomerase 1. Irinotecan is a semisynthetic analogue of the natural alkaloid camptothecin and is named (*S*)-4,11-diethyl-3,4,12,14-tetrahydro-4-hydroxy- 3,14-dioxo1*H*-pyrano[3',4':6,7]-indolizino[1,2-blquinolin-9-yl-[1,4'bipiperidine]-1'-carboxylate. Irinotecan has the structure:

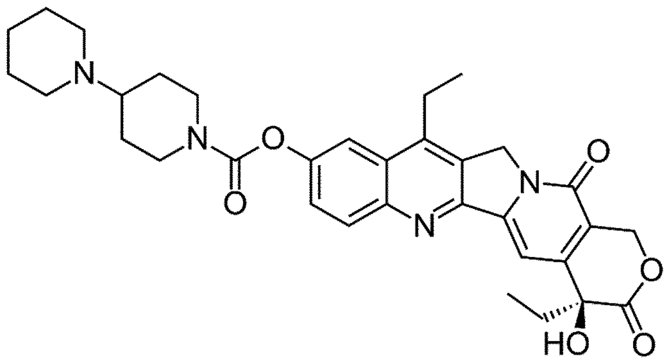

Doxorubicin (ADRIAMYCIN®) is chemotherapeutic agent using in the treatment of a wide range of cancers. Doxorubicin intercalates with DNA, leading to the inhibition of DNA synthesis; interfers with topoisomerase 2, preventing DNA replication and increases production of free radicals, contributing to its cytotoxicitiy. Doxorubicin is an anthracycline antibiotic that is closely related to natural daunoycin and is named (7S,9S)-7-[(2R,4S,5S,6S)-4-amino-5-hydroxy-6-methyloxan-2-yl]oxy-6,9,11-trihydroxy-9-(2-hydroxyacetyl)-4-methoxy-8,10-dihydro-7H-tetracene-5,12-dione. Doxorubicin has the structure:

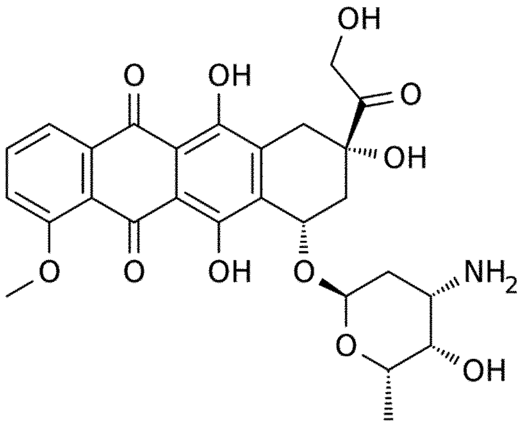

Oxaliplatin (ELOXATIN®, Sanofi) is a platinum-based chemotherapy drug used for the treatment of cancer, in particular colorectal cancer. In physiological solutions, oxaliplatin undergoes nonenzymatic conversion to active derivatives and leads to cross-linking of DNA, inhibiting DNA synthesis and transcription Oxaliplatin is named [(1*R*,2*R*)-cyclohexane-1,2-diamine](ethanedioato-*O,O'*)platinum(II) and has the structure:

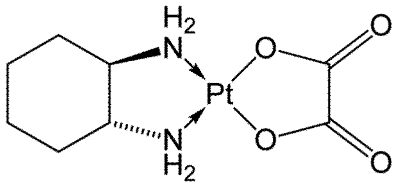

5-FU (5-fluorouracil) is widely used in the treatment of cancer. 5-FU is an analogue of uracil which is transported into cells and is converted into active metabolites. These metabolites disrupt RNA synthesis and inhibits the thymidylate synthase enzyme, blocking synthesis of thymidine which is necessary for DNA replication and repair. 5-FU is named 5-Fluoro-1*H*,3*H*-pyrimidine-2,4-dione and has the structure:

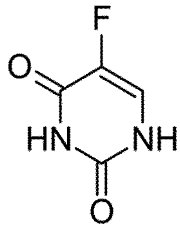

MDM2 inhibitors block p53-MDM2 binding, leading to activation of the p53 pathway which results in cell cycle arrest and/or apoptosis. In one embodiment the MDM2 inhibitor is RG7388.

The MDM2 inhibitor RG7388 is small-molecule pyrrolidine compound undergoing clinical investigation in the treatment of cancer (Ding et al., J. Med. Chem. 56(14): 5979-5983, 2013). MDM2 inhibitor RG7388 is named 4-((2R,3S,4R,5S)-3-(3-chloro-2-fluorophenyl)-4-(4-chloro-2-fluorophenyl)-4-cyano-5-neopentylpyrrolidine-2-carboxamido)-3-methoxybenzoic acid and has the structure:

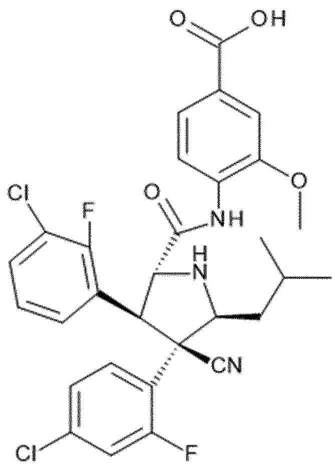

In another aspect the MDM2 inhibitor is MK-8242. Other MDM2 inhibitors are known in the art and are described e.g. in Swatu Palit Deb and Sumitra Deb: Mutant p53 and MDM2 in Cancer, Subcellular Biochemistry 85, Springer (ISBN 978-94-017-9211-0).

Bcl-2 (B-cell lymphoma 2) inhibitors target the Bcl-2 family proteins and are intended to restore the sensitivity of cancer cells to pro-apoptotic signals. In one embodiment the Bcl-2 inhibitor is ABT199.

The Bcl-2 inhibitor ABT199, also known as GDC-0199, is a selective inhibitor undergoing clinical investigation in the treatment of cancer (Souers et al., (2013) Nat. Med. 19(2):202-208). It was designed to mimic the binding of the BH3 structural element present on the Bcl-2 protein, important in the regulation of apoptosis. ABT199 is named (4-(4-{[2-(4-chlorophenyl)-4,4-dimethylcyclohex-1-en-1-yl]methyl}piperazin-1-yl)-*N*-({3-nitro-4-[(tetrahydro-2*H*-pyran-4-ylmethyl)amino]phenyl}sulfonyl)-2-(1*H*-pyrrolo[2,3-*b*]pyridin-5-yloxy)benzamide) and has the structure:

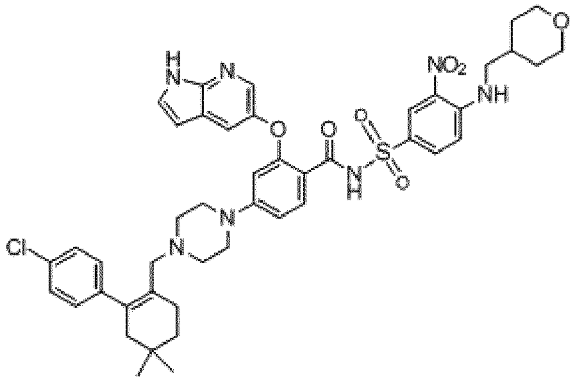

Taxanes (paclitaxel and abraxane) are drugs derived from the twigs, needles and bark of Pacific yew trees

*Taxus brevifolia.* They have demonstrated antitumor activity in a variety of tumor types. The taxanes function by interfering with microtubule growth by hyperstabilising their structure, destroying the cell's ability to use its cytoskeleton during cell division and resulting in aberrant cell function and eventual cell death. Paclitaxel is delivered into the cell dissolved in a solvent called Cremophor, a toxic derivative of castor oil. Albumin-bound paclitaxel (trade name Abraxane, also called nab-paclitaxel) is an alternative formulation where paclitaxel is bound to albumin nano-particles, which is an alternative, less toxic, delivery agent. Paclitaxel is named 5β,20-Epoxy-1,2α,4,7β,10β,13β-hexahydroxytax-11-en-9-one 4,10-diacetate 2-benzoate 13-ester with (2R,3S)-N-benzoyl-3-phenylisoserine and has the structure:

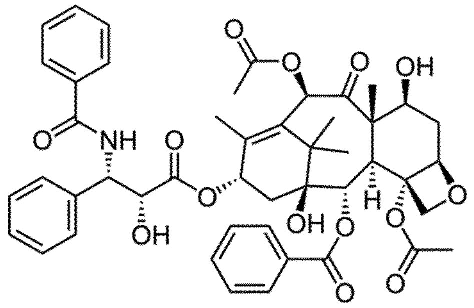

Bortezomib (VELCADE®) is a therapeutic proteasome inhibitor used in the treatment of cancer. Also known as PS-341, Bortezomib specifically and reversibly inhibits the threonine residue of the 26S proteasome, an enzyme complex involved in the degradation of various proteins critical to cancer cell survival. Inhibiting degradation of these proteins sensitises cells to apoptosis. Bortezomib is named [(1R)-3-methyl-1-[(2S)-3-phenyl-2-(pyrazin-2-ylformamido)propanamido]butyl]boronic acid and has the structure:

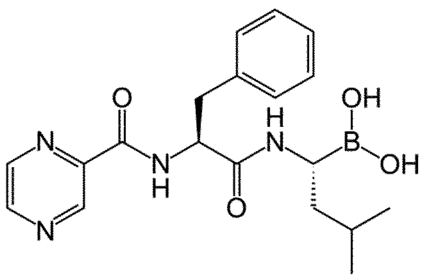

Gemcitabine (GEMZAR®) is a chemotherapeutic that kills cells undergoing DNA synthesis. Gemcitabine is a nucleoside analog that is metabolised by nucleoside kinases to diphosphate and triphosphate nucleosides. Gemcitabine diphosphate inhibits ribonucleotide reductase, an enzyme required for DNA synthesis, and gemcitabine triphosphate competes with deoxycytidine for incorporation into DNA. Gemcitabine is named 4-amino-1-(2-deoxy-2,2-difluoro-β-D-*erythro*-pentofuranosyl)pyrimidin-2(1*H*)-on and has the structure:

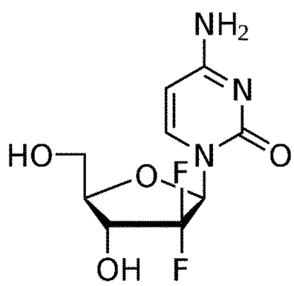

Cyclopamine is a natural alkaloid that is being investigated as a potential cancer treatment. Inappropriate activation of the hedgehog pathway is associated with tumor formation and growth. Cylopamine is able to kill cancer cells by disrupting the hedgehog signalling pathway by directly binding to the receptor smoothened. Cyclopamine is named (3β,23*R*)-17,23-Epoxyveratraman-3-ol and has the structure:

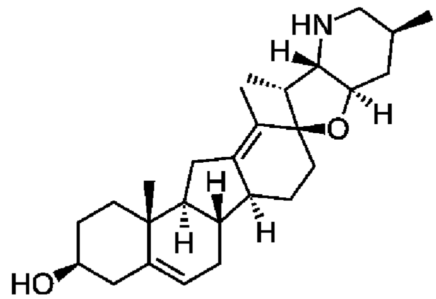

PARP inhibitors are a group of pharmacological inhibitors of the enzyme poly ADP ribose polymerase (PARP).
In one embodiment the PARP inhibitor is PJ34.
The PARP inhibitor PJ34 causes a PARP 1-independent, p21 dependent mitotic arrest. See Madison et al., DNA Repair (Amst). 2011 Oct 10;10(10):1003-13. doi: 10.1016/j.dnarep.2011.07.006. Epub 2011 Aug 12. PJ34 blocks the activity of PARP-1, preventing cells which contain damaged DNA from repairing single-stand breaks and leading to cell death. PJ34 is named N-(5,6-Dihydro-6-oxo-2-phenanthridinyl)-2-acetamide hydrochloride and has the structure:

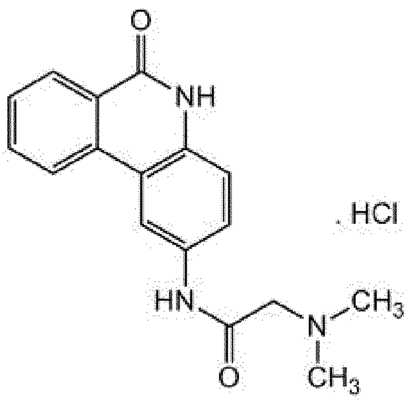

In one aspect the PARP inhibitor is Olaparip. Olaparip is an inhibitor of poly ADP ribose polymerase (PARP), an enzyme involved in DNA repair. It acts against cancers in people with hereditary BRCA1 or BRCA2 mutations, which includes many ovarian, breast and prostate cancers. Olaparip is named 4-[(3-[(4-cyclopropylcarbonyl) piperazin-4-yl]carbonyl) -4-fluorophenyl]methyl(2H)phthalazin-1-one and has the structure:

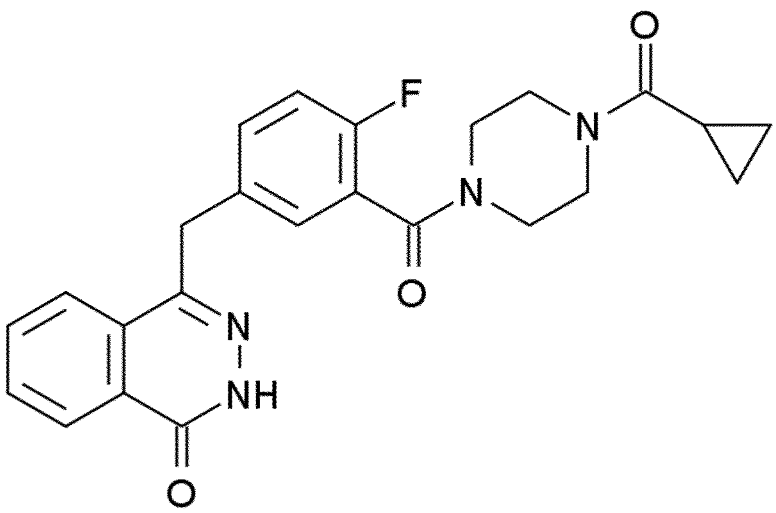

Ifosfamide is a nitrogen mustard alkylating agent used in the treatment of cancer with the systematic name N-3-bis(2-chloroethyl)-1,3,2-oxazaphosphinan-2-amide-2-oxide, also known e.g. under the Trade name Ifex and has the structure:

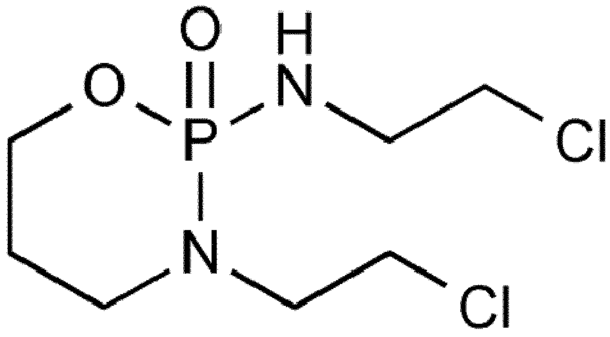

Ifosfamide is a chemotherapy drug used to treat different cancers including testicular cancer, sarcoma and some types of lymphoma.

A further class of chemotherapeutic agents that may be employed in therapeutic combinations with the bispecific DR5-FAP antibodies of the present invention are anti-VEGF antibodies. Examples of anti-VEGF antibodies include anti-VEGF antibodies or peptide-antibody fusions targeted to angiogenesis-promoting growth factor receptors, e.g. Bevacizumab (Avastin®), Lucentis® (ranibizumab), Cetuximab (Erbitux®), Ramucirumab (Cyramza®), Icrucumab, HuMV833, 2C3, Aflibercept (Zaltrap®) and IMC-1C11. A preferred anti-VEGF antibody is Bevacizumab (Avastin®). A further example of an anti-VEGF antibody is a anti-VEGF Ang2 bispecific antibody as described in WO2010/040508 and WO2011/117329.

In a further aspect, the present invention provides a pharmaceutical composition comprising a bispecific antibody as described herein and a chemotherapeutic agent selected from Irinotecan, Doxorubicin, Oxaliplatin, 5-FU, a MDM2 inhibitor, a Bcl-2 inhibitor, Abraxane, Paclitaxel, Gemcitabine, Bortezomib, Cyclopamine, a PARP inhibitor, Ifosfamide or an anti-VEGF antibody.

In a further aspect, the present invention provides a pharmaceutical composition comprising a bispecific antibody as described herein and a chemotherapeutic agent selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody. In a further aspect, the present invention provides a pharmaceutical composition comprising a bispecific antibody as described herein and Abraxane and Gemcitabine.

In a further aspect, the present invention provides a kit comprising:

(i) a first container comprising a composition which comprises a bispecific antibody as described herein; and
(ii) a second container comprising a composition comprising a chemotherapeutic agent selected from Irinotecan, Doxorubicin, Oxaliplatin, 5-FU, a MDM2 inhibitor , a Bcl-2 inhibitor, Abraxane, Paclitaxel, Gemcitabine, Bortezomib, Cyclopamine, aPARP inhibitor, Ifosfamide or an anti-VEGF antibody.

In a further aspect, the present invention provides a kit comprising:

(i) a first container comprising a composition which comprises a bispecific antibody as described herein; and
(ii) a second container comprising a composition comprising a chemotherapeutic agent selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody.

In a further aspect, the present invention provides a kit comprising:

(i) a first container comprising a composition which comprises a bispecific antibody as described herein; and
(ii) a second container comprising a composition comprising a chemotherapeutic agent selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody.

In a further aspect, the present invention provides a kit comprising:

(i) a first container comprising a composition which comprises a bispecific antibody as described herein; and
(ii) a second container comprising Paclitaxel or Abraxane, and
(iii) a third container comprising Gemcitabine.

In a further aspect, the present invention provides the use of a combination of a bispecific antibody that binds to Death Receptor 5 (DR5) and Fibroblast Activation Protein (FAP) and a chemotherapeutic agent in the manufacture

of a medicament for the treatment of cancer, wherein the bispecific antibody is used in combination with a chemotherapeutic agent selected from Irinotecan, Doxorubicin, Oxaliplatin, 5-FU, a MDM2 inhibitor, the Bcl-2 inhibitor ABT199, Abraxane, Paclitaxel, Gemcitabine, Bortezomib, Cyclopamine, a PARP inhibitor, Ifosfamide or an anti-VEGF antibody. In a further aspect, the present invention provides the use of a combination of a bispecific antibody that binds to Death Receptor 5 (DR5) and Fibroblast Activation Protein (FAP) and a chemotherapeutic agent in the manufacture of a medicament for the treatment of cancer, wherein the bispecific antibody is used in combination with a chemotherapeutic agent selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody.

In a further aspect, the present invention provides the use of a combination of a bispecific antibody that binds to Death Receptor 5 (DR5) and Fibroblast Activation Protein (FAP) and a chemotherapeutic agent in the manufacture of a medicament for the treatment of cancer, wherein the bispecific antibody is used in combination with Irinotecan. In one such aspect the cancer to be treated is colorectal cancer.

In a further aspect, the present invention provides the use of a combination of a bispecific antibody that binds to Death Receptor 5 (DR5) and Fibroblast Activation Protein (FAP) and a chemotherapeutic agent in the manufacture of a medicament for the treatment of cancer, wherein the bispecific antibody is used in combination with an anti-VEGF antibody, preferably bevacizumab. In one such aspect the cancer to be treated is colorectal cancer.

In a further aspect, the present invention provides the use of a combination of a bispecific antibody that binds to Death Receptor 5 (DR5) and Fibroblast Activation Protein (FAP) and a chemotherapeutic agent in the manufacture of a medicament for the treatment of cancer, wherein the bispecific antibody is used in combination with Doxorubicin. In one such aspect the cancer to be treated is a sarcoma.

In a further aspect, the present invention provides the use of a combination of a bispecific antibody that binds to Death Receptor 5 (DR5) and Fibroblast Activation Protein (FAP) and a chemotherapeutic agent in the manufacture of a medicament for the treatment of cancer, wherein the bispecific antibody is used in combination with Ifosfamide. In one such aspect the cancer to be treated is a sarcoma.

In a further aspect, the present invention provides the use of a combination of a bispecific antibody that binds to Death Receptor 5 (DR5) and Fibroblast Activation Protein (FAP) and a chemotherapeutic agent in the manufacture of a medicament for the treatment of cancer, wherein the bispecific antibody is used in combination with Abraxane and Gemcitabine. In one such aspect the cancer to be treated is pancreatic cancer.

In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one antigen binding site specific for FAP and a therapeutically effective amount of a chemotherapeutic agent selected from Irinotecan, Doxorubicin, Oxaliplatin, 5-FU, a MDM2 inhibitor, a Bcl-2 inhibitor, Abraxane, Paclitaxel, Gemcitabine, Bortezomib, Cyclopamine, a PARP inhibitor, Ifosfamide or an anti-VEGF antibody.
In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one antigen binding site specific for FAP and a therapeutically effective amount of a chemotherapeutic agent selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody.

In some embodiments, the cancer is colorectal cancer, sarcoma, head and neck cancers, squamous cell carcinomas, breast cancer, pancreatic cancer, gastric cancer, non-small-cell lung carcinoma, small-cell lung cancer, desmoplastic melanoma and mesothelioma. In one embodiment, the cancer is colorectal cancer. In other embodiments, the sarcoma is chondrosarcoma, leiomyosarcoma, gastrointestinal stromal tumours, fibrosarcoma, osteosarcoma. liposarcoma or maligant fibrous histiocytoma.

In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one antigen binding site specific for FAP and a therapeutically effective amount of Irinotecan. In one such aspect the cancer to be treated is colorectal cancer.

In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one

antigen binding site specific for FAP and a therapeutically effective amount of an anti-VEGF antibody, e.g. bevacizumab. In one such aspect the cancer to be treated is colorectal cancer.

In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one antigen binding site specific for FAP and a therapeutically effective amount of Abraxane and a therapeutically effective amount of Gemcitabine. In one such aspect the cancer to be treated is pancreatic cancer.

In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one antigen binding site specific for FAP and a therapeutically effective amount of Doxorubicin. In one such aspect the cancer to be treated is a sarcoma.

In a further aspect, the present application provides a method for the treatment of a cancer comprising administering a therapeutic combination as a combined formulation or by alternation to a mammal, wherein the therapeutic combination comprises a therapeutically effective amount of a bispecific antibody that binds to Death Receptor 5 (DR5) and Fibroblast Activation Protein (FAP) comprising at least one antigen binding site specific for DR5 and at least one antigen binding site specific for FAP and a therapeutically effective amount of Ifosfamide. In one such aspect the cancer to be treated is a sarcoma.

In some embodiments, the bispecific antibody and the chemotherapeutic agent are administered together, optionally as a combined formulation. Alternatively, the bispecific antibody and the chemotherapeutic agent may be administered by alternation, with either the chemotherapeutic agent administered before the bispecific antibody, or the chemotherapeutic agent administered after the bispecific antibody. The combination may be administered in accordance with clinical practice, for example being administered at intervals from about one week to three weeks.

### B. Exemplary bispecific antibodies that bind to DR5 and FAP

In one aspect, the invention provides isolated bispecific antibodies that bind to DR5 and FAP. FAP binding moieties have been described in WO 2012/020006, herein referred to. FAP binding moieties of particular interest to be used in the DR5-FAP bispecific antibodies are outlined in the embodiments below.

In certain embodiments, a bispecific antibody that binds to DR5 and FAP specifically crosslinks the death receptors and apoptosis of the target cell is induced. The advantage of these bispecific death receptor agonistic antibodies over conventional death receptor targeting antibodies is the specificity of induction of apoptosis only at the site where FAP is expressed. As outlined above the inventors of the present invention developed DR5 binding moieties with superior properties compared to known DR5 binders that can be incorporated into novel and advantageous DR5-FAP bispecific antibodies.

In one aspect, the present invention provides therapeutic combinations that comprise a bispecific antibody that binds to DR5 and FAP comprising
at least one antigen binding site specific for DR5, comprising

(a) a heavy chain CDR1 of SEQ ID NO.:1;
(b) a heavy chain CDR2 of SEQ ID NO.:2;
(c) a heavy chain CDR3 of SEQ ID NO.:3;
(d) a light chain CDR1 of SEQ ID NO.:4;
(e) a light chain CDR2 of SEQ ID NO.:5;
(f) a light chain CDR3 of SEQ ID NO.:6

and at least one antigen binding site specific for FAP, comprising

(a) a heavy chain CDR1 of SEQ ID NO.:9;
(b) a heavy chain CDR2 of SEQ ID NO.:10;
(c) a heavy chain CDR3 of SEQ ID NO.:11;
(d) a light chain CDR1 of SEQ ID NO.:12;
(e) a light chain CDR2 of SEQ ID NO.:13;
(f) a light chain CDR3 of SEQ ID NO.:14.

In one embodiment, the bispecific antibody comprises at least one antigen binding site specific for DR5, comprising a variable heavy chain comprising an amino acid sequence of SEQ ID NO.:7 and a variable light chain comprising an amino acid sequence of SEQ ID NO.:8; and at least one antigen binding site specific for FAP, comprising a heavy chain variable region comprising an amino acid sequence of SEQ ID NO.:15 and a light chain variable region comprising an amino acid sequence of SEQ ID NO.:16.

In one preferred embodiment a bispecific antibody is provided comprising SEQ ID NO.:18, SEQ ID NO.:19 and SEQ ID NO.:20. In another embodiment, a bispecific antibody is provided comprising SEQ ID NO.:17, SEQ ID NO.:19 and SEQ ID NO.:20.

In another aspect, a bispecific antibody that binds to DR5 and FAP comprises at least one antigen binding site specific for DR5 comprising a heavy chain variable domain (VH) sequence having at least 90%, 91%, 92%, 93%, 94%, 95%, 96%, 97%, 98%, 99%, or 100% sequence identity to the amino acid sequence of SEQ ID NO.:7, and at least one antigen binding site specific for FAP comprising a variable heavy chain of SEQ ID NO.:15 and a variable light chain of SEQ ID NO.:16.

In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but a bispecific antibody that binds to DR5 and FAP comprising that sequence retains the ability to bind to FAP and DR5. In certain embodiments, a total of 1 to 10 amino acids have been substituted, inserted and/or deleted in SEQ ID NO.:7. In certain embodiments, substitutions, insertions, or deletions occur in regions outside the HVRs (i.e., in the FRs). Optionally, the bispecific antibody that binds to DR5 and FAP comprises the VH sequence in SEQ ID NO.:7, including post-translational modifications of that sequence.

In another aspect, a bispecific antibody that binds to DR5 and FAP comprises at least one antigen binding site specific for DR5 comprising a light chain variable domain (VL) sequence having at least 90%, 91%, 92%, 93%, 94%, 95%, 96%, 97%, 98%, 99%, or 100% sequence identity to the amino acid sequence of SEQ ID NO.:8, and at least one antigen binding site specific for FAP comprising a variable heavy chain of SEQ ID NO.:15 and a variable light chain of SEQ ID NO.:16.

In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but a bispecific antibody that binds to DR5 and FAP comprising that sequence retains the ability to bind to DR5 and FAP. In certain embodiments, a total of 1 to 10 amino acids have been substituted, inserted and/or deleted in SEQ ID NO.:8. In certain embodiments, the substitutions, insertions, or deletions occur in regions outside the HVRs (i.e., in the FRs). Optionally, the bispecific antibody that binds to DR5 and FAP comprises the VL sequence in SEQ ID NO:8, including post-translational modifications of that sequence.

In another aspect, a bispecific antibody that binds to DR5 and FAP is provided, comprising at least one antigen binding site specific for DR5 comprising a variable light chain of SEQ ID NO.:8 and a variable heavy chain of SEQ ID NO.:7; and at least one antigen binding site specific for FAP, comprising a heavy chain variable domain (VH) sequence having at least 90%, 91%, 92%, 93%, 94%, 95%, 96%, 97%, 98%, 99%, or 100% sequence identity to the amino acid sequence of SEQ ID NO.:15. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but a bispecific antibody that binds to DR5 and FAP comprising that sequence retains the ability to bind to FAP and DR5. In certain embodiments, a total of 1 to 10 amino acids have been substituted, inserted and/or deleted in SEQ ID NO.:15. In certain embodiments, substitutions, insertions, or deletions occur in regions outside the HVRs (i.e., in the FRs). Optionally, the bispecific antibody that binds to DR5 and FAP comprises the VH sequence in SEQ ID N0.:15, including post-translational modifications of that sequence.

In another aspect, a bispecific antibody that binds to DR5 and FAP is provided, comprising at least one antigen binding site specific for DR5, comprising a variable light chain of SEQ ID NO.:8 and a variable heavy chain of SEQ ID NO.:7, and at least one antigen binding site specific for FAP, comprising a light chain variable domain (VL) having at least 90%, 91%, 92%, 93%, 94%, 95%, 96%, 97%, 98%, 99%, or 100% sequence identity to the amino acid sequence of SEQ ID NO.:16. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but a bispecific antibody that binds to DR5 and FAP comprising that sequence retains the ability to bind to DR5 and FAP. In certain embodiments, a total of 1 to 10 amino acids have been substituted, inserted and/or deleted in SEQ ID NO.:16. In certain embodiments, the substitutions, insertions, or deletions occur in regions outside the HVRs (i.e., in the FRs). Optionally, the bispecific antibody that binds to DR5 and FAP comprises the VL sequence in SEQ ID NO:16, including post-translational modifications of that sequence.

In another aspect, a a bispecific antibody that binds to DR5 and FAP is provided, wherein the antibody comprises a VH as in any of the embodiments provided above, and a VL as in any of the embodiments provided above. In one embodiment, the antibody comprises the VH and VL sequences in SEQ ID NO:7 and SEQ ID NO:8, and SEQ ID NO:15 and SEQ ID NO:16, respectively, including post-translational modifications of those sequences.

In a further aspect of the invention, a bispecific antibody that binds to DR5 and FAP according to any of the

above embodiments is a monoclonal antibody, including a chimeric, humanized or human antibody. In one embodiment, said bispecific antibody that binds to DR5 and FAP according to any of the above embodiments is a human antibody.

**C. Exemplary formats of bispecific antibodies binding to DR5 and FAP**

In one embodiment, a bispecific antibody that binds to DR5 and FAP comprises an antibody fragment, e.g., a Fv, Fab, Fab', scFv,xFab, scFab, diabody, or $F(ab')_2$ fragment. In another embodiment, the antibody comprises a full length antibody, e.g., an intact IgG1 antibody or other antibody class or isotype as defined herein.

The bispecific antibodies according to the invention are at least bivalent and can be trivalent or multivalent e.g. tetravalent or hexavalent.

The bispecific antibody of the invention comprise an Fc domain, at least one Fab fragment comprising an antigen binding site specific for DR5, and at least one Fab fragment comprising an antigen binding site specific for FAP, wherein either the variable regions or the constant regions of the heavy and light chain of at least one Fab fragment are exchanged.

In another embodiment, the bispecific antibody comprises an Fc domain, at least one Fab fragment comprising an antigen binding site specific for DR5, and at least one Fab fragment comprising an antigen binding site specific for FAP, wherein at least one of the Fab fragments is connected to the first or second subunit of the Fc domain via the heavy chain (VHCH1).

In any of the embodiments, the Fab fragments may be fused to the Fc domain or to each other directly or through a peptide linker, comprising one or more amino acids, typically about 2-20 amino acids. Peptide linkers are known in the art and are described herein. Suitable, non-immunogenic peptide linkers include, for example, $(G_4S)_n$, $(SG_4)_n$, $(G_4S)_n$ or $G_4(SG_4)_n$ peptide linkers. "n" is generally a number between 1 and 10, typically between 2 and 4. A particularly suitable peptide linker for fusing the Fab light chains of the first and the second antigen binding moiety to each other is $(G_4S)_2$. An exemplary peptide linker suitable for connecting the Fab heavy chains of the first and the second antigen binding moiety is EPKSC(D)-$(G_4S)_2$. Additionally, linkers may comprise (a portion of) an immunoglobulin hinge region. In particular, where an antigen binding moiety is fused to the N-terminus of an Fc domain subunit, it may be fused via an immunoglobulin hinge region or a portion thereof, with or without an additional peptide linker.

Preferably, said bispecific antibodies are tetravalent with two binding sites each targeting FAP and DR5, respectively (2+2 format). In another embodiment said bispecific antibodies are tetravalent with three binding sites for DR5 and one binding site for FAP (3+1 format). The 3+1 format can be achieved, for example, through fusing one Fab fragment targeting FAP and one Fab fragment targeting DR5 to the C-terminus of the heavy chain of an IgG molecule that has two DR5 binding sites. This is outlined in more detail below.

In another preferred embodiment, said bispecific antibodies are trivalent (2+1 format) with two binding sites each targeting DR5 and one binding site targeting FAP. The 2+1 format can be achieved, for example, through fusing a Fab fragment targeting FAP to the C-terminus of the heavy chain of an IgG molecule that has two DR5 binding sites., wherein the Fc part of the first antibody is modified according to the knobs-into hole strategy as outlined below.

In another preferred embodiment, said bispecific antibodies are bivalent (1+1 format), i.e. monovalent for each DR5 and FAP. Bivalent antibodies of the invention have one binding site targeting DR5 and one binding site targeting FAP. The 1+1 format can be achieved, for example, by the Crossmab technology described in Schaefer et al. Proc Natl Acad Sci USA 2011; 108:11187-92 and as outlined below.

Provided therein are different bispecific antibody formats that are binding to DR5 and FAP comprising any of the sequences according to any of the above embodiments.

**1. Bispecific DR5-FAP antibodies in a 2+2 Format**

In one preferred embodiment a bispecific antibody that binds to DR5 and FAP according to any of the above embodiments comprises
an Fc domain,
two Fab fragments comprising each an antigen binding site specific for DR5, and
two Fab fragments comprising each an antigen binding site specific for FAP, wherein either the variable regions or the constant regions of the heavy and light chain of at least one Fab fragment are exchanged.

Since the above bispecific antibody is bivalent both for FAP and DR5, with 2 binding sites each for FAP and DR5, this format is also referred to as "2+2" format. Exemplary structures of bispecific antibodies with a 2+2 format are depicted in Figure 1. Due to the exchange of either the variable regions or the constant regions, the Fab fragments above are also referred to as "cross-Fab fragment" or "xFab fragment" or "crossover Fab fragment".

In one preferred embodiment a bispecific antibody that binds to DR5 and FAP according to any of the above embodiments comprises
an Fc domain,

two Fab fragments comprising each an antigen binding site specific for DR5, and
two Fab fragments comprising each an antigen binding site specific for FAP, wherein either the variable regions or the constant regions of the heavy and light chain of both Fab fragments comprising an antigen binding site specific for FAP are exchanged.

In another embodiment a bispecific antibody that binds to DR5 and FAP according to any of the above embodiments comprises
an Fc domain,
two Fab fragments comprising each an antigen binding site specific for DR5, and
two Fab fragments comprising each an antigen binding site specific for FAP,
wherein either the variable regions or the constant regions of the heavy and light chain of both Fab fragments comprising an antigen binding site specific for DR5 are exchanged.

In one embodiment a bispecific antibody that binds to DR5 and FAP according to any of the above embodiments comprises
an Fc domain,
two Fab fragments comprising each an antigen binding site specific for DR5, and
two Fab fragments comprising each an antigen binding site specific for FAP, wherein the variable regions of the heavy and light chain of both Fab fragments comprising an antigen binding site specific for FAP are exchanged.

In another embodiment a bispecific antibody that binds to DR5 and FAP according to any of the above embodiments comprises
an Fc domain,
two Fab fragments comprising each an antigen binding site specific for DR5, and
two Fab fragments comprising each an antigen binding site specific for FAP,
wherein the variable regions of the heavy and light chain of both Fab fragments comprising an antigen binding site specific for DR5 are exchanged.

Due to the exchange of the variable regions of the Fab heavy and light chain the crossover Fab fragments specific for FAP each comprise a peptide chain composed of the light chain variable region (VL) and the heavy chain constant region (CH1), and a peptide chain composed of the heavy chain variable region (VH) and the light chain constant region (CL). These crossover Fab fragments are also referred to as $CrossFab_{(VLVH)}$ and each comprise a VLCH1 and a VHCL chain.

In one preferred embodiment a bispecific antibody that binds to DR5 and FAP according to any of the above embodiments comprises
an Fc domain,
two Fab fragments comprising each an antigen binding site specific for DR5, and
two Fab fragments comprising each an antigen binding site specific for FAP, wherein the constant regions of the heavy and light chain of both Fab fragments comprising an antigen binding site specific for FAP are exchanged.

In another embodiment a bispecific antibody that binds to DR5 and FAP according to any of the above embodiments comprises
an Fc domain,
two Fab fragments comprising each an antigen binding site specific for DR5, and
two Fab fragments comprising each an antigen binding site specific for FAP, wherein the constant regions of the heavy and light chain of both Fab fragments comprising an antigen binding site specific for DR5 are exchanged.

Due to the exchange of the constant regions of the Fab heavy and light chain the crossover Fab fragments specific for FAP each comprise a peptide chain composed of the heavy chain variable region (VH) and the light chain constant region (CL), and a peptide chain composed of the light chain variable region (VL) and the heavy chain constant region (CH1). These crossover Fab fragments are also referred to as $CrossFab_{(CLCH1)}$ and comprise a VHCL and a VLCH1 chain.

In one embodiment, said bispecific antibody that binds to DR5 and FAP according to any of the above embodiments comprises an Fc domain to which two Fab fragments are fused to the N-terminus and two Fab fragments are fused to the C-terminus, wherein either the variable regions or the constant regions of the heavy and light chain of at least one Fab fragment are exchanged. In one embodiment, two Fab fragments are fused to the N-terminus of the Fc domain through an immunoglobulin hinge region. In one embodiment, the immunoglobulin hinge region is a human IgG1 hinge region. In one embodiment, the two Fab fragments comprising an antigen binding site specific for DR5 and the Fc domain are part of an immunoglobulin molecule. In a particular embodiment the immunoglobulin molecule is an IgG class immunoglobulin. In an even more particular embodiment, the immunoglobulin is an IgG1 subclass immunoglobulin. In another embodiment, the immunoglobulin is an IgG4 subclass immunoglobulin. In a further particular embodiment, the immunoglobulin is a human immunoglobulin. In other embodiments the immunoglobulin is a chimeric immunoglobulin or a humanized immunoglobulin.

In one embodiment, two Fab fragments comprising the antigen binding site specific for FAP are connected to

the Fc domain via a peptide linker. In one embodiment two Fab fragments comprising an antigen binding site specific for FAP are connected to the C-terminus of the first or second subunit of the Fc domain via a peptide linker. In one such embodiment, said Fab fragments comprising an antigen binding site specific for FAP are connected to the C-terminus of the second subunit (CH3 chain) of the Fc domain via a peptide linker.

In one embodiment, said bispecific antibody that binds to DR5 and FAP according to any of the above embodiments comprises

a) an Immunoglobulin G (IgG) molecule with two binding sites specific for DR5 (i.e. two Fab fragments specific for DR5) and
b) two Fab fragments specific for FAP, wherein either the variable regions or the constant regions of the heavy and light chain are exchanged.

In another embodiment, a bispecific antibody that binds to DR5 and FAP according to any of the above embodiments comprises

a) an Immunoglobulin G (IgG) molecule with two binding sites (Fab fragments) specific for DR5 wherein either the variable regions or the constant regions of the heavy and light chain are exchanged and
b) two Fab fragments specific for FAP.

In one embodiment, a bispecific antibody that binds to DR5 and FAP according to any of the above embodiments comprises

a) an Immunoglobulin G (IgG) molecule with two binding sites specific for DR5 and

b) two Fab fragments specific for FAP, wherein the variable regions of the Fab heavy and light chain are exchanged.

In one embodiment a bispecific antibody that binds to DR5 and FAP according to any of the above embodiments comprises

a) an Immunoglobulin G (IgG) molecule with two binding sites specific for DR5 and

b) two Fab fragments specific for FAP, wherein the constant regions of the Fab heavy and light chain are exchanged.

In one embodiment a bispecific antibody that binds to DR5 and FAP according to any of the above embodiments comprises

a) an Immunoglobulin G (IgG) molecule with two binding sites specific for DR5 and

b) two Fab fragments specific for FAP, wherein either the variable regions or the constant regions of the heavy and light chain are exchanged, wherein the two Fab fragments are fused to the constant heavy chain of said IgG molecule.

In one embodiment, said two Fab fragments are fused to the C-terminus of the constant heavy chain of said IgG molecule. In one embodiment said two Fab fragments are fused to the N-terminus of the variable heavy chain (VH) to the first or second subunit of the Fc domain of said IgG molecule.

In one embodiment, said two Fab fragments are fused to the C-terminus of the second subunit (CH3) of the Fc domain of said IgG molecule.

In one embodiment, a bispecific antibody that binds to DR5 and FAP according to any of the above embodiments comprises

a) an Immunoglobulin G (IgG) molecule with two binding sites specific for DR5; and
b) two Fab fragments specific for FAP, wherein the variable regions of the Fab heavy and light chain are exchanged, wherein the two Fab fragments are fused to the constant heavy chain of said IgG molecule.

In one embodiment said two Fab fragments are fused to the C-terminus of the constant heavy chain of said IgG molecule. In one embodiment said two Fab fragments are fused to the C-terminus of the constant heavy chain (CH1) to the first or second subunit of the Fc domain of said IgG molecule. In one embodiment said two Fab fragments are fused to the C-terminus of the second subunit (CH3) of the Fc domain of said IgG molecule.

In one embodiment, a bispecific antibody that binds to DR5 and FAP according to any of the above embodiments

comprises

a) an Immunoglobulin G (IgG) molecule with two binding sites specific for DR5 and
b) two Fab fragments specific for FAP, wherein the constant regions of the Fab heavy and light chain are exchanged, wherein the two Fab fragments are fused to the constant heavy chain of said IgG molecule.

In one embodiment, said two Fab fragments are fused to the C-terminus of the constant heavy chain of said IgG molecule. In one embodiment, said two Fab fragments are fused to the C-terminus of the constant heavy chain (CH1) to the first or second subunit of the Fc domain of said IgG molecule. In one embodiment said two Fab fragments are fused to the C-terminus of the second subunit (CH3) of the Fc domain of said IgG molecule.
In a further preferred embodiment, the two Fab fragments specific for FAP are fused to the IgG molecule by a peptide linker, preferably a peptide linker having a length of about 10-30 amino acids. Preferably, said peptide linker is a $(G_4S)_2$ or $(G_4S)_4$ linker.
In one embodiment, a bispecific antibody that binds to DR5 and FAP according to any of the above embodiments comprises

a) an Immunoglobulin G (IgG) molecule with two binding sites specific for DR5 and
b) two Fab fragments specific for FAP, wherein the variable regions of the Fab heavy and light chain are exchanged, wherein the two Fab fragments are fused to the constant heavy chain of said IgG molecule by a peptide linker.

In one embodiment said two Fab fragments are fused to the C-terminus of the constant heavy chain of said IgG molecule by a peptide linker. In one embodiment, said two Fab fragments are fused to the C-terminus of the constant heavy chain (CH1) to the first or second subunit of the Fc domain of said IgG molecule by a peptide linker.
In one embodiment, said two Fab fragments are fused to the C-terminus of the second subunit (CH3) of the Fc domain of said IgG molecule by a peptide linker.
In one embodiment, a bispecific antibody that binds to DR5 and FAP according to any of the above embodiments comprises

a) an Immunoglobulin G (IgG) molecule with two binding sites specific for DR5 and
b) two Fab fragments specific for FAP, wherein the constant regions of the Fab heavy and light chain are exchanged, wherein the two Fab fragments are fused to the constant heavy chain of said IgG molecule by a peptide linker.

In one embodiment, said two Fab fragments are fused to the C-terminus of the constant heavy chain of said IgG molecule by a peptide linker.
In one embodiment, said two Fab fragments are fused at the N-terminus of the variable heavy chain (VH) to the first or second subunit of the Fc domain of said IgG molecule by a peptide linker.
In one embodiment, said two Fab fragments are fused to the C-terminus of the second subunit (CH3) of the Fc domain of said IgG molecule by a peptide linker.
In one embodiment, the bispecific antibody comprises an Fc domain, two Fab fragments comprising the antigen binding site specific for DR5, and two Fab fragments comprising the antigen binding site specific for FAP, wherein at least one of the Fab fragments is fused to the first or second subunit of the Fc domain via the heavy chain (VHCH1).
In one aspect, the bispecific antibody comprises:

a) an Fc domain,
b) two Fab fragments comprising an antigen binding site specific for DR5,
wherein said Fab fragments are connected at the C-terminus of the constant heavy chain (CH1) to the first or second subunit of the Fc domain, and
c) two Fab fragments comprising the antigen binding site specific for FAP, wherein the two Fab fragments are connected at the N-terminus of the variable heavy chain (VH) to the first or second subunit of the Fc domain.

In one aspect, the bispecific antibody comprises:

a) an Fc domain,
b) two Fab fragments comprising an antigen binding site specific for DR5,
wherein said Fab fragments are connected at the C-terminus of the constant heavy chain (CH1) to the N-terminus of the first subunit of the Fc domain, and
c) two Fab fragments comprising the antigen binding site specific for FAP,
wherein the two Fab fragments are connected at the N-terminus of the variable light chain (VL) to the second subunit

(CH3) of the Fc domain.

In one embodiment, said two Fab fragments comprising an antigen binding site specific for DR5 are each fused to the Fc domain through an immunoglobulin hinge region. I n a specific embodiment, the immunoglobulin hinge region is a human IgG1 hinge region. In one embodiment the two Fab fragments comprising an antigen binding site specific for DR5 and the Fc domain are part of an immunoglobulin molecule. In a particular embodiment, the immunoglobulin molecule is an IgG class immunoglobulin. In an even more particular embodiment, the immunoglobulin is an IgG1 subclass immunoglobulin. In another embodiment, the immunoglobulin is an IgG4 subclass immunoglobulin. In a further particular embodiment, the immunoglobulin is a human immunoglobulin. In other embodiments, the immunoglobulin is a chimeric immunoglobulin or a humanized immunoglobulin.

In one embodiment two Fab fragments comprising the antigen binding site specific for FAP are connected to the Fc domain via a peptide linker.

**Exemplary antibodies with a 2+2 format**

In one embodiment a bispecific antibody that binds to DR5 and FAP according to any of the above embodiments comprises

a) an Immunoglobulin G (IgG) molecule with two binding sites specific for DR5, comprising

a heavy chain CDR1 of SEQ ID NO.:1;
a heavy chain CDR2 of SEQ ID NO.:2;
a heavy chain CDR3 of SEQ ID NO.:3;
a light chain CDR1 of SEQ ID NO.:4;
a light chain CDR2 of SEQ ID NO.:5;
a light chain CDR3 of SEQ ID NO.:6; and

b) two Fab fragments specific for FAP, comprising

a heavy chain CDR1 of SEQ ID NO.:9;
a heavy chain CDR2 of SEQ ID NO.:10;
a heavy chain CDR3 of SEQ ID NO.:11;
a light chain CDR1 of SEQ ID NO.:12;
a light chain CDR2 of SEQ ID NO.:13;
a light chain CDR3 of SEQ ID NO.:14;

wherein the constant regions of the Fab heavy and light chain are exchanged, wherein the two Fab fragments are fused to the constant heavy chain of said IgG molecule by a peptide linker.

In one embodiment a bispecific antibody that binds to DR5 and FAP according to any of the above embodiments comprises

a) an Immunoglobulin G (IgG) molecule with two binding sites specific for DR5, comprising

a heavy chain CDR1 of SEQ ID NO.:1;
a heavy chain CDR2 of SEQ ID NO.:2;
a heavy chain CDR3 of SEQ ID NO.:3;
a light chain CDR1 of SEQ ID NO.:4;
a light chain CDR2 of SEQ ID NO.:5; and
a light chain CDR3 of SEQ ID NO.:6; and

b) two Fab fragments specific for FAP, comprising

a heavy chain CDR1 of SEQ ID NO.:9;
a heavy chain CDR2 of SEQ ID NO.:10;
a heavy chain CDR3 of SEQ ID NO.:11;
a light chain CDR1 of SEQ ID NO.:12;
a light chain CDR2 of SEQ ID NO.:13; and
a light chain CDR3 of SEQ ID NO.:14;

wherein the variable regions of the Fab heavy and light chain are exchanged, wherein the two Fab fragments are fused to the constant heavy chain of said IgG molecule by a peptide linker.

In one embodiment a bispecific antibody that binds to DR5 and FAP according to any of the above embodiments comprises

a) an Immunoglobulin G (IgG) molecule with two binding sites specific for DR5,
comprising a variable heavy chain of SEQ ID NO.:7 and a variable light chain of SEQ ID NO.:8; and
b) two Fab fragments specific for FAP, comprising a heavy chain variable region comprising an amino acid sequence of SEQ ID NO.:15 and a light chain variable region comprising an amino acid sequence of SEQ ID NO.:16; wherein the constant regions of the Fab heavy and light chain are exchanged, wherein the two Fab fragments are fused to the constant heavy chain of said IgG molecule by a peptide linker.

In one embodiment, a bispecific antibody that binds to DR5 and FAP according to any of the above embodiments comprises

a) an Immunoglobulin G (IgG) molecule with two binding sites specific for DR5,
comprising a variable heavy chain of SEQ ID NO.:7 and a variable light chain of SEQ ID NO.:8; and
b) two Fab fragments specific for FAP, comprising a heavy chain variable region comprising an amino acid sequence of SEQ ID NO.:15 and a light chain variable region comprising an amino acid sequence of SEQ ID NO.:16; wherein the variable regions of the Fab heavy and light chain are exchanged, wherein the two Fab fragments are fused to the constant heavy chain of said IgG molecule by a peptide linker.

In another embodiment, said bispecific antibody of the invention comprises a modification in the Fc part of the IgG molecule, as outlined below.

In one embodiment, a bispecific antibody with a 2+2 format as described above is provided comprising two $VH_{(DR5)}$-Fc part - $VH_{(FAP)}$-CL chains of SEQ ID NO.:18, two VL (DR5)-kappa light chains of SEQ ID NO.:19 and two VLCH1 (FAP) chains of SEQ ID NO.:20.

In one embodiment, a bispecific antibody with a 2+2 format as described above is provided comprising two $VH_{(DR5)}$-Fc part - $VH_{(FAP)}$-CL chains of SEQ ID NO.:17, two VL (DR5)-kappa light chains of SEQ ID NO.:19 and two VLCH1 (FAP) chains of SEQ ID NO.:20.

**2. Bispecific DR5-FAP antibodies in a 2+1 Format**

In one embodiment, a bispecific antibody that binds to DR5 and FAP according to any of the above embodiments comprises
an Fc domain,
two Fab fragments comprising an antigen binding site specific for DR5, and
one Fab fragment comprising the antigen binding site specific for FAP, wherein either the variable regions or the constant regions of the heavy and light chain of at least one Fab fragment are exchanged.

Since the above bispecific antibodies are trivalent with one binding site for FAP and two binding sites for DR5, this format is also referred to as "2+1" format. Hence the bispecific antibodies provided in this section are bivalent for DR5 and monovalent for FAP. Due to the exchange of either the variable regions or the constant regions, the Fab fragments above are also referred to as "cross-Fab fragment" or "xFab fragment" or "crossover Fab fragment".

In one embodiment, a bispecific antibody that binds to DR5 and FAP according to any of the above embodiments comprises
an Fc domain,
two Fab fragments comprising each an antigen binding site specific for DR5, and
one Fab fragment comprising an antigen binding site specific for FAP, wherein either the variable regions or the constant regions of the heavy and light chain of the Fab fragments comprising an antigen binding site specific for FAP are exchanged.

In one embodiment, a bispecific antibody that binds to DR5 and FAP according to any of the above embodiments comprises
an Fc domain,
two Fab fragments comprising each an antigen binding site specific for DR5, and
one Fab fragment comprising an antigen binding site specific for FAP, wherein the variable regions of the heavy and light chain of the Fab fragment comprising an antigen binding site specific for FAP are exchanged.

Due to the exchange of the variable regions of the Fab heavy and light chain the crossover Fab fragment specific for FAP each comprise a peptide chain composed of the light chain variable region (VL) and the heavy chain

constant region (CH1), and a peptide chain composed of the heavy chain variable region (VH) and the light chain constant region (CL). These crossover Fab fragment is also referred to as $CrossFab_{(VLVH)}$ and comprises a VLCH1 and a VHCL chain.

In one embodiment a bispecific antibody that binds to DR5 and FAP according to any of the above embodiments comprises
an Fc domain,
two Fab fragments comprising each an antigen binding site specific for DR5, and
one Fab fragment comprising an antigen binding site specific for FAP, wherein the constant regions of the heavy and light chain of both Fab fragments comprising the antigen binding site specific for FAP are exchanged.

Due to the exchange of the constant regions of the Fab heavy and light chain the crossover Fab fragment specific for FAP each comprise a peptide chain composed of the heavy chain variable region (VH) and the light chain constant region (CL), and a peptide chain composed of the light chain variable region (VL) and the heavy chain constant region (CH1). These crossover Fab fragments is also referred to as $CrossFab_{(CLCH1)}$ and comprises a VHCL and a VLCH1 chain.

In one embodiment, said bispecific antibody that binds to DR5 and FAP according to any of the above embodiments comprises an Fc domain to which two Fab fragments are fused to the N-terminus.

In one embodiment, two Fab fragments are fused to the N-terminus of the Fc domain through an immunoglobulin hinge region. In one embodiment, the immunoglobulin hinge region is a human IgG1 hinge region. In a particular embodiment, the immunoglobulin molecule is an IgG class immunoglobulin. In an even more particular embodiment, the immunoglobulin is an IgG1 subclass immunoglobulin. In another embodiment, the immunoglobulin is an IgG4 subclass immunoglobulin. In a further particular embodiment, the immunoglobulin is a human immunoglobulin. In other embodiments, the immunoglobulin is a chimeric immunoglobulin or a humanized immunoglobulin.

**Bispecific DR5-FAP antibodies in a 2+1 Format with FAP binder fused to C-terminus**

In one embodiment, the two Fab fragments comprising an antigen binding site specific for DR5 and the Fc domain are part of an immunoglobulin molecule. In one embodiment one Fab fragment comprising the antigen binding site specific for FAP is fused to the C-terminus of the first or second subunit of the Fc domain via a peptide linker. In one such embodiment, said Fab fragment comprising an antigen binding site specific for FAP is fused to the C-terminus of the second subunit (CH3 chain) of the Fc domain via a peptide linker.

In one embodiment, a bispecific antibody that binds to DR5 and FAP according to any of the above embodiments comprises:

a) an Immunoglobulin G (IgG) molecule with two binding sites (Fab fragments) specific for DR5 and
b) one Fab fragment specific for FAP, wherein either the variable regions or the constant regions of the heavy and light chain are exchanged.

In one embodiment, a bispecific antibody that binds to DR5 and FAP according to any of the above embodiments comprises:

a) an Immunoglobulin G (IgG) molecule with two binding sites specific for DR5 and

b) one Fab fragment specific for FAP, wherein the variable regions of the Fab heavy and light chain are exchanged.

In one embodiment, a bispecific antibody that binds to DR5 and FAP according to any of the above embodiments comprises:

a) an Immunoglobulin G (IgG) molecule with two binding sites specific for DR5 and

b) one Fab fragment specific for FAP, wherein the constant regions of the Fab heavy and light chain are exchanged.

In one preferred embodiment, a bispecific antibody that binds to DR5 and FAP according to any of the above embodiments comprises:

a) an Immunoglobulin G (IgG) molecule with two binding sites specific for DR5 and

b) one Fab fragment specific for FAP, wherein either the variable regions or the constant regions of the heavy and light chain are exchanged, wherein the Fab fragment is fused to the constant heavy chain of said IgG molecule.

In one embodiment said Fab fragment specific for FAP of b) is fused to the C-terminus of the first or second subunit of the Fc domain of said IgG molecule. In one embodiment said Fab fragment of b) is fused to the C-terminus of the second subunit (CH3) of the Fc domain of said IgG molecule.

In one embodiment, a bispecific antibody that binds to DR5 and FAP according to any of the above embodiments comprises:

a) an Immunoglobulin G (IgG) molecule with two binding sites specific for DR5 and

b) one Fab fragment specific for FAP, wherein the variable regions of the Fab heavy and light chain are exchanged, wherein the Fab fragment is fused to the constant heavy chain of said IgG molecule.

In one embodiment said Fab fragment specific for FAP of b) is fused to the C-terminus of the first or second subunit of the Fc domain of said IgG molecule. In one embodiment said Fab fragment of b) is fused to the C-terminus of the second subunit (CH3) of the Fc domain of said IgG molecule.

In one embodiment, a bispecific antibody that binds to DR5 and FAP according to any of the above embodiments comprises:

a) an Immunoglobulin G (IgG) molecule with two binding sites specific for DR5 and

b) one Fab fragment specific for FAP, wherein the constant regions of the Fab heavy and light chain are exchanged, wherein the Fab fragment is fused to the constant heavy chain of said IgG molecule.

In one embodiment, said Fab fragment specific for FAP of b) is fused to the C-terminus of the first or second subunit of the Fc domain of said IgG molecule. In one embodiment said Fab fragment of b) is fused to the C-terminus of the second subunit (CH3) of the Fc domain of said IgG molecule.

**Bispecific DR5-FAP antibodies in a 2+1 Format with FAP binder fused to the N-terminus**

In another embodiment, one Fab fragment comprising an antigen binding site specific for DR5, one Fab fragment comprising an antigen binding site specific for FAP and the Fc domain are part of an immunoglobulin molecule. In one embodiment, another Fab fragment comprising an antigen binding site specific for DR5 is fused to the N-terminus of the Fab fragment comprising an antigen binding site specific for DR5 of the IgG molecule via a peptide linker.

In one embodiment, a bispecific antibody that binds to DR5 and FAP according to any of the above embodiments comprises:

a) an Immunoglobulin G (IgG) molecule with one binding site (Fab fragment) specific for DR5 and one binding site (Fab fragment) specific for FAP, wherein the Fab fragment specific for FAP is a Crossfab fragment (i.e. either the variable regions or the constant regions of the heavy and light chain are exchanged); and
b) one Fab fragment specific for DR5.

In one embodiment, a bispecific antibody that binds to DR5 and FAP according to any of the above embodiments comprises:

a) an Immunoglobulin G (IgG) molecule with one binding site specific for DR5 and one binding site specific for FAP, wherein the Fab fragment specific for FAP is a $CrossFab_{(VLVH)}$ fragment (i.e. the variable regions of the heavy and light chain are exchanged); and
b) one Fab fragment specific for DR5.

In one embodiment, a bispecific antibody that binds to DR5 and FAP according to any of the above embodiments comprises:

a) an Immunoglobulin G (IgG) molecule with one binding site specific for DR5 and one binding site specific for FAP, wherein the Fab fragment specific for FAP is a $CrossFab_{(CLCH1}$ fragment (i.e. the constant regions of the heavy and light chain are exchanged); and
b) one Fab fragment specific for DR5.

In one embodiment, a bispecific antibody that binds to DR5 and FAP according to any of the above embodiments comprises:

a) an Immunoglobulin G (IgG) molecule with one binding site specific for DR5 and one binding site specific for FAP, wherein the Fab fragment specific for FAP is a Crossfab fragment (i.e. either the variable regions or the constant regions of the heavy and light chain are exchanged); and
b) one Fab fragment specific for DR5, wherein said Fab fragment is fused to the N-terminus of the variable heavy or light chain of the IgG molecule.

In one embodiment, a bispecific antibody that binds to DR5 and FAP according to any of the above embodiments comprises:

a) an Immunoglobulin G (IgG) molecule with one binding site (Fab fragment) specific for DR5 and one binding site specific for FAP, wherein the Fab fragment specific for FAP is a $CrossFab_{(VLVH)}$ fragment (i.e. the variable regions of the heavy and light chain are exchanged); and
b) one Fab fragment specific for DR5, wherein said Fab fragment is fused to the N-terminus of the variable heavy or light chain of the IgG molecule.

In one embodiment, the Fab fragment specific for DR5 of b) is fused to the N-terminus of the variable heavy or light chain of the Fab fragment specific for DR5 of a).
In one embodiment, a bispecific antibody that binds to DR5 and FAP according to any of the above embodiments comprises :

a) an Immunoglobulin G (IgG) molecule with one binding site specific for DR5 and one binding site specific for FAP, wherein the Fab fragment specific for FAP is a $CrossFab_{(CLCH1}$ fragment (i.e. the constant regions of the heavy and light chain are exchanged); and
b) one Fab fragment specific for DR5, wherein said Fab fragment is fused to the N-terminus of the variable heavy or light chain of the IgG molecule

In one embodiment, the Fab fragment specific for DR5 of b) is fused to the N-terminus of the variable heavy or light chain of the Fab fragment specific for DR5 of a).
In a further preferred embodiment, the Fab fragment specific for DR5 is fused to the IgG molecule by a peptide linker, preferably a peptide linker having a length of about 10-30 amino acids. Preferably said peptide linker is a $(G_4S)_2$ or $(G_4S)_4$ linker.
In another embodiment, said bispecific antibody of the invention comprises a modification in the Fc part of the IgG molecule, as outlined below.

### 3. Bispecific DR5-FAP antibodies in a 3+1 Format

In one embodiment, a bispecific antibody that binds to DR5 and FAP according to any of the above embodiments comprises:

an Fc domain,
three Fab fragments comprising each an antigen binding site specific for DR5, and
one Fab fragment comprising an antigen binding site specific for FAP,

wherein either the variable regions or the constant regions of the heavy and light chain of at least one Fab fragment are exchanged.
Since the above bispecific antibody is tetravalent with one binding site for FAP and three binding sites for DR5, this format is also referred to as "3+1" format. Hence the bispecific molecules described in this section are trivalent for DR5 and monovalent for FAP. Due to the exchange of either the variable regions or the constant regions, the Fab fragments above are also referred to as "cross-Fab fragment" or "xFab fragment" or "crossover Fab fragment".
In one embodiment, a bispecific antibody that binds to DR5 and FAP according to any of the above embodiments comprises
an Fc domain,
three Fab fragments comprising each an antigen binding site specific for DR5, and
one Fab fragment comprising an antigen binding site specific for FAP, wherein either the variable regions or the constant regions of the heavy and light chain of the Fab fragments comprising an antigen binding site specific for FAP are exchanged.
In one embodiment a bispecific antibody that binds to DR5 and FAP according to any of the above embodiments comprises

an Fc domain,
three Fab fragments comprising each an antigen binding site specific for DR5, and
one Fab fragment comprising an antigen binding site specific for FAP, wherein the variable regions of the heavy and light chain of the Fab fragment comprising an antigen binding site specific for FAP are exchanged.

Due to the exchange of the variable regions of the Fab heavy and light chain the crossover Fab fragment specific for FAP comprises a peptide chain composed of the light chain variable region (VL) and the heavy chain constant region (CH1), and a peptide chain composed of the heavy chain variable region (VH) and the light chain constant region (CL). This crossover Fab fragments is also referred to as $CrossFab_{(VLVH)}$ and comprises a VLCH1 and a VHCL chain.

In one embodiment a bispecific antibody that binds to DR5 and FAP according to any of the above embodiments comprises
an Fc domain,
three Fab fragments comprising each an antigen binding site specific for DR5, and
one Fab fragment comprising an antigen binding site specific for FAP, wherein the constant regions of the heavy and light chain of the Fab fragment comprising the antigen binding site specific for FAP are exchanged.

Due to the exchange of the constant regions of the Fab heavy and light chain the crossover Fab fragment specific for FAP each comprise a peptide chain composed of the heavy chain variable region (VH) and the light chain constant region (CL), and a peptide chain composed of the light chain variable region (VL) and the heavy chain constant region (CH1). This crossover Fab fragment is also referred to as $CrossFab_{(CLCH1)}$ and comprise a VHCL and a VLCH1 chain.

In one embodiment, said bispecific antibody that binds to DR5 and FAP according to any of the above embodiments comprises an Fc domain to which two Fab fragments are fused to the N terminus and two Fab fragments are fused to the C-terminus, wherein either the variable regions or the constant regions of the heavy and light chain of the one Fab fragment specific fro FAP are exchanged.

In one embodiment, two Fab fragments are fused to the N-terminus of the Fc domain through an immunoglobulin hinge region. In one embodiment, the immunoglobulin hinge region is a human IgG1 hinge region. In one embodiment, the two Fab fragments comprising an antigen binding site specific for DR5 and the Fc domain are part of an immunoglobulin molecule. In a particular embodiment, the immunoglobulin molecule is an IgG class immunoglobulin. In an even more particular embodiment, the immunoglobulin is an IgG1 subclass immunoglobulin. In another embodiment, the immunoglobulin is an IgG4 subclass immunoglobulin. In a further particular embodiment, the immunoglobulin is a human immunoglobulin. In other embodiments, the immunoglobulin is a chimeric immunoglobulin or a humanized immunoglobulin.

In one embodiment, a bispecific antibody that binds to DR5 and FAP according to any of the above embodiments comprises:

a) an Immunoglobulin G (IgG) molecule with two binding sites (Fab fragments) specific for DR5,
b) one Fab fragment specific for FAP, wherein either the variable regions or the constant regions of the heavy and light chain are exchanged; and
c) one Fab fragment specific for DR5.

In one embodiment, a bispecific antibody that binds to DR5 and FAP according to any of the above embodiments comprises:

a) an Immunoglobulin G (IgG) molecule with two binding sites (Fab fragments) specific for DR5,
b) one Fab fragment specific for FAP, wherein the variable regions of the Fab heavy and light chain are exchanged; and
c) one Fab fragment specific for DR5.

Optionally a bispecific antibody that binds to DR5 and FAP according to any of the above embodiments comprises:

a) an Immunoglobulin G (IgG) molecule with two binding sites (Fab fragments) specific for DR5,
b) one Fab fragment specific for FAP, wherein the constant regions of the Fab heavy and light chain are exchanged; and
c) one Fab fragment specific for DR5.

In one preferred embodiment, a bispecific antibody that binds to DR5 and FAP according to any of the above embodiments comprises:

a) an Immunoglobulin G (IgG) molecule with two binding sites (Fab fragments) specific for DR5,
b) one Fab fragment specific for FAP, wherein either the variable regions or the constant regions of the heavy and light chain are exchanged; and
c) one Fab fragment specific for DR5,

wherein the Fab fragments of b) and c) are fused to the C-terminus of the first or second subunit of the Fc domain of said IgG molecule.

In one embodiment, said two Fab fragments of b) and c) are fused to the C-terminus of the second subunit (CH3) of the Fc domain of said IgG molecule.

In one embodiment, a bispecific antibody that binds to DR5 and FAP according to any of the above embodiments comprises:

a) an Immunoglobulin G (IgG) molecule with two binding sites (Fab fragments) specific for DR5,
b) one Fab fragment specific for FAP, wherein the variable regions of the Fab heavy and light chain are exchanged; and
c) one Fab fragment specific for DR5,

wherein the Fab fragments of b) and c) are fused to the first or second subunit of the Fc domain of said IgG molecule.

In one embodiment, said two Fab fragments of b) and c) are fused to the C-terminus of the second subunit (CH3) of the Fc domain of said IgG molecule.

In one embodiment, a bispecific antibody that binds to DR5 and FAP according to any of the above embodiments comprises:

a) an Immunoglobulin G (IgG) molecule with two binding sites (Fab fragments) specific for DR5,
b) one Fab fragment specific for FAP, wherein the constant regions of the Fab heavy and light chain are exchanged, and
c) one Fab fragment specific for DR5,

wherein the Fab fragments of b) and c) are fused to the first or second subunit of the Fc domain of said IgG molecule.

In one embodiment, said two Fab fragments of b) and c) are fused to the C-terminus of the second subunit (CH3) of the Fc domain of said IgG molecule.

In a further preferred embodiment, the two Fab fragments of b) and c) of any of the embodiments described in this section are fused to the IgG molecule by a peptide linker, preferably a peptide linker having a length of about 10 - 30 amino acids. Preferably said peptide linker is a $(G_4S)_2$ or $(G_4S)_4$ linker.

In another embodiment, said bispecific antibody of the invention comprises a modification in the Fc part of the IgG molecule, as outlined below.

**4. Bispecific DR5-FAP antibodies in a 1+1 Format**

In one embodiment, a bispecific antibody that binds to DR5 and FAP according to any of the above embodiments comprises

an Fc domain,
one Fab fragment comprising an antigen binding site specific for DR5, and
one Fab fragment comprising an antigen binding site specific for FAP,

wherein either the variable regions or the constant regions of the heavy and light chain of at least one Fab fragment are exchanged.

Since the above bispecific antibody is bivalent with one binding site for FAP and one binding site for DR5, this format is also referred to as "1+1" format. Hence the bispecific antibodies described in this section are monovalent for DR5 and monovalent for FAP. Due to the exchange of either the variable regions or the constant regions, the Fab fragment above is also referred to as "cross-Fab fragment" or "xFab fragment" or "crossover Fab fragment". The IgG molecule in a 1+1 format is also referred to as Crossmab format (see Schaefer et al. Proc Natl Acad Sci USA 2011; 108:11187-92).

In one embodiment, a bispecific antibody that binds to DR5 and FAP according to any of the above embodiments comprises:

an Fc domain,

one Fab fragment comprising an antigen binding site specific for DR5, wherein either the variable regions or the constant regions of the heavy and light chain of the Fab fragment comprising an antigen binding site specific for DR5 are exchanged, and
and one Fab fragment comprising an antigen binding site specific for FAP.

In one embodiment, a bispecific antibody that binds to DR5 and FAP according to any of the above embodiments comprises:

an Fc domain,
one Fab fragment comprising an antigen binding site specific for DR5, and
one Fab fragment comprising an antigen binding site specific for FAP, wherein either the variable regions or the constant regions of the heavy and light chain of the Fab fragment comprising an antigen binding site specific for FAP are exchanged.

In one embodiment, a bispecific antibody that binds to DR5 and FAP according to any of the above embodiments comprises:

an Fc domain,
one Fab fragment comprising an antigen binding site specific for DR5, and
one Fab fragment comprising an antigen binding site specific for FAP, wherein the variable regions of the heavy and light chain of the Fab fragment comprising an antigen binding site specific for FAP are exchanged.

In one embodiment, a bispecific antibody that binds to DR5 and FAP according to any of the above embodiments comprises:

an Fc domain,
one Fab fragments comprising an antigen binding site specific for DR5, and
one Fab fragment comprising an antigen binding site specific for FAP, wherein the constant regions of the heavy and light chain of the Fab fragment comprising the antigen binding site specific for FAP are exchanged.

In one embodiment, said bispecific antibody that binds to DR5 and FAP according to any of the above embodiments comprises an Fc domain to which two Fab fragments are fused to the N-terminus, wherein either the variable regions or the constant regions of the heavy and light chain of at least one Fab fragment are exchanged. In one embodiment, the two Fab fragments are fused to the N-terminus of the Fc domain through an immunoglobulin hinge region. In one embodiment, the immunoglobulin hinge region is a human IgG1 hinge region. In one embodiment, the Fab fragment comprising an antigen binding site specific for DR5, the Fab fragment comprising an antigen binding site specific for FAP and the Fc domain are part of an immunoglobulin molecule. In a particular embodiment, the immunoglobulin molecule is an IgG class immunoglobulin. In an even more particular embodiment, the immunoglobulin is an IgG1 subclass immunoglobulin. In another embodiment, the immunoglobulin is an IgG4 subclass immunoglobulin. In a further particular embodiment, the immunoglobulin is a human immunoglobulin. In other embodiments, the immunoglobulin is a chimeric immunoglobulin or a humanized immunoglobulin.

In one embodiment, a bispecific antibody that binds to DR5 and FAP according to any of the above embodiments comprises an Immunoglobulin G (IgG) molecule with one binding site specific for DR5 and one binding site specific for FAP, wherein either the variable regions or the constant regions of the heavy and light chain of one arm (Fab fragment) of the IgG molecule are exchanged.

In one embodiment, a bispecific antibody that binds to DR5 and FAP according to any of the above embodiments comprises an Immunoglobulin G (IgG) molecule with one binding site specific for DR5 and one binding site specific for FAP, wherein the variable regions of the heavy and light chain of one arm (Fab fragment) of the IgG molecule are exchanged. This antibody format is also referred to as $CrossMab_{(VHVL)}$.

In one embodiment, the variable regions of the heavy and light chain of the one arm (Fab fragment) of the IgG molecule which comprises the binding site specific for FAP are exchanged.

In one embodiment, a bispecific antibody that binds to DR5 and FAP according to any of the above embodiments comprises an Immunoglobulin G (IgG) molecule with one binding site specific for DR5 and one binding site specific for FAP, wherein the constant regions of the heavy and light chain of one arm (Fab fragment) of the IgG molecule are exchanged. This antibody format is also referred to as $CrossMab_{(CH1CL)}$.

In one embodiment, the constant regions of the heavy and light chain of the one arm (Fab fragment) of the IgG molecule which comprises the binding site specific for FAP are exchanged.

In one embodiment, a bispecific antibody that binds to DR5 and FAP according to any of the above embodiments

comprises an Immunoglobulin G (IgG) molecule with one binding site specific for DR5 and one binding site specific for FAP, wherein the complete VH-CH1 and VL-CL domains of one arm (Fab fragment) of the IgG molecule are exchanged. This means that at least one of the Fab fragments is fused to the N-terminus of the Fc domain via the light chain (VLCL). In one embodiment, the other Fab fragment is fused to the the N-terminus of the Fc domain via the heavy chain (VHCH1).

This antibody format is also referred to as $CrossMab_{Fab}$. In one embodiment, both Fab fragments are are fused to the N-terminus of the Fc domain through an immunoglobulin hinge region.

**D. Fc domain modifications reducing Fc receptor binding and/or effector function**

In one preferred embodiment, a bispecific antibody that binds to DR5 and FAP according to any of the above embodiments comprises an Immunoglobulin G (IgG) molecule wherein the Fc part is modified. The modified Fc part has a reduced binding affinity for the Fcγ receptors compared to a wildtype Fc part.

The Fc domain of the bispecific antibodies of the invention consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other.

In one embodiment, the Fc domain of the bispecific antibodies of the invention is an IgG Fc domain. In a particular embodiment, the Fc domain is an $IgG_1$ Fc domain. In another embodiment, the Fc domain is an $IgG_4$ Fc domain. In a more specific embodiment, the Fc domain is an $IgG_4$ Fc domain comprising an amino acid substitution at position S228 (Kabat numbering), particularly the amino acid substitution S228P. In a more specific embodiment, the Fc domain is an $IgG_4$ Fc domain comprising amino acid substitutions L235E and S228P and P329G. This amino acid substitution reduces *in vivo* Fab arm exchange of $IgG_4$ antibodies (see Stubenrauch et al., Drug Metabolism and Disposition 38, 84-91 (2010)). In a further particular embodiment, the Fc domain is human. The Fc domain confers favorable pharmacokinetic properties to the bispecific antibodies of the invention, including a long serum half-life which contributes to good accumulation in the target tissue and a favorable tissue-blood distribution ratio. At the same time it may, however, lead to undesirable targeting of the bispecific antibodies of the invention to cells expressing Fc receptors rather than to the preferred antigen-bearing cells. Accordingly, in particular embodiments, the Fc domain of the the bispecific antibodies of the invention exhibits reduced binding affinity to an Fc receptor and/or reduced effector function, as compared to a native $IgG_1$ Fc domain. In one such embodiment, the Fc domain (or the bispecific antibodies of the invention comprising said Fc domain) exhibits less than 50%, preferably less than 20%, more preferably less than 10% and most preferably less than 5% of the binding affinity to an Fc receptor, as compared to a native $IgG_1$ Fc domain (or a bispecific antibodies of the invention comprising a native $IgG_1$ Fc domain), and/or less than 50%, preferably less than 20%, more preferably less than 10% and most preferably less than 5% of the effector function, as compared to a native $IgG_1$ Fc domain domain (or a bispecific antibodies of the invention comprising a native $IgG_1$ Fc domain). In one embodiment, the Fc domain (or the bispecific antibodies of the invention comprising said Fc domain) does not substantially bind to an Fc receptor and/or induce effector function. In a particular embodiment, the Fc receptor is an Fcγ receptor. In one embodiment, the Fc receptor is a human Fc receptor. In one embodiment, the Fc receptor is an activating Fc receptor. In a specific embodiment, the Fc receptor is an activating human Fcγ receptor, more specifically human FcγRIIIa, FcγRI or FcγRIIa, most specifically human FcγRIIIa. In one embodiment the Fc receptor is an inhibitory Fc receptor. In a specific embodiment, the Fc receptor is an inhibitory human Fcγ receptor, more specifically human FcgRIIB. In one embodiment the effector function is one or more of CDC, ADCC, ADCP, and cytokine secretion. In a particular embodiment, the effector function is ADCC. In one embodiment, the Fc domain domain exhibits substantially similar binding affinity to neonatal Fc receptor (FcRn), as compared to a native $IgG_1$ Fc domain domain. Substantially similar binding to FcRn is achieved when the Fc domain (or the bispecific antibodies of the invention comprising said Fc domain) exhibits greater than about 70%, particularly greater than about 80%, more particularly greater than about 90% of the binding affinity of a native $IgG_1$ Fc domain (or the bispecific antibodies of the invention comprising a native $IgG_1$ Fc domain) to FcRn.

In certain embodiments, the Fc domain is engineered to have reduced binding affinity to an Fc receptor and/or reduced effector function, as compared to a non-engineered Fc domain. In particular embodiments, the Fc domain of the bispecific antibodies of the invention comprises one or more amino acid mutation that reduces the binding affinity of the Fc domain to an Fc receptor and/or effector function. Typically, the same one or more amino acid mutation is present in each of the two subunits of the Fc domain. In one embodiment, the amino acid mutation reduces the binding affinity of the Fc domain to an Fc receptor. In one embodiment, the amino acid mutation reduces the binding affinity of the Fc domain to an Fc receptor by at least 2-fold, at least 5-fold, or at least 10-fold. In embodiments, where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In one embodiment, the bispecific antibodies of the invention comprising an engineered Fc domain exhibits less than 20%, particularly less than 10%, more particularly less than 5% of the binding affinity to an Fc receptor as compared to a bispecific antibodies of the invention comprising a non-engineered Fc domain.

In a particular embodiment, the Fc receptor is an Fcγ receptor. In some embodiments, the Fc receptor is a human Fc receptor. In one embodiment, the Fc receptor is an inhibitory Fc receptor. In a specific embodiment, the Fc receptor is an inhibitory human Fcγ receptor, more specifically human FcgRIIB. In some embodiments the Fc receptor is an activating Fc receptor. In a specific embodiment, the Fc receptor is an activating human Fcγ receptor, more specifically human FcγRIIIa, FcγRI or FcγRIIa, most specifically human FcγRIIIa. Preferably, binding to each of these receptors is reduced. In some embodiments, binding affinity to a complement component, specifically binding affinity to C1q, is also reduced. In one embodiment binding affinity to neonatal Fc receptor (FcRn) is not reduced. Substantially similar binding to FcRn, i.e. preservation of the binding affinity of the Fc domain to said receptor, is achieved when the Fc domain (or the bispecific antibodies of the invention comprising said Fc domain) exhibits greater than about 70% of the binding affinity of a non-engineered form of the Fc domain (or the bispecific antibodies of the invention comprising said non-engineered form of the Fc domain) to FcRn. The Fc domain, or the bispecific antibodies of the invention of the invention comprising said Fc domain, may exhibit greater than about 80% and even greater than about 90% of such affinity. In certain embodiments, the Fc domain of the bispecific antibodies of the invention is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced dendritic cell maturation, or reduced T cell priming. In one embodiment, the reduced effector function is one or more of reduced CDC, reduced ADCC, reduced ADCP, and reduced cytokine secretion. In a particular embodiment, the reduced effector function is reduced ADCC. In one embodiment, the reduced ADCC is less than 20% of the ADCC induced by a non-engineered Fc domain (or a bispecific antibody of the invention comprising a non-engineered Fc domain).

In one embodiment, the amino acid mutation that reduces the binding affinity of the Fc domain to an Fc receptor and/or effector function is an amino acid substitution. In one embodiment the Fc domain comprises an amino acid substitution at a position of E233, L234, L235, N297, P331 and P329. In a more specific embodiment, the Fc domain comprises an amino acid substitution at a position of L234, L235 and P329. In some embodiments the Fc domain comprises the amino acid substitutions L234A and L235A. In one such embodiment, the Fc domain is an $IgG_1$ Fc domain, particularly a human $IgG_1$ Fc domain. In one embodiment, the Fc domain comprises an amino acid substitution at position P329. In a more specific embodiment, the amino acid substitution is P329A or P329G, particularly P329G. In one embodiment, the Fc domain comprises an amino acid substitution at position P329 and a further amino acid substitution at a position selected from E233, L234, L235, N297 and P331. In a more specific embodiment, the further amino acid substitution is E233P, L234A, L235A, L235E, N297A, N297D or P331S. In particular embodiments, the Fc domain comprises amino acid substitutions at positions P329, L234 and L235. In more particular embodiments, the Fc domain comprises the amino acid mutations L234A, L235A and P329G ("P329G LALA"). In one such embodiment, the Fc domain is an $IgG_1$ Fc domain, particularly a human $IgG_1$ Fc domain.

The "P329G LALA" combination of amino acid substitutions almost completely abolishes Fcγ receptor binding of a human $IgG_1$ Fc domain, as described in WO 2012/130831herein referred to. WO 2012/130831 also describes methods of preparing such mutant Fc domains and methods for determining its properties such as Fc receptor binding or effector functions.

$IgG_4$ antibodies exhibit reduced binding affinity to Fc receptors and reduced effector functions as compared to $IgG_1$ antibodies. Hence, in some embodiments, the Fc domain of the bispecific antibodies of the invention is an $IgG_4$ Fc domain, particularly a human $IgG_4$ Fc domain. In one embodiment, the $IgG_4$ Fc domain comprises amino acid substitutions at position S228, specifically the amino acid substitution S228P. To further reduce its binding affinity to an Fc receptor and/or its effector function, in one embodiment the $IgG_4$ Fc domain comprises an amino acid substitution at position L235, specifically the amino acid substitution L235E. In another embodiment, the $IgG_4$ Fc domain comprises an amino acid substitution at position P329, specifically the amino acid substitution P329G. In a particular embodiment, the $IgG_4$ Fc domain comprises amino acid substitutions at positions S228, L235 and P329, specifically amino acid substitutions S228P, L235E and P329G. Such $IgG_4$ Fc domain mutants and their Fcγ receptor binding properties are described in WO 2012/130831, herein referred to.

In a particular embodiment, the Fc domain exhibiting reduced binding affinity to an Fc receptor and/or reduced effector function, as compared to a native $IgG_1$ Fc domain, is a human $IgG_1$ Fc domain comprising the amino acid substitutions L234A, L235A and optionally P329G, or a human $IgG_4$ Fc domain comprising the amino acid substitutions S228P, L235E and optionally P329G.

In certain embodiments, N-glycosylation of the Fc domain has been eliminated. In one such embodiment the Fc domain comprises an amino acid mutation at position N297, particularly an amino acid substitution replacing asparagine by alanine (N297A) or aspartic acid (N297D).

In addition to the Fc domains described hereinabove and in WO 2012/130831, Fc domains with reduced Fc

receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor.

Effector function of an Fc domain, or bispecific antibodies of the invention comprising an Fc domain, can be measured by methods known in the art. Examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo,* e.g. in a animal model such as that disclosed in Clynes et al., Proc Natl Acad Sci USA 95, 652-656 (1998).

In some embodiments, binding of the Fc domain to a complement component, specifically to C1q, is reduced. Accordingly, in some embodiments in which the Fc domain is engineered to have reduced effector function, said reduced effector function includes reduced CDC. C1q binding assays may be carried out to determine whether the bispecific antibodies of the invention is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

The following section describes preferred embodiments of the bispecific antibodies of the invention comprising Fc domain modifications reducing Fc receptor binding and/or effector function.

In one preferred embodiment, a bispecific antibody that binds to DR5 and FAP according to any of the above embodiments comprises:

a) an Immunoglobulin G (IgG) molecule with two binding sites specific for DR5, wherein the Fc domain exhibits reduced binding affinity to an Fc receptor and/or reduced effector function, as compared to a native IgG1 Fc domain; and
b) two Fab fragments specific for FAP, wherein either the variable regions or the constant regions of the heavy and light chain are exchanged.

In one preferred embodiment, a bispecific antibody that binds to DR5 and FAP according to any of the above embodiments comprises:

a) an Immunoglobulin G (IgG) molecule with two binding sites specific for DR5, wherein the Fc domain comprises one or more amino acid substitution that reduces binding to an Fc receptor and/or effector function; and
b) two Fab fragments specific for FAP, wherein either the variable regions or the constant regions of the heavy and light chain are exchanged.

In one preferred embodiment, a bispecific antibody that binds to DR5 and FAP according to any of the above embodiments comprises:

a) an Immunoglobulin G (IgG) molecule with two binding sites specific for DR5, wherein said one or more amino acid substitution is at one or more position of L234, L235, and P329, and
b) two Fab fragments specific for FAP, wherein either the variable regions or the constant regions of the heavy and light chain are exchanged.

In one preferred embodiment, a bispecific antibody that binds to DR5 and FAP according to any of the above

embodiments comprises:

a) an Immunoglobulin G (IgG) molecule with two binding sites specific for DR5, wherein each subunit of the Fc domain comprises three amino acid substitutions that reduce binding to an activating or inhibitory Fc receptor and/or effector function wherein said amino acid substitutions are L234A, L235A and P329G; and
b) two Fab fragments specific for FAP, wherein either the variable regions or the constant regions of the heavy and light chain are exchanged.

In one preferred embodiment, a bispecific antibody that binds to DR5 and FAP according to any of the above embodiments comprises:

a) an Immunoglobulin G (IgG) molecule with two binding sites specific for DR5, wherein each subunit of the Fc domain comprises three amino acid substitutions that reduce binding to an activating or inhibitory Fc receptor and/or effector function wherein said amino acid substitutions are L234A, L235A and P329G; and
b) two Fab fragments specific for FAP, wherein the variable regions of the Fab heavy and light chain are exchanged.

In one preferred embodiment, a bispecific antibody that binds to DR5 and FAP according to any of the above embodiments comprises:

a) an Immunoglobulin G (IgG) molecule with two binding sites specific for DR5, wherein each subunit of the Fc domain comprises three amino acid substitutions that reduce binding to an activating or inhibitory Fc receptor and/or effector function wherein said amino acid substitutions are L234A, L235A and P329G; and
b) two Fab fragments specific for FAP, wherein the constant regions of the Fab heavy and light chain are exchanged.

In one embodiment, a bispecific antibody that binds to DR5 and FAP according to any of the above embodiments comprises:

a) an Immunoglobulin G (IgG) molecule with two binding sites specific for DR5, wherein each subunit of the Fc domain comprises three amino acid substitutions that reduce binding to an activating or inhibitory Fc receptor and/or effector function wherein said amino acid substitutions are L234A, L235A and P329G; and
b) two Fab fragments specific for FAP, wherein the variable regions of the Fab heavy and light chain are exchanged, wherein the two Fab fragments are fused to the constant heavy chain of said IgG molecule by a peptide linker.

In one embodiment, a bispecific antibody that binds to DR5 and FAP according to any of the above embodiments comprises:

a) an Immunoglobulin G (IgG) molecule with two binding sites specific for DR5, wherein each subunit of the Fc domain comprises three amino acid substitutions that reduce binding to an activating or inhibitory Fc receptor and/or effector function wherein said amino acid substitutions are L234A, L235A and P329G; and
b) two Fab fragments specific for FAP, wherein the constant regions of the Fab heavy and light chain are exchanged, wherein the two Fab fragments are fused to the constant heavy chain of said IgG molecule by a peptide linker.

In one embodiment, a bispecific antibody that binds to DR5 and FAP according to any of the above embodiments comprises:

a) an Immunoglobulin G (IgG) molecule with two binding sites specific for DR5, comprising

a heavy chain CDR1 of SEQ ID NO.:1;
a heavy chain CDR2 of SEQ ID NO.:2;
a heavy chain CDR3 of SEQ ID NO.:3;
a light chain CDR1 of SEQ ID NO.:4;
a light chain CDR2 of SEQ ID NO.:5; and
a light chain CDR3 of SEQ ID NO.:6;
wherein each subunit of the Fc domain comprises three amino acid substitutions that reduce binding to activating and inhibitory Fc receptors and/or effector function wherein said amino acid substitutions are L234A, L235A and P329G; and

b) two Fab fragments specific for FAP, comprising

a heavy chain CDR1 of SEQ ID NO.:9;
a heavy chain CDR2 of SEQ ID NO.:10;
a heavy chain CDR3 of SEQ ID NO.:11;
a light chain CDR1 of SEQ ID NO.:12;
a light chain CDR2 of SEQ ID NO.:13; and
a light chain CDR3 of SEQ ID NO.:14;
wherein either the variable regions or the constant regions of the heavy and light chain are exchanged, wherein the two Fab fragments are fused to the constant heavy chain of said IgG molecule by a peptide linker.

In one embodiment, a bispecific antibody that binds to DR5 and FAP according to any of the above embodiments comprises:

a) an Immunoglobulin G (IgG) molecule with two binding sites specific for DR5, comprising

a heavy chain CDR1 of SEQ ID NO.:1;
a heavy chain CDR2 of SEQ ID NO.:2;
a heavy chain CDR3 of SEQ ID NO.:3;
a light chain CDR1 of SEQ ID NO.:4;
a light chain CDR2 of SEQ ID NO.:5; and
a light chain CDR3 of SEQ ID NO.:6;

wherein each subunit of the Fc domain comprises three amino acid substitutions that reduce binding to an activating or inhibitory Fc receptor and/or effector function wherein said amino acid substitutions are L234A, L235A and P329G and
b) two Fab fragments specific for FAP, comprising

a heavy chain CDR1 of SEQ ID NO.:9;
a heavy chain CDR2 of SEQ ID NO.:10;
a heavy chain CDR3 of SEQ ID NO.:11;
a light chain CDR1 of SEQ ID NO.:12;
a light chain CDR2 of SEQ ID NO.:13; and
a light chain CDR3 of SEQ ID NO.:14;

wherein the constant regions of the Fab heavy and light chain are exchanged, wherein the two Fab fragments are fused to the constant heavy chain of said IgG molecule by a peptide linker.

In one embodiment, a bispecific antibody that binds to DR5 and FAP according to any of the above embodiments comprises

a) an Immunoglobulin G (IgG) molecule with two binding sites specific for DR5, comprising

a heavy chain CDR1 consisting of SEQ ID NO.:1;
a heavy chain CDR2 of SEQ ID NO.:2;
a heavy chain CDR3 of SEQ ID NO.:3;
a light chain CDR1 of SEQ ID NO.:4;
a light chain CDR2 of SEQ ID NO.:5;
a light chain CDR3 of SEQ ID NO.:6;

wherein each subunit of the Fc domain comprises three amino acid substitutions that reduce binding to an activating or inhibitory Fc receptor and/or effector function wherein said amino acid substitutions are L234A, L235A and P329G and
b) two Fab fragments specific for FAP, comprising

a heavy chain CDR1 of SEQ ID NO.:9;
a heavy chain CDR2 of SEQ ID NO.:10;
a heavy chain CDR3 of SEQ ID NO.:11;
a light chain CDR1 of SEQ ID NO.:12;
a light chain CDR2 of SEQ ID NO.:13; and

a light chain CDR3 of SEQ ID NO.:14;

wherein the variable regions of the Fab heavy and light chain are exchanged, wherein the two Fab fragments are fused to the constant heavy chain of said IgG molecule by a peptide linker.

In one embodiment, a bispecific antibody that binds to DR5 and FAP according to any of the above embodiments comprises :

a) an Immunoglobulin G (IgG) molecule with two binding sites specific for DR5,
comprising a variable heavy chain of SEQ ID NO.:7 and a variable light chain of SEQ ID NO.:8;
wherein each subunit of the Fc domain comprises three amino acid substitutions that reduce binding to an activating or inhibitory Fc receptor and/or effector function wherein said amino acid substitutions are L234A, L235A and P329G and
b) two Fab fragments specific for FAP, comprising a heavy chain variable region comprising an amino acid sequence of SEQ ID NO.:15 and a light chain variable region comprising an amino acid sequence of SEQ ID NO.:16, wherein either the variable regions or the constant regions of the heavy and light chain are exchanged, wherein the two Fab fragments are fused to the constant heavy chain of said IgG molecule by a peptide linker.

In one embodiment, a bispecific antibody that binds to DR5 and FAP according to any of the above embodiments comprises

a) an Immunoglobulin G (IgG) molecule with two binding sites specific for DR5,
comprising a variable heavy chain of SEQ ID NO.:7 and a variable light chain of SEQ ID NO.:8;
wherein each subunit of the Fc domain comprises three amino acid substitutions that reduce binding to an activating or inhibitory Fc receptor and/or effector function wherein said amino acid substitutions are L234A, L235A and P329G and
b) two Fab fragments specific for FAP, comprising a heavy chain variable region comprising an amino acid sequence of SEQ ID NO.:15 and a light chain variable region comprising an amino acid sequence of SEQ ID NO.:16,
wherein the constant regions of the Fab heavy and light chain are exchanged, wherein the two Fab fragments are fused to the constant heavy chain of said IgG molecule by a peptide linker.

In one embodiment, a bispecific antibody that binds to DR5 and FAP according to any of the above embodiments comprises

a) an Immunoglobulin G (IgG) molecule with two binding sites specific for DR5,
comprising a variable heavy chain of SEQ ID NO.:7 and a variable light chain of SEQ ID NO.:8;
wherein each subunit of the Fc domain comprises three amino acid substitutions that reduce binding to an activating or inhibitory Fc receptor and/or effector function wherein said amino acid substitutions are L234A, L235A and P329G and
b) two Fab fragments specific for FAP, comprising a heavy chain variable region comprising an amino acid sequence of SEQ ID NO.:15 and a light chain variable region comprising an amino acid sequence of SEQ ID NO.:16, wherein the variable regions of the Fab heavy and light chain are exchanged, wherein the two Fab fragments are fused to the constant heavy chain of said IgG molecule by a peptide linker.

## E. Fc domain modifications promoting heterodimerization

The bispecific DR5-FAP antibodies of the invention comprise different antigen binding moieties, fused to one or the other of the two subunits of the Fc domain, thus the two subunits of the Fc domain are typically comprised in two non-identical polypeptide chains. Recombinant co-expression of these polypeptides and subsequent dimerization leads to several possible combinations of the two polypeptides. To improve the yield and purity of the bispecific antibodies of the invention in recombinant production, it will thus be advantageous to introduce in the Fc domain of the bispecific antibodies of the invention a modification promoting the association of the desired polypeptides.

Accordingly, in particular embodiments, the Fc domain of the bispecific antibodies of the invention comprises a modification promoting the association of the first and the second subunit of the Fc domain. The site of most extensive protein-protein interaction between the two subunits of a human IgG Fc domain is in the CH3 domain of the Fc domain. Thus, in one embodiment said modification is in the CH3 domain of the Fc domain.

In a specific embodiment, said modification is a so-called "knob-into-hole" modification, comprising a "knob" modification in one of the two subunits of the Fc domain and a "hole" modification in the other one of the two subunits

of the Fc domain. The knob-into-hole technology is described e.g. in US 5,731,168; US 7,695,936; Ridgway et al., Prot Eng 9, 617-621 (1996) and Carter, J Immunol Meth 248, 7-15 (2001).

Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular embodiment, in the CH3 domain of the first subunit of the Fc domain of the bispecific antibodies of the invention an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g. by site-specific mutagenesis, or by peptide synthesis.

In a specific embodiment, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A).

In yet a further embodiment, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in formation of a disulfide bridge between the two subunits of the Fc domain, further stabilizing the dimer (Carter, J Immunol Methods 248, 7-15 (2001)).

In an alternative embodiment a modification promoting association of the first and the second subunit of the Fc domain comprises a modification mediating electrostatic steering effects, e.g. as described in WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

In one embodiment, a bispecific antibody that binds to DR5 and FAP according to any of the above embodiments comprises an Immunoglobulin G (IgG) molecule with two binding sites specific for DR5, wherein the Fc part of the first heavy chain comprises a first dimerization module and the Fc part of the second heavy chain comprises a second dimerization module allowing a heterodimerization of the two heavy chains of the IgG molecule.

In a further preferred embodiment, the first dimerization module comprises knobs and the second dimerization module comprises holes according to the knobs into holes strategy (see Carter P.; Ridgway J.B.B.; Presta L.G.: Immunotechnology, Volume 2, Number 1, February 1996 , pp. 73-73(1)).

## F. Nucleic Acid sequences, vectors and methods of production

Polynucleotides encoding a bispecific antibody capable of binding to DR5 and FAP may be used for its production. The polynucleotides encoding bispecific antibodies or the antibodies binding to DR5 of the invention may be expressed as a single polynucleotide that encodes the entire bispecific antigen binding molecule or the entire antibody binding to DR5 or as multiple (e.g., two or more) polynucleotides that are co-expressed. Polypeptides encoded by polynucleotides that are co-expressed may associate through, e.g., disulfide bonds or other means to form a functional bispecific antibody or an antibody binding to DR5. For example, the light chain portion of a Fab fragment may be encoded by a separate polynucleotide from the portion of the bispecific antibody or the antibody binding to DR5 comprising the heavy chain portion of the Fab fragment, an Fc domain subunit and optionally (part of) another Fab fragment. When co-expressed, the heavy chain polypeptides will associate with the light chain polypeptides to form the Fab fragment. In another example, the portion of the bispecific antibody or the antibody binding to DR5 provided therein comprising one of the two Fc domain subunits and optionally (part of) one or more Fab fragments could be encoded by a separate polynucleotide from the portion of the bispecific antibody or the antibody binding to DR5 provided therein comprising the the other of the two Fc domain subunits and optionally (part of) a Fab fragment. When co-expressed, the Fc domain subunits will associate to form the Fc domain.

In certain embodiments the polynucleotide or nucleic acid is DNA. In other embodiments, a polynucleotide of the present invention is RNA, for example, in the form of messenger RNA (mRNA). RNA of the present invention may

be single stranded or double stranded.

### G. Antibody Variants

In certain embodiments, amino acid sequence variants of the bispecific antibodies and antibodies binding to DR5 provided herein are contemplated, in addition to those described above. For example, it may be desirable to improve the binding affinity and/or other biological properties of the bispecific antibody or the antibody binding to DR5. Amino acid sequence variants of a bispecific antibody or an antibody binding to DR5 may be prepared by introducing appropriate modifications into the nucleotide sequence encoding the bispecific antibody or the antibody binding to DR5, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

#### 1. Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table B under the heading of "conservative substitutions." More substantial changes are provided in Table B under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE B**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; He | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;

(2) neutral hydrophilic: Cys, Ser, Thr, Asn, Gln;

(3) acidic: Asp, Glu;

(4) basic: His, Lys, Arg;

(5) residues that influence chain orientation: Gly, Pro;

(6) aromatic: Trp, Tyr, Phe.

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

**2. Glycosylation variants**

In certain embodiments, a bispecific antibody or an antibody binding to DR5 provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the bispecific antibody or the antibody binding to DR5 comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in a bispecific antibody or an antibody binding to DR5 of the invention may be made in order to create antibody variants with certain improved properties.

In one embodiment, bispecific antibody variants or variants of antibodies binding to DR5 are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Bispecific antibodies variants or variants of antibodies binding to DR5 are further provided with bisected oligosaccharides, e.g., in which a biantennary oligosaccharide attached to the Fc region of the bispecific antibody or the antibody binding to DR5 is bisected by GlcNAc. Such bispecific antibody variants or variants of antibodies binding to DR5 may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### 3. Cysteine engineered antibody variants

In certain embodiments, it may be desirable to create cysteine engineered bispecific antibodies or antibodies binding to DR5, e.g., "thioMAbs," in which one or more residues of a bispecific antibody or antibodies binding to DR5 are substituted with cysteine residues. In particular embodiments, the substituted residues occur at accessible sites of the bispecific antibody or the antibody binding to DR5. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

## H. Recombinant Methods and Compositions

Bispecific antibodies and antibodies binding to DR5 of the invention may be obtained, for example, by solid-state peptide synthesis (e.g. Merrifield solid phase synthesis) or recombinant production. For recombinant production one or more polynucleotide encoding the bispecific antibodies or antibodies binding to DR5 (or fragments), e.g., as described above, is isolated and inserted into one or more vectors for further cloning and/or expression in a host cell. Such polynucleotide may be readily isolated and sequenced using conventional procedures. In one aspect a vector, preferably an expression vector, comprising one or more of the polynucleotides of the invention is provided. Methods which are well known to those skilled in the art can be used to construct expression vectors containing the coding sequence of a bispecific antibody (fragment) or an antibody (fragment) binding to DR5 along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the bispecific antibody (fragment) or an antibody (fragment) binding to DR5 (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present invention may encode one or more polypeptides, which are post- or co-translationally separated into the final proteins via proteolytic cleavage. In addition, a vector, polynucleotide, or nucleic acid of the invention may encode heterologous coding regions, either fused or unfused to a polynucleotide encoding the bispecific antibody (fragment) or an antibody (fragment) binding to DR5 of the invention, or variant or derivative thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells.

Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present invention may be associated with additional coding regions which encode secretory or signal peptides, which direct the secretion of a polypeptide encoded by a polynucleotide of the present invention. For example, if secretion of the bispecific antibody or the antibody binding to DR5 is desired, DNA encoding a signal sequence may be placed upstream of the nucleic acid encoding a bispecific antibody of the invention or the antibody binding to DR5 of the invention or a fragment thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain aspects, the native signal peptide, *e.g.* an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag)

or assist in labeling the bispecific antibody or the antibody binding to DR5 may be included within or at the ends of the bispecific antibody (fragment) or the antibody (fragment) binding to DR5 encoding polynucleotide. In a further aspect, a host cell comprising one or more polynucleotides of the invention is provided. In certain aspect a host cell comprising one or more vectors of the invention is provided. The polynucleotides and vectors may incorporate any of the features, singly or in combination, described herein in relation to polynucleotides and vectors, respectively. In one such aspect a host cell comprises (e.g. has been transformed or transfected with) a vector comprising a polynucleotide that encodes (part of) a bispecific antibody or an antibody binding to DR5 of the invention. As used herein, the term "host cell" refers to any kind of cellular system which can be engineered to generate the bispecific antibodies or an antibody binding to DR5 of the invention or fragments thereof. Host cells suitable for replicating and for supporting expression of bispecific antibodies or of antibodies binding to DR5 are well known in the art. Such cells may be transfected or transduced as appropriate with the particular expression vector and large quantities of vector containing cells can be grown for seeding large scale fermenters to obtain sufficient quantities of the bispecific antibody or of the antibodies binding to DR5 for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr$^-$ CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one aspect, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell).

Standard technologies are known in the art to express foreign genes in these systems. Cells expressing a polypeptide comprising either the heavy or the light chain of an antigen binding domain such as an antibody, may be engineered so as to also express the other of the antibody chains such that the expressed product is an antibody that has both a heavy and a light chain.

In one aspect, a method of producing a bispecific antibody or an antibody binding to DR5 according to the invention is provided, wherein the method comprises culturing a host cell comprising a polynucleotide encoding the bispecific antibody or the antibody binding to DR5, as provided herein, under conditions suitable for expression of the bispecific antibody or the antibody binding to DR5, and recovering the bispecific antibody or the antibody binding to DR5 from the host cell (or host cell culture medium). The components of the bispecific antibody or the antibody binding to DR5 are genetically fused to each other. Bispecific antibodies or the antibodies binding to DR5 can be designed such that its components are fused directly to each other or indirectly through a linker sequence. The composition and length of the linker may be determined in accordance with methods well known in the art and may be tested for efficacy. Examples of linker sequences between different components of bispecific antibodies are found in the sequences provided herein. Additional sequences may also be included to incorporate a cleavage site to separate the individual components of the fusion if desired, for example an endopeptidase recognition sequence.

In certain embodiments, the Fab fragments forming part of the bispecific antibody or the antibody binding to DR5 comprise at least an antibody variable region capable of binding an antigenic determinant. Variable regions can form part of and be derived from naturally or non-naturally occurring antibodies and fragments thereof. Methods to

produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of antibody, antibody fragment, antigen binding domain or variable region can be used in the bispecific antibodies or the antibodies binding to DR5 of the invention. Non-limiting antibodies, antibody fragments, antigen binding domains or variable regions useful in the present invention can be of murine, primate, or human origin. If the bispecific antibody or the antibody binding to DR5 is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain embodiments, the Fab fragments useful in the present invention are engineered to have enhanced binding affinity according to, for example, the methods disclosed in U.S. Pat. Appl. Publ. No. 2004/0132066, herein referred to. The ability of the bispecific antibody or the antibody binding to DR5 of the invention to bind to a specific antigenic determinant can be measured either through an enzyme-linked immunosorbent assay (ELISA) or other techniques familiar to one of skill in the art, e.g. surface plasmon resonance technique (analyzed on a BIACORE T100 system) (Liljeblad, et al., Glyco J 17, 323-329 (2000)), and traditional binding assays (Heeley, Endocr Res 28, 217-229 (2002)). Competition assays may be used to identify an antibody, antibody fragment, antigen binding domain or variable domain that competes with a reference antibody for binding to a particular antigen. In certain embodiments, such a competing antibody binds to the same epitope (e.g. a linear or a conformational epitope) that is bound by the reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). In an exemplary competition assay, immobilized antigen is incubated in a solution comprising a first labeled antibody that binds to the antigen and a second unlabeled antibody that is being tested for its ability to compete with the first antibody for binding to the antigen. The second antibody may be present in a hybridoma supernatant. As a control, immobilized antigen is incubated in a solution comprising the first labeled antibody but not the second unlabeled antibody.

After incubation under conditions permissive for binding of the first antibody to the antigen, excess unbound antibody is removed, and the amount of label associated with immobilized antigen is measured. If the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY).

Bispecific antibodies or antibodies binding to DR5 prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the bispecific antibody or the antibody binding to DR5 binds. For example, for affinity chromatography purification of bispecific antibodies of the invention, a matrix with protein A or protein G may be used. Sequential Protein A or G affinity chromatography and size exclusion chromatography can be used to isolate a bispecific antibody essentially as described in the Examples. The purity of the bispecific antibody or the antibody binding to DR5 can be determined by any of a variety of well known analytical methods including gel electrophoresis, high pressure liquid chromatography, and the like.

**I. Assays**

Bispecific antibodies that bind to DR5 and FAP and antibodies binding to DR5 provided herein may be identified, screened for, or characterized for their physical/chemical properties and/or biological activities by various assays known in the art.

**1. Affinity assays**

The affinity of the bispecific antibody and the antibody binding to DR5 provided therein for DR5 and/ or FAP can be determined in accordance with the methods set forth in the Examples by surface plasmon resonance (SPR), using standard instrumentation such as a BIAcore instrument (GE Healthcare), and receptors or target proteins such as may be obtained by recombinant expression. Alternatively, binding of bispecific antibody and the antibody binding to DR5 provided therein to DR5 and/or FAP may be evaluated using cell lines expressing the particular receptor or target antigen, for example by flow cytometry (FACS).

$K_D$ may be measured by surface plasmon resonance using a BIACORE® T100 machine (GE Healthcare) at 25°C. To analyze the interaction between the Fc-portion and Fc receptors, His-tagged recombinant Fc-receptor is captured by an anti-Penta His antibody (Qiagen) immobilized on CM5 chips and the bispecific constructs are used as analytes. Briefly, carboxymethylated dextran biosensor chips (CM5, GE Healthcare) are activated with N-ethyl-N'-(3-dimethylaminopropyl)-carbodiimide hydrochloride (EDC) and N-hydroxysuccinimide (NHS) according to the supplier's instructions. Anti Penta-His antibody is diluted with 10 mM sodium acetate, pH 5.0, to 40 μg/ml before injection at a flow rate of 5 μl/min to achieve approximately 6500 response units (RU) of coupled protein. Following the injection of the ligand, 1 M ethanolamine is injected to block unreacted groups. Subsequently the Fc-receptor is captured for 60 s at 4 or 10 nM. For kinetic measurements, four-fold serial dilutions of the bispecific construct (range between 500 nM and 4000 nM) are injected in HBS-EP (GE Healthcare, 10 mM HEPES, 150 mM NaCl, 3 mM EDTA, 0.05 % Surfactant P20, pH 7.4) at 25°C at a flow rate of 30 μl/min for 120 s.

To determine the affinity to the target antigen, bispecific constructs are captured by an anti human Fab specific antibody (GE Healthcare) that is immobilized on an activated CM5-sensor chip surface as described for the anti Penta-His antibody. The final amount of coupled protein is is approximately 12000 RU. The bispecific constructs are captured for 90 s at 300 nM. The target antigens are passed through the flow cells for 180 s at a concentration range from 250 to 1000 nM with a flowrate of 30 μl/min. The dissociation is monitored for 180 s.

Bulk refractive index differences are corrected for by subtracting the response obtained on reference flow cell. The steady state response was used to derive the dissociation constant $K_D$ by non-linear curve fitting of the Langmuir binding isotherm. Association rates ($k_{on}$) and dissociation rates ($k_{off}$) are calculated using a simple one-to-one Langmuir binding model (BIACORE® T100 Evaluation Software version 1.1.1) by simultaneously fitting the association and dissociation sensorgrams. The equilibrium dissociation constant ($K_D$) is calculated as the ratio $k_{off}/k_{on}$. See, e.g., Chen et al., J Mol Biol 293, 865-881 (1999).

**2. Binding assays and other assays**

In one aspect, a bispecific antibody or an antibody that binds to DR5 of the invention is tested for its antigen binding activity, e.g., by known methods such as ELISA, Western blot, etc.

In another aspect, competition assays may be used to identify an antibody that competes with a specific anti-FAP antibody or a specific anti-DR5 antibody for binding to FAP or DR5 respectively. In certain embodiments, such a competing antibody binds to the same epitope (e.g., a linear or a conformational epitope) that is bound by a specific anti-FAP antibody or a specific anti-DR5 antibody. Detailed exemplary methods for mapping an epitope to which an antibody binds are provided in Morris (1996) "Epitope Mapping Protocols," in Methods in Molecular Biology vol. 66

(Humana Press, Totowa, NJ). Further methods are described in the example section.

### 3. Activity assays

In one aspect, assays are provided for identifying bispecific antibodies that bind to DR5 and FAP or antibodies that binds to DR5 thereof having biological activity. Biological activity may include, e.g., DNA fragmentation, induction of apoptosis and lysis of targeted cells. Antibodies having such biological activity *in vivo* and/or *in vitro* are also provided.

In certain embodiments, a bispecific antibody or an antibodiy that binds to DR5 of the invention is tested for such biological activity. Assays for detecting cell lysis (e.g. by measurement of LDH release) or apoptosis (e.g. using the TUNEL assay) are well known in the art. Assays for measuring ADCC or CDC are also described in WO 2004/065540 (see Example 1 therein), herein referred to.

### J. Pharmaceutical Formulations

Pharmaceutical formulations of a bispecific antibody that binds to DR5 and FAP or an antibody that binds to DR5 as described herein are prepared by mixing such bispecific antibody or antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

### K. Therapeutic Methods and Compositions

The therapeutic combinations comprising one or more of the bispecific antibodies that bind to DR5 and FAP and a further chemotherapeutic agent provided herein may be used in therapeutic methods.

In one aspect, a bispecific antibody that binds to DR5 and FAP for use as a medicament is provided for use in combination with a further chemotherapeutic agent. In certain embodiments, a bispecific antibody that binds to DR5 and FAP for use in combination with a further chemotherapeutic agent is provided for use in a method of treatment. In certain embodiments, the invention provides a bispecific antibody that binds to DR5 and FAP for use in a method of treating an

individual having cancer comprising administering to the individual an effective amount of the bispecific antibody that binds to DR5 and FAP. In one such embodiment, the method further comprises administering to the individual an effective amount of at least one additional therapeutic agent, e.g., as described below. An "individual" according to any of the above embodiments is preferably a human. In one preferred embodiment, said cancer is pancreatic cancer, sarcoma or colorectal carcinoma. In other embodiments, the cancer is colorectal cancer, sarcoma, head and neck cancers, squamous cell carcinomas, breast cancer, pancreatic cancer, gastric cancer, non-small-cell lung carcinoma, small-cell lung cancer or mesothelioma. In embodiments in which the cancer is breast cancer, the breast cancer may be triple negative breast cancer.

In a further aspect, the invention provides the use of a therapeutic combination comprising a bispecific antibody that binds to DR5 and FAP and a further chemotherapeutic agent in the manufacture or preparation of a medicament. In one embodiment, the medicament is for treatment of cancer. In a further embodiment, the medicament is for use in a method of treating cancer comprising administering to an individual having cancer an effective amount of the medicament. In one such embodiment, the method further comprises administering to the individual an effective amount of at least one additional therapeutic agent, e.g., as described below. An "individual" according to any of the above embodiments may be a human.

In a further aspect, the application provides a method for treating cancer. In one embodiment, the method comprises administering to an individual having cancer an effective amount of a therapeutic combination comprising a bispecific antibody that binds to DR5 and FAP for use in combination with a further chemotherapeutic agent. In one such embodiment, the method further comprises administering to the individual an effective amount of at least one additional therapeutic agent, as described below. An "individual" according to any of the above embodiments may be a human. In one preferred embodiment said cancer is pancreatic cancer, sarcoma or colorectal carcinoma. In other embodiments, the cancer is colorectal cancer, sarcoma, head and neck cancers, squamous cell carcinomas, breast cancer, pancreatic cancer, gastric cancer, non-small-cell lung carcinoma, small-cell lung cancer or mesothelioma.

In a further aspect, the invention provides pharmaceutical formulations comprising any of the bispecific antibodies that bind to DR5 and FAP provided herein, e.g., for use in any of the above therapeutic methods, and a further chemotherapeutic agent. In one aspect, a pharmaceutical formulation comprises any of the bispecific antibodies that bind to DR5 and FAP provided herein and a pharmaceutically acceptable carrier. In another embodiment, a pharmaceutical formulation comprises any of the bispecific antibodies that bind to DR5 and FAP provided herein and at least one additional therapeutic agent, e.g., as described below.

A bispecific antibody can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Bispecific antibodies may be be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The bispecific antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of a bispecific antibody will depend on the type of disease to be treated, the type of antibody, the severity and course of the disease, whether the bispecific antibody is administered for preventive or therapeutic purposes, previous therapy, the patient's clinical history and response to the bispecific antibody and the discretion of the attending physician. The bispecific antibody is suitably administered to the patient at one time or over a series of treatments. Depending on the type and severity of the disease, about 1 μg/kg to 15 mg/kg (e.g. 0.1mg/kg-10mg/kg) of the bispecific antibody or the novel antibody binding to DR5 can be an initial candidate dosage for administration to the patient, whether, for example, by one or more separate administrations, or by continuous infusion. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of the bispecific would be in the range from about 0.05 mg/kg to about 10 mg/kg. Thus, one or more doses of about 0.5 mg/kg, 2.0 mg/kg, 4.0 mg/kg or 10 mg/kg may be administered to the patient. Such doses may be administered intermittently, e.g. every week or every three weeks (*e.g.* such that the patient receives from about two to about twenty, or *e.g.* about six doses of the bispecific antibody). An initial higher loading dose, followed by one or more lower doses may

be administered. However, other dosage regimens may be useful. The progress of this therapy is easily monitored by conventional techniques and assays.

**L. Articles of Manufacture**

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a bispecific antibody and an additional active agent is the further chemotherapeutic agent as described herein. The label or package insert indicates that the composition is used for treating the condition of choice. Moreover, the article of manufacture may comprise (a) a first container with a composition contained therein, wherein the composition comprises a bispecific antibody; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer’s solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to a bispecific antibody that binds to DR5 and FAP.

**III. EXAMPLES**

The following are examples of methods and compositions of the invention. It is understood that various other embodiments may be practiced, given the general description provided above.

Although the foregoing invention has been described in some detail by way of illustration and example for purposes of clarity of understanding, the descriptions and examples should not be construed as limiting the scope of the invention. It is expressely referred to the entire disclosures of all patent and scientific literature cited herein.

**Example 1: A DR5 - FAP death receptor agonistic bispecific antibody**

One approach of induction of apoptosis by cross-linking of death receptors as DR5 (apart from cross-linking via an antigen expressed by the tumor cell), is targeting the stroma surrounding the tumor. In that case, the targeted antigen is not displayed directly by the tumor cells but by a second, different cell type. One example for this kind of antigen would be FAP (fibroblast activation protein). This protein is expressed on activated fibroblast as they are found in the tumor stroma.

Novel DR5 binders were identified by phage display. The DR5 binders obtained by phage display were screened for apoptosis induction, specificity, species crossreactivity, and epitope specifity. DR5 binder 5E11 was selected and converted into tetravalent bispecific molecules. These bispecific antibodies contain two binding moieties, each for DR5 and FAP. The FAP binding moieties have been described in WO2012//020006. The 28H1 CrossFab domain (VHCL) was fused to the C-terminus of the anti DR5 heavy chain using a $(G_4S)_4$ connector providing bispecific antibodies in a 2+2 format.

**Table 1:** Bispecific, tetravalent DR5 - FAP CrossMab molecules (all with 28H1 CrossFab domain (VHCL) fused to the C-terminus of the anti DR5 heavy chain using a $(G_4S)_4$ connector, FAP binder: VH SEQ ID **NO.:15,** VL SEQ ID NO.:16)

| DR5 Binder | SEQ ID NO VH /VL (DR5) | **Name** | **Description** |
|---|---|---|---|
| 5E11 | 7/8 | DR5(5E11)-28H1 VHCL 2+2 | 28H1 CrossFab domain (VHCL) fused to the C-terminus of the anti DR5 (5E11) heavy chain using a $(G_4S)_4$ connector: VH $_{(DR5)}$-Fc part VH $_{(FAP)}$ -CL chain (SEQ ID NO.: 17) |

(continued)

| DR5 Binder | SEQ ID NO VH /VL (DR5) | **Name** | **Description** |
|---|---|---|---|
| | | | VL (DR5)-kappa light chain (SEQ ID NO.:19) VLCH1 (FAP) chain (SEQ ID NO.:20). |
| 5E11 | 7/8 | DR5(5E11)-28H1 VHCL 2+2 P329GLALA | As above, and removal of C-term. Lysine and P329G/LALA mutation in Fc VH $_{(DR5)}$-Fc part VH $_{(FAP)}$ -CL chain (SEQ ID NO.: 18) VL (DR5)-kappa light chain (SEQ ID NO.:19) VLCH1 (FAP) chain (SEQ ID NO.:20). |

The DR5 - FAP bispecific molecules were produced in transiently transfected HEK293 EBNA cells and were purified via Protein A and size exclusion chromatography. The obtained product yields were in a reasonable range (around 20 mg/L). The monomer content after the final purification step was above 96 % for all molecules.

Target binding analysis by surface plasmon resonance (Biacore) revealed that bispecific antibodies in the 2+2 format were able to simultaneously bind to recombinant DR5 and FAP (human and murine).

To evaluate if the DR5-FAP bispecific molecules are able to induce apoptosis of the MDA-MB-231 target cell line, 96 well plates were coated with recombinant human FAP for cross-linking of DR5 on the target cells via the subsequently added bispecific antibodies. After addition of the target cells (MDA-MB 231) and incubation for 24 hrs, apoptosis induction was determined by the standard DNA fragmentation ELISA assay. The DR5-FAP bispecific molecules exhibited apoptosis induction activity in the presence of FAP coated on the plates indicating that this activity is dependent on the cross-linking via recombinant FAP.

## Example 2: DR5 - FAP bispecific antibodies are able to induce apoptosis on different target cells

DR5 - FAP bispecific antibodies in the 2+2 format comprising newly isolated DR5 binders fused to the FAP 28H1 CrossFab moiety were tested in an experiment for induction of apoptosis on two different cell lines (MDA-MB-231 and G401) in a co-culture assay with GM05389 human FAP$^+$ fibroblasts. The bispecific antibodies were tested over a concentration range from 0.0007 - 7 nM. The results of the DNA fragmentation assay showed that the bispecific antibodies tested demonstrated good apoptosis induction activity on both cell lines. According to the results obtained with the MDA-MB-231 cells, the antibodies in the bispecific 2+2 format did not show the decline in activity at high concentrations but stayed constant or even more increased up to the highest concentration. In the experiment, with G401 cell in co-culture with GM05389 fibroblasts, the maximum of apoptosis induction was reached already at a concentration of 0.07 nM and then stayed constant. In this setting all molecules performed similarly in terms of apoptosis induction levels.

**Table 1 a:** Characterisation of FAP-DR5 bispecific antibody

| Clone Name | 5E11 | | 28H1 | |
|---|---|---|---|---|
| Affinity human [nM] | 165 | (IgG) | 2.6 | (IgG) |
| Affinity Cyno [nM] | 1.02 | (IgG) | 3.7 | (IgG) |
| Avidity Human [nM] | 0.06 | (IgG) | 0.25 | (IgG) |
| Avidity Cyno [nM] | 0.06 | (IgG) | 0.06 | (IgG) |
| Binding Mode | agonistic (only upon crosslinking) TRAIL competitive, conformational epitope | | No interference with signaling/protease function, conformational epitope | |
| Specificity | No binding to huDR4, DcR1/2, OPG | | No binding to hu DPP-IV (CD26, closest FAP homologue) | |
| Species CrossReactivity | Human, cyno | | Human, cyno, murine | |

## Example 3: FAP prevalence in human tumors

The prevalence of FAP in human tumors was evaluated by IHC to get an understanding on possible clinical

use of bispecificific DR5-FAP antibody.

Rat anti-human Seprase antibody (IgG2a, clone D8) from Vitatex (MABS1001) was used to immunostain 2,5 μm FFPET sections from various tumour indications on the Ventana Benchmark XT. Sections were subjected to standard CC1 treatment followed by antibody incubation for 60' at 37°C at a concentration of 5 μg/mL in Dako antibody diluent (S3022) and positive staining was detected using the Ultraview DAB detection system (Ventana #760-4456). Matched isotype antibody from Abcam (ab18450) was used as the negative control.

FAP+ stromal infiltrate was present in human tumors of different indications including SCLC marking potentially interesting clinical indications for a bispecificific DR5-FAP antibody (Table 2).

**Table 2: FAP prevalence in human epithelial tumors**

| Tumor Type | % cases with moderate to high grade of FAP+ infiltrate | N of samples investigated |
|---|---|---|
| HNSCC | 90 | 10 |
| Breast Cancer | 77 | 105 |
| triple negative BC | 80 | 7 |
| CRC | 77 | 90 |
| PAC | 74 | 19 |
| Gastric Cancer | 68 | 28 |
| NSCLC | 66 | 90 |
| SCLC | 67 | 18 |
| Mesothelioma | 60 | 10 |

Another interesting clinical indication for FAP-DR5 is sarcoma where FAP is expressed not on stroma but on the malignant cells themselves in approximately 50% of cases across sarcoma subtypes (Table 3).

**Table 3: FAP prevalence in human sarcoma subtypes**

| Sarcoma Subtype | N of cases with FAP expression in > 10% of tumour cells | N of samples investigated | % of cases with FAP expression in > 10% of tumor cells |
|---|---|---|---|
| Chondrosarcoma | 6 | 9 | 67% |
| Leiomyosarcoma | 3 | 9 | 33% |
| GIST | 6 | 10 | 60% |
| Fibrosarcoma | 3 | 9 | 33% |
| Osteosarcoma | 2 | 7 | 29% |
| Liposarcoma | 5 | 9 | 56% |
| Malignant fibrous Histiocytoma | 6 | 7 | 85% |

**Example 4: *In vitro* combination studies**

To assess the combination potential of FAP-DR5 with different anti-cancer drugs, a DR5 antibody (drozitumab, described in US2007/0031414) + crosslinking via an anti-Fc antibody was used as a surrogate for crosslinking in the absence of FAP + cells *in vitro.* A good correlation between FAP crosslinking and Fc crosslinking in *in vitro* cell culture experiments was confirmed in a subset of cell lines for proof of principle (data not shown). A panel of CRC and PDAC cell lines was assessed and combination partners of potential clinical relevance for the respective tumor indications were used for evaluation of combination effects (CRC: irinotecan, oxaliplatin, 5-FU, MDM2 inhibitor (RG7388), Bcl-2 inhibitor (ABT199); PDAC: Abraxane, Paclitaxel, Gemcitabine, Doxorubicin, MDM2i (RG7388), Bcl2i (ABT199), Bortezomib, Cyclopamine, PARP inhibitor (PJ34)). Results are described in Table 4 and Table 5 and Figures 2-4.

**Determination of IC50-values of compounds**

Cells were seeded (numbers vary depending on the cell line) in black 96-well microplate with clear, flat bottom and incubated overnight at 37°C and 5% $CO_2$. After checking the adherence/confluence of cells, the medium was removed and 100μl of fresh medium containing the corresponding compound was added to each well. A sequential dilution series (1:4) of 8 concentrations per compound (n=3) were used. Corresponding DMSO concentration and media alone were used as controls.

After incubation for 3 days at 37°C and 5% $CO_2$ 100 μl per well CellTiter-Glo reagent (Promega) was added to the plate. Luminescence was measured after 30 minutes agitation at room temperature. IC50-values were calculated with XL-Fit software. Each experiment with one compound was repeated at least twice.

**DR5 Ab Combination:**

Cells were seeded (numbers vary depending on the cell line) in black 96-well microplate with clear, flat bottom and incubated overnight at 37°C and 5% $CO_2$. After checking the adherence/confluence of cells, the medium was removed and 100μl of fresh medium containing the corresponding compound or combination was added to each well.

A sequential 1:3 dilution series of 9 concentrations of Drozitumab/anti-human-Fc was done on a 96-well PP-V-bottom microplate (n=6). A pre-dilution of Drozitumab/anti-human Fc which were mixed at equimolar concentrations ranging from 0 - 28 nM for Drozitumab/anti-human Fc and 800nM for the chemotherapeutic agents. 25 μl/well of the Drozitumab/anti-human-Fc series were then transferred to the cells. The final concentrations were then reached with 1x IC50-concentration of the compound and either 7 nM or 200 nM for the highest concentration of Drozitumab/anti-human-Fc.

After incubation for 3 days at 37°C and 5% $CO_2$ 100 μl per well CellTiter-Glo reagent (Promega) were given to the plates. Luminescence was measured after 30 minutes agitation at room temperature.

For each concentration of Drozitumab/anti-human-Fc and Drozitumab/anti-human-Fc + compound triplicates were done on the same plate and each experiment was repeated at least twice.

**Table 4: Combination screen PDAC cell lines**

| Cell line | Compound | IC50 monotherapy (nM) | IC50 Combo (nM) | Fold change IC50 (DR5 ab mono vs combo) | % max. inhibition (mono / combo) |
|---|---|---|---|---|---|
| Aspc1 | Abraxane | 20.000 | 0.275 | 18 | 40 / 90 |
| Aspc1 | Paclitaxel | n.c. | 0.160 | 31 | 30/75 |
| Aspc1 | Gemcitabine | 2000.000 | 0.100 | 50 | 25/55 |
| Aspc1 | Doxorubicin | 300.000 | 1.000 | 5 | n.s. |
| Aspc1 | MDM2i (RG7388) | n.c. | n.c. | n.s. | n.s. |
| Aspc1 | Bcl2i (ABT199) | n.c. | n.c. | n.s. | n.s. |
| Aspc1 | Bortezomib | 30.000 | n.c. | n.s. | n.s. |
| Aspc1 | Cyclopamine | n.c. | 1.800 | 3 | n.s. |
| Aspc1 | PARPi | n.c. | 0.300 | 17 | 10 / 70 |
| Aspc1 | DR5 ab + Fc | 5.000 | | | |
| | | | | | |
| Bxpc3 | Abraxane | 20.000 | 0.050 | 60 | 50 /100 |
| Bxpc3 | Paclitaxel | 250.000 | 3.900 | 1 | 80 /100 |
| Bxpc3 | Gemcitabine | 30.000 | 0.200 | 15 | 45 / 80 |
| Bxpc3 | Doxorubicin | 4000.000 | 1.400 | 2 | 25 / 70 |
| Bxpc3 | MDM2i (RG7388) | n.c. | 0.300 | 10 | 45 /100 |
| Bxpc3 | Bcl2i (ABT199) | n.c. | n.c. | n.s. | n.s. |

(continued)

| Cell line | Compound | IC50 monotherapy (nM) | IC50 Combo (nM) | Fold change IC50 (DR5 ab mono vs combo) | % max. inhibition (mono / combo) |
|---|---|---|---|---|---|
| Bxpc3 | Bortezomib | 10.000 | 0.400 | 8 | 60 / 100 |
| Bxpc3 | Cyclopamine | n.c. | 1.800 | 2 | 65 / 95 |
| Bxpc3 | PARPi (PJ34) | n.c. | 0.300 | 10 | n.s. |
| Bxpc3 | DR5 ab + Fc | 3.000 | | | |
| | | | | | |
| Capan2 | Abraxane | n.t. | n.t. | n.c. | n.t. |
| Capan2 | Paclitaxel | n.c. | 1.100 | n.c. | 50 / 80 |
| Capan2 | Gemcitabine | n.c. | 1.100 | n.c. | 35 / 60 |
| Capan2 | Doxorubicin | 1500.000 | 794.00 0 | n.c. | n.s. |
| Capan2 | MDM2i (RG7388) | n.c. | 9.800 | n.c. | 10 / 70 |
| Capan2 | Bcl2i (ABT199) | n.c. | n.c. | n.c. | 20 / 60 |
| Capan2 | Bortezomib | 20.000 | 0.010 | n.c. | 40 / 95 |
| Capan2 | Cyclopamine | n.c. | n.c. | n.c. | n.s. |
| Capan2 | PARPi (PJ34) | n.c. | 5.600 | n.c. | n.s. |
| Capan2 | DR5 ab + Fc | n.c. | | | |
| | | | | | |
| Panc1 | Abraxane | 40.000 | 0.002 | 131000 | 60 / 80 |
| Panc1 | Paclitaxel | 15.000 | 0.030 | 8733 | 50 / 70 |
| Panc1 | Gemcitabine | 2000.000 | 52.900 | 5 | 50 / 75 |
| Panc1 | Doxorubicin | 620.000 | n.c. | n.s. | n.s. |
| Panc1 | MDM2i (RG7388) | n.c. | n.c. | n.s. | n.s. |
| Panc1 | Bcl2i (ABT199) | n.c. | n.c. | n.s. | n.s. |
| Panc1 | Bortezomib | 8.500 | n.c. | n.s. | n.s. |
| Panc1 | Cyclopamine | n.c. | n.c. | n.s. | n.s. |
| Panc1 | PARPi (PJ34) | n.c. | n.c. | n.s. | n.s. |
| Panc1 | DR5 ab + Fc | 262.000 | | | |

**Table 5: Combination screen CRC cell lines**

| Cell line | Compound | IC50 monotherapy (nM) | IC50 combo (nM) | Fold change IC50 (DR5 ab mono vs combo) | % max. inhibition (mono / combo) |
|---|---|---|---|---|---|
| HT29 | Irinotecan | 9000.000 | 0.030 | 83 | **40/100** |
| HT29 | Oxaliplatin | 5000.000 | 0.200 | 13 | **30/90** |
| HT29 | 5-FU | 2500.000 | 0.100 | 25 | 50/85 |
| HT29 | Bcl2i (ABT199) | n.c. | 1.000 | 3 | n.s. |

(continued)

| Cell line | Compound | IC50 monotherapy (nM) | IC50 combo (nM) | Fold change IC50 (DR5 ab mono vs combo) | % max. inhibition (mono / combo) |
|---|---|---|---|---|---|
| HT29 | MDM2i (RG7388) | 10.000 | n.c. | n.s | n.s. |
| HT29 | DR5 ab + Fc | 2.500 | | | |
| | | | | | |
| HCT116 | Irinotecan | 0.150 | n.c. | n.c. | 60/95 |
| HCT116 | Oxaliplatin | 5000.000 | 0.040 | 4 | **20/90** |
| HCT116 | 5-FU | 2500.000 | n.c | n.c. | 60/90 |
| HCT116 | Bcl2i (ABT199) | n.c. | 0.0003 | 500 | 50/80 |
| HCT116 | MDM2i (RG7388) | 10.000 | 0.030 | 5 | 40/95 |
| HCT116 | DR5 ab + Fc | 0.150 | | | |
| | | | | | |
| SW480 | Irinotecan | n.c. | n.c. | n.s. | 60/95 |
| SW480 | Oxaliplatin | n.c. | n.c | n.s. | 60/80 |
| SW480 | 5-FU | n.c. | 0.002 | 70000 | 50/65 |
| SW480 | Bcl2i (ABT199) | n.c. | n.s. | n.s. | 5/30 |
| SW480 | MDM2i (RG7388) | n.c. | 0.300 | 467 | 25/65 |
| SW480 | DR5 ab + Fc | 140.000 | | | |
| n.s. = not significant<br>n.c. = not calculated (as the data not suitable)<br>n.t. = not tested | | | | | |

**Example 5: *In vivo* antitumor efficacy of FAP-DR5 in combination with chemotherapeutics**

The *in vivo* antitumor efficacy of the bispecific antibody DR5-FAP (DR5 binder: VH SEQ ID NO.:7, VL SEQ ID NO.: 8, FAP binder: VH SEQ ID NO.:15, VL SEQ ID NO.: 16) could be detected in cell and fragment based patient derived (PDX) models of various tumor origin (e.g. CRC, pancreatic cancer, desmoplastic melanoma and sarcoma) transplanted on nude mice. As example data for combination efficacy of bispecific antibody DR5-FAP (DR5 binder: VH SEQ ID NO.:7, VL SEQ ID NO.: 8, FAP binder: VH SEQ ID NO.:15, VL SEQ ID NO.: 16) and chemotherapeutics that activate the intrinsic apoptosis pathway are shown for the CRC xenograft model DLD-1 and HCT116 (cell line based, co-injection model) and Co5896 (fragment based).

**Test agents**

The bispecific antibody DR5-FAP (DR5 binder: VH SEQ ID NO.:7, VL SEQ ID NO.: 8, FAP binder: VH SEQ ID NO.:15, VL SEQ ID NO.: 16) was provided as stock solution from Roche, Penzberg, Germany. Antibody buffer included histidine. Antibody solution was diluted appropriately in buffer from stock prior injections. An Fc mutant of the prior art DR5 specific antibody Drozitumab was provided as stock solution from Roche, Penzberg, Germany. This antibody comprises three amino acid substitutions in the Fc domain that abolish binding to an activating or inhibitory Fc receptor and/or effector function wherein said amino acid substitutions are L234A, L235A and P329G. This Fc mutant of Drozitumab is also referred to as "Drozitumab LALA".

### Cell lines and culture conditions

DLD-1 and HCT116 human CRC cells were originally obtained from ATCC; LOX-IMVI human desmoplastic melanoma cells were originally established at NCI and purchased from ATCC. The tumor cell lines were routinely cultured in DMEM high glucose medium with 1.0mM Sodiumpyruvat supplemented with 10% fetal bovine serum, 2.0mM L-glutamine, 10mM HEPES at 37 °C in a water-saturated atmosphere at 5% $CO_2$. Culture passage was performed with trypsin / EDTA 1x splitting every third day. Additionally, murine fibroblasts NIH3T3 were purchased from ATCC and cultured in DMEM high glucose with 1.0mM Sodiumpyruvat, FCS 10% and L-Glutamin 2.0mM.

### Patient-derived xenograft model (PDX)

The CRC tumor xenograft Co5896, sarcoma tumor xenograft Sarc4605 and pancreatic ductal adenocarcinoma (PDAC) tumor xenografts PA1178 and PA3137 were originally obtained from patients and passaged approximately three to five times until establishment of stable growth patterns. For the subsequent *in vivo* studies Co5896, Sarc4605, PA1178 and PA3137 tumor fragments were obtained from xenografts in serial passage in nude mice. After removal from donor mice, tumors were cut into fragments (4-5 mm diameter) and placed in PBS until subcutaneous implantation. Mice under isofluorane anesthesia received unilateral, subcutaneous tumor implants in the flank.

### Animals

Nude mice were purchased from breeder (e.g. Charles River, Sulzfeld, Germany) and maintained under specific-pathogen-free condition with daily cycles of 12 h light /12 h darkness according to committed guidelines (GV-Solas; Felasa; TierschG). Experimental study protocol was reviewed and approved by local government. After arrival animals were maintained in the quarantine part of the animal facility for one week to get accustomed to new environment and for observation. Continuous health monitoring was carried out on regular basis. Diet food (Provimi Kliba 3337) and water (acidified pH 2.5-3) were provided ad libitum.

### Monitoring

Animals were controlled daily for clinical symptoms and detection of adverse effects. For monitoring throughout the experiment body weight of animals was documented.

### Treatment of animals

Animal treatment started after animal randomisation after cell or fragment transplantation when median tumor size was about 100-200mm3. Antibody was administered as single agent at 1.0, 10 or 30mg/kg i.v. once or twice weekly for several weeks depending on the model. The corresponding vehicle was administered on the same days.

### Antibody efficacy

### DLD-1 and HCT116 CRC co-injection cell line based xenograft model

DLD-1 CRC xenograft bearing mice were treated with bispecific antibody DR5-FAP (DR5 binder: VH SEQ ID NO.:7, VL SEQ ID NO.: 8, FAP binder: VH SEQ ID NO.:15, VL SEQ ID NO.: 16) from study day 9 to 20 at dosages of 10 and 1.0mg/kg for 4 times. As a result, treatment with bispecific antibody DR5-FAP (DR5 binder: VH SEQ ID NO.:7, VL SEQ ID NO.: 8, FAP binder: VH SEQ ID NO.:15, VL SEQ ID NO.: 16) showed dose-related significant anti-tumor efficacy with strong anti-tumor efficacy against s.c. DLD-1 xenografts. The Tumor Growth Inhibition (TGI) was calculated at 89% (10mg/kg) and 79% (1.0mg/kg), respectively. In contrast, after treatment with DR5 Fc mutant antibody Drozitumab LALA (10mg/kg, once weekly) no anti-tumor efficacy was noticed (Figure 5). Furthermore in a combination DLD-1 tumor bearing mice were treated with bispecific antibody DR5-FAP (DR5 binder: VH SEQ ID NO.:7, VL SEQ ID NO.: 8, FAP binder: VH SEQ ID NO.:15, VL SEQ ID NO.: 16) (10mg/kg, iv once weekly, 5x) and irinotecan (15mg/kg, ip 5 days, 3x). The combination treatment displayed superior efficacy compared to respective single agent and 72% tumor regression was achieved (Figure 6). Treatment of DLD-1 tumor bearing mice with bispecific antibody DR5-FAP (DR5 binder: VH SEQ ID NO.:7, VL SEQ ID NO.: 8, FAP binder: VH SEQ ID NO.:15, VL SEQ ID NO.: 16) 5 days before, after or in parallel with irinotecan treatment was tested. Combinational treatment in parallel displayed superior efficacy compared to sequential treatment of the two agents (Figure 7). In a further preclinical study DLD-1 tumor bearing mice received a combination treatment of bispecific antibody DR5-FAP (DR5 binder: VH SEQ ID NO.:7, VL SEQ ID NO.: 8, FAP binder: VH SEQ ID NO.:15, VL SEQ ID NO.: 16) (10 mg/kg) together with anti-VEGF antibody B20 (10 mg/kg) or Ang2 / VEGF

antibody (10 mg/kg), as described in WO2011/117329. Both antibodies were given once weekly on days 8, 15 and 22. While treatment with the DR5-FAP antibody as single agent resulted in significant tumor growth inhibition (TGI 87%) the combination with anti-Ang/VEGF Mab was additive efficacious and increased tumor growth inhibition to 94%. Treatment with Ang2/VEGF antibody alone inhibited tumor growth at 75% (Figure 15).

In a second cell line based CRC model (HCT116) the combination of bispecific antibody DR5-FAP (DR5 binder: VH SEQ ID NO.:7, VL SEQ ID NO.: 8, FAP binder: VH SEQ ID NO.:15, VL SEQ ID NO.: 16) (10mg/kg, iv, once weekly, 3x) with irinotecan (15mg/kg, ip, 5 days, 2x) and oxaliplatin (5mg/kg) was evaluated. Treatment started 7 days after implantation and monotherapy with bispecific antibody DR5-FAP (DR5 binder: VH SEQ ID NO.:7, VL SEQ ID NO.: 8, FAP binder: VH SEQ ID NO.:15, VL SEQ ID NO.: 16) resulted in 46% TGI. The combination with irinotecan displayed superior efficacy and caused tumor regression (Figure 8). Furthermore the combination therapy of bispecific antibody DR5-FAP (DR5 binder: VH SEQ ID NO.:7, VL SEQ ID NO.: 8, FAP binder: VH SEQ ID NO.:15, VL SEQ ID NO.: 16) with oxaliplatin improved the efficacy to 67% TGI (Figure 9).

**LOX-IMVI desmoplastic melanoma cell line based xenograft model**

LOX-IMVI xenograft bearing mice were treated with bispecific antibody DR5-FAP (DR5 binder: VH SEQ ID NO.:7, VL SEQ ID NO.: 8, FAP binder: VH SEQ ID NO.:15, VL SEQ ID NO.: 16) from study days 13 to 20 at dosages of 10mg/kg for 2 times. Another group of tumor bearing mice received treatment with DR5 targeting Fc mutant antibody drozitumab LALA at 10mg/kg (days 13 and 20). As a result, treatment with bispecific antibody DR5-FAP (DR5 binder: VH SEQ ID NO.:7, VL SEQ ID NO.: 8, FAP binder: VH SEQ ID NO.:15, VL SEQ ID NO.: 16) showed significant strong anti-tumor efficacy (tumor stasis) against s.c. LOX-IMVI xenografts. The Tumor Growth Inhibition (TGI) was calculated at over 100% (10mg/kg) for bispecific antibody DR5-FAP (DR5 binder: VH SEQ ID NO.:7, VL SEQ ID NO.: 8, FAP binder: VH SEQ ID NO.:15, VL SEQ ID NO.: 16), whereas treatment with drozitumab LALA was less efficacious (TGI 65%) (Figure 10). Additionally LOX-IMVI bearing mice were treated with the bispecific antibody DR5-FAP (DR5 binder: VH SEQ ID NO.:7, VL SEQ ID NO.: 8, FAP binder: VH SEQ ID NO.:15, VL SEQ ID NO.: 16) (10mg/kg) in combination with the anthracyclin doxorubicin (Adriamycin, 5mg/kg, q7d). While treatment with the DR5-FAP antibody (10mg/kg, days 8 and 15) as single agent resulted in tumor stasis (TGI 97%) the combination with doxorubicin was more than additive efficacious and caused distinct tumor regression (93%). Treatment with doxorubicin alone inhibited tumor growth at 77% (Figure 16).

**Co5896 CRC fragment based patient derived xenograft model (PDX)**

Co5896 CRC xenograft bearing mice were treated with bispecific antibody DR5-FAP (DR5 binder: VH SEQ ID NO.:7, VL SEQ ID NO.: 8, FAP binder: VH SEQ ID NO.:15, VL SEQ ID NO.: 16) from study day 18 to 34 at dose of 30mg/kg for 6 times as single agent (see Figure 11). As a result, treatment with bispecific antibody DR5-FAP (DR5 binder: VH SEQ ID NO.:7, VL SEQ ID NO.: 8, FAP binder: VH SEQ ID NO.:15, VL SEQ ID NO.: 16) showed significant anti-tumor efficacy with strong anti-tumor efficacy against s.c. Co5896 patient-derived xenografts. The Tumor Growth Inhibition (TGI) was calculated at 76%. Additionally in a combination study the bispecific antibody DR5-FAP (DR5 binder: VH SEQ ID NO.:7, VL SEQ ID NO.: 8, FAP binder: VH SEQ ID NO.:15, VL SEQ ID NO.: 16) (30mg/kg) was given from study day 15 as single agent once weekly (4 times) and in combination with irinotecan (15mg/kg, days 15-19). Superior efficacy was observed in combination of bispecific DR5-FAP antibody with irinotecan resulting in complete tumor regression in all animals (10/10 tumor free) (Figure 12).

The presence of FAP in the stroma of Co5896 patient-derived xenografts was confirmed by IHC (Figure 13).

**Sarc4605 sarcoma fragment based patient derived xenograft model (PDX)**

Sarc4605 sarcoma xenograft bearing mice were treated with bispecific antibody DR5-FAP (DR5 binder: VH SEQ ID NO.:7, VL SEQ ID NO.: 8, FAP binder: VH SEQ ID NO.:15, VL SEQ ID NO.: 16) from study day 10 to 31 at dose of 10mg/kg for 4 times as single agent (Figure 14). As a result, treatment with bispecific antibody DR5-FAP (DR5 binder: VH SEQ ID NO.:7, VL SEQ ID NO.: 8, FAP binder: VH SEQ ID NO.:15, VL SEQ ID NO.: 16) showed significant anti-tumor efficacy with strong anti-tumor efficacy (tumor stasis) against s.c. Sarc4605 patient-derived xenografts. The Tumor Growth Inhibition (TGI) was calculated over 100%. Additionally, Sarc4605 tumor bearing mice were treated with bispecific antibody DR5-FAP (DR5 binder: VH SEQ ID NO.:7, VL SEQ ID NO.: 8, FAP binder: VH SEQ ID NO.:15, VL SEQ ID NO.: 16) (10mg/kg) in combination with the anthracyclin doxorubicin (Adriamycin, 5mg/kg, q7d). While treatment with the DR5-FAP antibody (10mg/kg, days 20, 27, 34 and 41) as single agent resulted in tumor stasis (TGI 99%) the combination with doxorubicin was more than additive efficacious and caused distinct tumor regression (83%). Treatment with doxorubicin alone inhibited tumor growth at 77% (Figure 17).

Furthermore Sarc4605 tumor bearing mice were treated with bispecific antibody DR5-FAP (DR5 binder: VH

SEQ ID NO.:7, VL SEQ ID NO.: 8, FAP binder: VH SEQ ID NO.:15, VL SEQ ID NO.: 16) (10mg/kg) in combination with alkylating drug ifosfamide (100mg/kg, q7dx2). Treatment with DR5-FAP antibody (10mg/kg, q7dx8) alone resulted in strong tumor regression (96% with 80% tumor free) whereas combination with ifosfamid was slightly more efficacious (86% tumor free). Additionally, the kinetic of tumor regression was faster in combination (Figure 19).

**PA1178 and PA3137 PDAC fragment-based xenograft models (PDX)**

PA1178 xenograft bearing mice were treated with bispecific DR5-FAP antibody (DR5 binder: VH SEQ ID NO.:7, VL SEQ ID NO.: 8, FAP binder: VH SEQ ID NO.:15, VL SEQ ID NO.: 16) from study day 32 to 81 at dose of 10mg/kg for 7 times as single agent and in combination with gemcitabine and nab-paclitaxel (see Figure 18). The bispecific antibody was administered as single agent at 10mg/kg ip once weekly. The corresponding vehicle was administered on the same days. Gemcitabine was given twice weekly ip at 40mg/kg for several weeks and nab-paclitaxel administered iv on four consecutive days at 6mg/kg for two cycles.

As a result, treatment with DR5-FAP bispec antibody (DR5 binder: VH SEQ ID NO.:7, VL SEQ ID NO.: 8, FAP binder: VH SEQ ID NO.:15, VL SEQ ID NO.: 16) showed significant anti-tumor efficacy as single agent with strong anti-tumor efficacy against s.c. PA1178 patient-derived xenografts. The Tumor Growth Inhibition (TGI) was calculated at 96% compared to control. Furthermore DR5-FAP bispec antibody (DR5 binder: VH SEQ ID NO.:7, VL SEQ ID NO.: 8, FAP binder: VH SEQ ID NO.:15, VL SEQ ID NO.: 16) was given in combination with gemcitabine and nab-paclitaxel (abraxane). The triple combination was efficacious with complete tumor remission in all treated animals which was not achieved in the gemcitabine / nab-paclitaxel dual combination dosing group.

In a second fragment based PDAC PDX model (PA3137) the efficacy of bispecific DR5-FAP antibody (DR5 binder: VH SEQ ID NO.:7, VL SEQ ID NO.: 8, FAP binder: VH SEQ ID NO.:15, VL SEQ ID NO.: 16) (10mg/kg, ip, once weekly x8) was evaluated as single agent and in combination with gemcitabine (ip once weekly, 40mg/kg) and nab-paclitaxel (6mg/kg, four consecutive days, iv). Animal treatment started 31 days after implantation until day 66. Efficacy with bispec DR5-FAP antibody (DR5 binder: VH SEQ ID NO.:7, VL SEQ ID NO.: 8, FAP binder: VH SEQ ID NO.:15, VL SEQ ID NO.: 16) in monotherapy resulted in 40% TGI. The combination of bispecific DR5-FAP antibody (DR5 binder: VH SEQ ID NO.:7, VL SEQ ID NO.: 8, FAP binder: VH SEQ ID NO.:15, VL SEQ ID NO.: 16) with gemcitabine and nab-paclitaxel (abraxane) displayed good efficacy with mostly complete tumor remissions (data not shown).

**Example 6: Preclinical pharmacodynamic biomarker and combination strategy**

Preclinical translational studies were conducted to demonstrate the on-target mode of action, to ensure maximal activity and to guide pharmacodynamic (PD) analysis to unravel potential resistance mechanisms.

Material and Methods

A kinetic study was designed in a colorectal cancer (CRC) cell line based xenograft model (DLD-1) co-injected with fibroblasts. Tumors were explanted 6, 16, 72 and 168 hours after bispecific DR5-FAP antibody (DR5 binder: VH SEQ ID NO.:7, VL SEQ ID NO.: 8, FAP binder: VH SEQ ID NO.:15, VL SEQ ID NO.: 16) (10mg/kg), single agent treatment and harvested for immunohistochemical (IHC) and ELISA based protein analysis of apoptosis markers, such as cleaved caspase 3 (cc3), cleaved PARP and activated caspase 8 and 9. Cleaved Caspase 3 levels were determined with Apoptosis Human 3-Plex Panel for Luminex® Platform (Life technologies) MILLIPLEX® MAP 7-Plex was used to determinate changes in phosphorylated Akt (Ser473), JNK (Thr183/Tyr185), Bad (Ser112), Bcl-2 (Ser70), p53 (Ser46), Active Caspase-8 (Asp384) and Active Caspase-9 (Asp315).

For IHC analysis the tumors were fixed in 3.8 % buffered formaldehyde solution for max. 48hrs and embedded in Paraplast. A rabbit polyclonal antibody against **cleaved Caspase-**3 (Asp175; Cell Signalling) was applied using the Ventana Discovery XT slide stainer following a routine IHC staining protocol.

For analysis of bispecific DR5-FAP antibody (DR5 binder: VH SEQ ID NO.:7, VL SEQ ID NO.: 8, FAP binder: VH SEQ ID NO.:15, VL SEQ ID NO.: 16) (10mg/kg) treatment in combination with doxorubicin (5 mg/kg), a FAP positive desmoplastic melanoma cell line derived model (LOX-IMVI) was used. Tumors were explanted 3, 6, 16, 72 and 168 hours after treatment.

Results

We observed significant time-dependent induction of apoptosis upon treatment with the bispecific DR5-FAP antibody by IHC and ELISA in xenograft tumors expressing FAP in stroma or on tumor cells directly. High, transient levels of apoptosis markers such as cc3 were observed by IHC early after treatment compared to vehicle control. Analysis of equivalent tissue lysates by ELISA revealed also rapid induction of cc3, cleaved PARP and activated caspase 8 and

9 in monotherapy in the DLD-1 CRC xenograft model which was superior when given together with doxorubicin in the LOX-IMVI desmoplastic melanoma model. Analysis of equivalent tissue lysates by ELISA revealed rapid induction of cc3, cleaved PARP and activated caspase 8 and 9 in monotherapy in the DLD-1 CRC xenograft model which was superior when given together with, irinotecan or oxaliplatin.

Figure 20 shows Luminex Data (ELISA) after treatment with single agent bispecific DR5-FAP antibody (DR5 binder: VH SEQ ID NO.:7, VL SEQ ID NO.: 8, FAP binder: VH SEQ ID NO.:15, VL SEQ ID NO.: 16). The bispecific DR5-FAP antibody induced strong time-related tumor cells apopotosis against DLD-1/3T3 xenografts. Strong effects were observed shortly after antibody treatment (6h).

Figure 21 shows Luminex Data (ELISA) after treatment with single agent bispecific DR5-FAP antibody (DR5 binder: VH SEQ ID NO.:7, VL SEQ ID NO.: 8, FAP binder: VH SEQ ID NO.:15, VL SEQ ID NO.: 16) or in combination with doxorubicin (10 mg/kg). The bispecific DR5-FAP antibody strongly induces tumor cell apoptosis in a time-related fashion. The tumor cell apoptosis induction is superior with the combination treatment of the bispecific DR5-FAP antibody together with doxorubicin in the LOX-IMVI desmoplastic melanoma model.

Figure 22 shows Luminex Data (ELISA) after treatment with single agent bispecific DR5-FAP antibody (10 mg/kg; DR5 binder: VH SEQ ID NO.:7, VL SEQ ID NO.: 8, FAP binder: VH SEQ ID NO.:15, VL SEQ ID NO.: 16) or in combination with irinotecan (15 mg/kg) or oxaliplatin (5 mg/kg). The bispecific DR5-FAP antibody strongly induces tumor cell apoptosis in a time-related fashion in the DLD-1 CRC xenograft model. The tumor cell apoptosis induction is superior with the combination treatment of the bispecific DR5-FAP antibody together with irinotecan or oxaliplatin.

We identified that the the bispecific DR5-FAP antibody (DR5 binder: VH SEQ ID NO.:7, VL SEQ ID NO.: 8, FAP binder: VH SEQ ID NO.:15, VL SEQ ID NO.: 16) strongly induces tumor cell apoptosis in vivo shortly after injection independently if FAP was expressed on tumor stroma or at tumor cells and discovered optimal pharmacodynamic markers and time points for sampling and analysis.

**Sequences**

**1. Amino acid sequences of phage display derived DR5 binders**

| Description | Amino acid sequence | SEQ ID NO. |
|---|---|---|
| DR5 (5E11)_ VH | EVQLLESGGGLVQPGGSLRLSCAASGFTFSSYAMSWV RQAPGKGLEWVSAISGSGGSTYYADSVKGRFTISRDNS KNTLYLQMNSLRAEDTAVYYCAKGVRVSFDYWGQGT LVTVSS | 7 |
| DR5 (5E11)_CDRH1 | SYAMS | 1 |
| DR5 (5E11)_CDRH2 | AISGSGGSTYYADSVKG | 2 |
| DR5 (5E11)_CDRH3 | GVRVSFDY | 3 |
| DR5 (5E11)_ VL | EIVLTQSPGTLSLSPGERATLSCRASQSVSSSYLAWYQQ KPGQAPRLLIYGASSRATGIPDRFSGSGSGTDFTLTISRL EPEDFAVYYCQQGTTHPITFGQGTKVEIK | 8 |
| DR5 (5E11)_CDRL1 | RASQSVSSSYLA | 4 |
| DR5 (5E11)_CDRL2 | GASSRAT | 5 |
| DR5 (5E11)_CDRL3 | QQGTTHPIT | 6 |

**2. Amino acid sequences of FAP binders**

| Name | Amino acid sequence | SEQ ID NO |
|---|---|---|
| FAP(28H1)_VH | EVQLLESGGGLVQPGGSLRLSCAASGFTFSSHAMSWV RQAPGKGLEWVSAIWASGEQYYADSVKGRFTISRDNS KNTLYLQMNSLRAEDTAVYYCAKGWLGNFDYWGQG TLVTVSS | 15 |
| FAP(28H1)_VL | EIVLTQSPGTLSLSPGERATLSCRASQSVSRSYLAWYQQ KPGQAPRLLIIGASTRATGIPDRFSGSGSGTDFTLTISRLE PEDFAVYYCQQGQVIPPTFGQGTKVEIK | 16 |
| FAP (28H1)_ CDRH1 | SHAMS | 9 |
| FAP (28H1)_ CDRH2 | AIWASGEQYYADSVKG | 10 |
| FAP (28H1)_ CDRH3 | GWLGNFDY | 11 |
| FAP (28H1)_ CDRL1 | RASQSVSRSYLA | 12 |
| FAP (28H1)_ CDRL2 | GASTRAT | 13 |
| FAP (28H1)_ CDRL3 | QQGQVIPPT | 14 |

**3. Amino acid sequences of bispecific molecules comprising phage display derived DR5 binders**

| Name | Amino acid sequence | SEQ ID NO: |
|---|---|---|
| DR5(5E11)-FAP (28H1) VHCL pETR10334 2+2 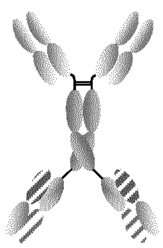 | EVQLLESGGGLVQPGGSLRLSCAASGFTFSSYAMSWVRQAP GKGLEWVSAISGSGGSTYYADSVKGRFTISRDNSKNTLYLQ MNSLRAEDTAVYYCAKGVRVSFDYWGQGTLVTVSSASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSRDELTKNQVSLTCLVK GFYPSDIAVEWESNGQPENNYKTTPPVLDSDGSFFLYSKLTV DKSRWQQGNVFSCSVMHEALHNHYTQKSLSLSPGKSGGGG SGGGGSGGGGSGGGGSEVQLLESGGGLVQPGGSLRLSCAAS GFTFSSHAMSWVRQAPGKGLEWVSAIWASGEQYYADSVKG RFTISRDNSKNTLYLQMNSLRAEDTAVYYCAKGWLGNFDY WGQGTLVTVSSASVAAPSVFIFPPSDEQLKSGTASVVCLLNN FYPREAKVQWKVDNALQSGNSQESVTEQDSKDSTYSLSSTL TLSKADYEKHKVYACEVTHQGLSSPVTKSFNRGEC | 17 |

(continued)

| Name | Amino acid sequence | SEQ ID NO: |
|---|---|---|
| DR5(5E11)-FAP (28H1) VHCL 2+2 Removal of C-term. Lysine in Fc P329G/LALA mut. pETR1 1025 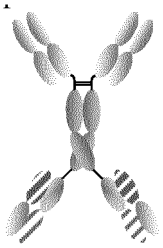 | EVQLLESGGGLVQPGGSLRLSCAASGFTFSSYAMSWVRQAPGKGLEWVSAISGSGGSTYYADSVKGRFTISRDNSKNTLYLQMNSLRAEDTAVYYCAKGVRVSFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGGGGGSGGGGSGGGGSGGGGSEVQLLESGGGLVQPGGSLRLSCAASGFTFSSHAMSWVRQAPGKGLEWVSAIWASGEQYYADSVKGRFTISRDNSKNTLYLQMNSLRAEDTAVYYCAKGWLGNFDYWGQGTLVTVSSASVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | 18 |
| DR5(5E11) LC pETR9044 | EIVLTQSPGTLSLSPGERATLSCRASQSVSSSYLAWYQQKPGQAPRLLIYGASSRATGIPDRFSGSGSGTDFTLTISRLEPEDFAVYYCQQGTTHPITFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | 19 |
| FAP (28H1) _VLCH1 pETR9537 | EIVLTQSPGTLSLSPGERATLSCRASQSVSRSYLAWYQQKPGQAPRLLIIGASTRATGIPDRFSGSGSGTDFTLTISRLEPEDFAVYYCQQGQVIPPTFGQGTKVEIKSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCD | 20 |

**Claims**

**1.** A bispecific antibody that binds to Death Receptor 5 (DR5) and Fibroblast Activation Protein (FAP) comprising at

least one antigen binding site specific for DR5 and at least one antigen binding site specific for FAP in combination with a chemotherapeutic agent for use in the treatment of cancer, wherein the chemotherapeutic agent is selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody, wherein
the antigen binding site specific for DR5 comprises:

(a) a heavy chain complementarity determining region 1 (CDR1) of SEQ ID NO.:1;
(b) a heavy chain complementarity determining region 2 (CDR2) of SEQ ID NO.:2;
(c) a heavy chain complementarity determining region 3 (CDR3) of SEQ ID NO.:3;
(d) a light chain complementarity determining region 1 (CDR1) of SEQ ID NO.:4;
(e) a light chain complementarity determining region 2 (CDR2) of SEQ ID NO.:5; and
(f) a light chain complementarity determining region 3 (CDR3) of SEQ ID NO.:6.

and wherein
the antigen binding site specific for FAP comprises:

(a) a heavy chain complementarity determining region 1 (CDR1) of SEQ ID NO.:9;
(b) a heavy chain complementarity determining region 2 (CDR2) of SEQ ID NO.:10;
(c) a heavy chain complementarity determining region 3 (CDR3) of SEQ ID NO.:1 1;
(d) a light chain complementarity determining region 1 (CDR1) of SEQ ID NO.:12;
(e) a light chain complementarity determining region 2 (CDR2) of SEQ ID NO.:13; and
(f) a light chain complementarity determining region 3 (CDR3) of SEQ ID NO.:14.

**2.** The bispecific antibody that binds to Death Receptor 5 (DR5) and Fibroblast Activation Protein (FAP) in combination with a chemotherapeutic agent for use in the treatment of claim 1 wherein the cancer is colorectal cancer, sarcoma, head and neck cancer, squamous cell carcinoma, breast cancer, pancreatic cancer, gastric cancer, non-small-cell lung carcinoma, small-cell lung cancer and mesothelioma.

**3.** The bispecific antibody that binds to Death Receptor 5 (DR5) and Fibroblast Activation Protein (FAP) in combination with a chemotherapeutic agent for use in the treatment of claim 2, wherein the sarcoma is chondrosarcoma, leiomyosarcoma, gastrointestinal stromal tumours, fibrosarcoma, osteosarcoma, liposarcoma or maligant fibrous histiocytoma.

**4.** The bispecific antibody that binds to Death Receptor 5 (DR5) and Fibroblast Activation Protein (FAP) in combination with a chemotherapeutic agent for use in the treatment of any one of claims 1 to 3, wherein the bispecific antibody and the chemotherapeutic agent are administered together.

**5.** The bispecific antibody that binds to Death Receptor 5 (DR5) and Fibroblast Activation Protein (FAP) in combination with a chemotherapeutic agent for use in the treatment of claim 4, wherein the bispecific antibody and the chemotherapeutic agent are administered as a combined formulation.

**6.** The bispecific antibody that binds to Death Receptor 5 (DR5) and Fibroblast Activation Protein (FAP) in combination with a chemotherapeutic agent for use in the treatment of any one of claims 1 to 3, wherein the bispecific antibody and the chemotherapeutic agent are administered by alternation.

**7.** The bispecific antibody that binds to Death Receptor 5 (DR5) and Fibroblast Activation Protein (FAP) in combination with a chemotherapeutic agent for use in the treatment of claim 6, wherein the chemotherapeutic agent is administered before the bispecific antibody.

**8.** The bispecific antibody that binds to Death Receptor 5 (DR5) and Fibroblast Activation Protein (FAP) in combination with a chemotherapeutic agent for use in the treatment of claim 6, wherein the chemotherapeutic agent is administered after the bispecific antibody.

**9.** The bispecific antibody that binds to Death Receptor 5 (DR5) and Fibroblast Activation Protein (FAP) in combination with a chemotherapeutic agent for use in the treatment of any one of claims 1 to 8, wherein the combination is administered at intervals from about one week to three weeks.

**10.** The bispecific antibody that binds to Death Receptor 5 (DR5) and Fibroblast Activation Protein (FAP) in combination with a chemotherapeutic agent for use in the treatment of any one of claims 1 to 9, wherein the antigen binding site specific for DR5 comprises a variable heavy chain comprising an amino acid sequence of SEQ ID NO.:7 and a variable light chain comprising an amino acid sequence of SEQ ID NO.:8; and the antigen binding site specific for FAP comprises a heavy chain variable region comprising an amino acid sequence of SEQ ID NO.:15 and a light chain variable region comprising an amino acid sequence of SEQ ID NO.:16.

**11.** The bispecific antibody that binds to Death Receptor 5 (DR5) and Fibroblast Activation Protein (FAP) in combination with a chemotherapeutic agent for use in the treatment of any one of claims 1 to 10, wherein the bispecific antibody comprises amino acid sequences SEQ ID NO: 18, 19 and 20 or the bispecific antibody comprises amino acid sequences SEQ ID NO: 17, 19 and 20.

**12.** The bispecific antibody that binds to Death Receptor 5 (DR5) and Fibroblast Activation Protein (FAP) in combination with a chemotherapeutic agent for use in the treatment of any of claims 1 to 11, wherein the bispecific antibody comprises an Fc domain, two Fab fragments comprising each an antigen binding site specific for DR5, and two Fab fragments comprising each an antigen binding site specific for FAP.

**13.** The bispecific antibody that binds to Death Receptor 5 (DR5) and Fibroblast Activation Protein (FAP) in combination with a chemotherapeutic agent for use in the treatment of claim 12, wherein either the variable regions or the constant regions of the heavy and light chain of the Fab fragment(s) comprising an antigen binding site specific for FAP are exchanged.

**14.** The bispecific antibody that binds to Death Receptor 5 (DR5) and Fibroblast Activation Protein (FAP) in combination with a chemotherapeutic agent for use in the treatment of any one of claims 12 or 13, wherein at least one of the Fab fragments are connected to the Fc domain via a peptide linker.

**15.** The bispecific antibody that binds to Death Receptor 5 (DR5) and Fibroblast Activation Protein (FAP) in combination with a chemotherapeutic agent for use in the treatment of any one of claims 12 to 14, wherein the Fc domain comprises one or more amino acid substitution that reduces binding to an Fc receptor and/or effector function, wherein said one or more amino acid substitution is at one or more position selected from the group of L234, L235, and P329.

**16.** The bispecific antibody that binds to Death Receptor 5 (DR5) and Fibroblast Activation Protein (FAP) in combination with a chemotherapeutic agent for use in the treatment of claim 15, wherein each subunit of the Fc domain comprises three amino acid substitutions that reduce binding to an activating or inhibitory Fc receptor and/or effector function wherein said amino acid substitutions are L234A, L235A and P329G.

**17.** A kit for use in the treatment of cancer comprising:

a first container comprising a composition which comprises a bispecific antibody that binds to Death Receptor 5 (DR5) and Fibroblast Activation Protein (FAP) comprising at least one antigen binding site specific for DR5 and at least one antigen binding site specific for FAP, wherein
the antigen binding site specific for DR5 comprises:

(a) a heavy chain complementarity determining region 1 (CDR1) of SEQ ID NO.:1;
(b) a heavy chain complementarity determining region 2 (CDR2) of SEQ ID NO.:2;
(c) a heavy chain complementarity determining region 3 (CDR3) of SEQ ID NO.:3;
(d) a light chain complementarity determining region 1 (CDR1) of SEQ ID NO.:4;
(e) a light chain complementarity determining region 2 (CDR2) of SEQ ID NO.:5; and
(f) a light chain complementarity determining region 3 (CDR3) of SEQ ID NO.:6. and wherein

the antigen binding site specific for FAP comprises:

(a) a heavy chain complementarity determining region 1 (CDR1) of SEQ ID NO.:9;
(b) a heavy chain complementarity determining region 2 (CDR2) of SEQ ID NO.:10;
(c) a heavy chain complementarity determining region 3 (CDR3) of SEQ ID NO.:11;
(d) a light chain complementarity determining region 1 (CDR1) of SEQ ID NO.:12;
(e) a light chain complementarity determining region 2 (CDR2) of SEQ ID NO.:13; and

(f) a light chain complementarity determining region 3 (CDR3) of SEQ ID NO.:14;

a second container comprising a composition comprising a chemotherapeutic agent selected from Irinotecan, Doxorubicin, Oxaliplatin, 5-FU, the MDM2 inhibitor RG7388, the Bcl-2 inhibitor ABT199, Abraxane, Paclitaxel, Gemcitabine, Bortezomib, Cyclopamine, the PARP inhibitor PJ34, Ifosfamide or an anti-VEGF antibody.

## Patentansprüche

1. Bispezifischer Antikörper, der an Todesrezeptor 5 (DR5) und Fibroblasten-Aktivierungsprotein (FAP) bindet, umfassend mindestens eine für DR5 spezifische Antigenbindungsstelle und mindestens eine für FAP spezifische Antigenbindungsstelle, in Kombination mit einem Chemotherapeutikum zur Verwendung bei der Behandlung von Krebs, wobei das Chemotherapeutikum aus Irinotecan, Doxorubicin, Oxaliplatin, 5-FU, dem MDM2-Inhibitor RG7388, dem Bcl-2-Inhibitor ABT199, Abraxane, Paclitaxel, Gemcitabin, Bortezomib, Cyclopamin, dem PARP-Inhibitor PJ34, Ifosfamid oder einem Anti-VEGF-Antikörper ausgewählt ist, wobei
die für DR5 spezifische Antigenbindungsstelle Folgendes umfasst:

   (a) eine komplementaritätsbestimmende Region 1 (CDR1) der schweren Kette von SEQ ID NO:1;
   (b) eine komplementaritätsbestimmende Region 2 (CDR2) der schweren Kette von SEQ ID NO:2;
   (c) eine komplementaritätsbestimmende Region 3 (CDR3) der schweren Kette von SEQ ID NO:3;
   (d) eine komplementaritätsbestimmende Region 1 (CDR1) der leichten Kette von SEQ ID NO:4;
   (e) eine komplementaritätsbestimmende Region 2 (CDR2) der leichten Kette von SEQ ID NO:5 und
   (f) eine komplementaritätsbestimmende Region 3 (CDR3) der leichten Kette von SEQ ID NO:6

   und wobei
   die für FAP spezifische Antigenbindungsstelle Folgendes umfasst:

   (a) eine komplementaritätsbestimmende Region 1 (CDR1) der schweren Kette von SEQ ID NO:9;
   (b) eine komplementaritätsbestimmende Region 2 (CDR2) der schweren Kette von SEQ ID NO:10;
   (c) eine komplementaritätsbestimmende Region 3 (CDR3) der schweren Kette von SEQ ID NO:11;
   (d) eine komplementaritätsbestimmende Region 1 (CDR1) der leichten Kette von SEQ ID NO:12;
   (e) eine komplementaritätsbestimmende Region 2 (CDR2) der leichten Kette von SEQ ID NO:13 und
   (f) eine komplementaritätsbestimmende Region 3 (CDR3) der leichten Kette von SEQ ID NO:14.

2. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach Anspruch 1, wobei der Krebs Dickdarmkrebs, Sarkom, Kopf- und Halskrebs, Plattenepithelkarzinom, Brustkrebs, Bauchspeicheldrüsenkrebs, Magenkrebs, nichtkleinzelliges Lungenkarzinom, kleinzelliger Lungenkrebs und Mesotheliom ist.

3. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach Anspruch 2, wobei das Sarkom Chondrosarkom, Leiomyosarkom, Magen-Darm-Stromatumoren, Fibrosarkom, Osteosarkom, Liposarkom oder malignes fibröses Histiozytom ist.

4. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach einem der Ansprüche 1 bis 3, wobei der bispezifische Antikörper und das Chemotherapeutikum zusammen verabreicht werden.

5. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach Anspruch 4, wobei der bispezifische Antikörper und das Chemotherapeutikum als eine kombinierte Formulierung verabreicht werden.

6. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach einem der Ansprüche 1 bis 3, wobei der bispezifische Antikörper und das Chemotherapeutikum abwechselnd verabreicht werden.

7. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach Anspruch 6, wobei das

Chemotherapeutikum vor dem bispezifischen Antikörper verabreicht wird.

8. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach Anspruch 6, wobei das Chemotherapeutikum nach dem bispezifischen Antikörper verabreicht wird.

9. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach einem der Ansprüche 1 bis 8, wobei die Kombination in Intervallen von etwa einer Woche bis drei Wochen verabreicht wird.

10. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach einem der Ansprüche 1 bis 9, wobei die für DR5 spezifische Antigenbindungsstelle eine variable schwere Kette, umfassend eine Aminosäuresequenz von SEQ ID NO:7, und eine variable leichte Kette, umfassend eine Aminosäuresequenz von SEQ ID NO:8, umfasst und die für FAP spezifische Antigenbindungsstelle eine variable Region der schweren Kette, umfassend eine Aminosäuresequenz von SEQ ID NO:15, und eine variable Region der leichten Kette, umfassend eine Aminosäuresequenz von SEQ ID NO:16, umfasst.

11. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach einem der Ansprüche 1 bis 10, wobei der bispezifische Antikörper die Aminosäuresequenzen SEQ ID NO:18, 19 und 20 umfasst oder der bispezifische Antikörper die Aminosäuresequenzen SEQ ID NO:17, 19 und 20 umfasst.

12. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach einem der Ansprüche 1 bis 11, wobei der bispezifische Antikörper eine Fc-Domäne, zwei Fab-Fragmente, die jeweils eine für DR5 spezifische Antigenbindungsstelle umfassen, und zwei Fab-Fragmente, die jeweils eine für FAP spezifische Antigenbindungsstelle umfassen, umfasst.

13. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach Anspruch 12, wobei entweder die variablen Regionen oder die konstanten Regionen der schweren und leichten Ketten des/der Fab-Fragments/Fragmente, das/die eine für FAP spezifische Antigenbindungsstelle umfasst/umfassen, ausgetauscht sind.

14. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach einem der Ansprüche 12 oder 13, wobei mindestens eines der Fab-Fragmente über einen Peptid-Linker mit der Fc-Domäne verbunden ist.

15. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach einem der Ansprüche 12 bis 14, wobei die Fc-Domäne eine oder mehrere Aminosäuresubstitutionen umfasst, die das Binden an einen Fc-Rezeptor und/oder die Effektorfunktion reduzieren, wobei die eine oder mehreren Aminosäuresubstitutionen an einer oder mehreren Stellen, ausgewählt aus der Gruppe von L234, L235 und P329, vorliegen.

16. Bispezifischer Antikörper, der an Todesrezeptor 5 (DR5) und Fibroblasten-Aktivierungsprotein (FAP) bindet, in Kombination mit einem Chemotherapeutikum zur Verwendung bei der Behandlung nach Anspruch 15, wobei jede Untereinheit der Fc-Domäne drei Aminosäuresubstitutionen umfasst, die das Binden an einen aktivierenden oder inhibierenden Fc-Rezeptor und/oder die Effektorfunktion reduzieren, wobei die Aminosäuresubstitutionen L234A, L235A und P329G sind.

17. Kit zur Verwendung bei der Behandlung von Krebs, umfassend:

einen ersten Behälter, umfassend eine Zusammensetzung, die einen bispezifischen Antikörper umfasst, der an Todesrezeptor 5 (DR5) und Fibroblasten-Aktivierungsprotein (FAP) bindet, umfassend mindestens eine für DR5 spezifische Antigenbindungsstelle und mindestens eine für FAP spezifische Antigenbindungsstelle, wobei die für DR5 spezifische Antigenbindungsstelle Folgendes umfasst:

(a) eine komplementaritätsbestimmende Region 1 (CDR1) der schweren Kette von SEQ ID NO:1;

(b) eine komplementaritätsbestimmende Region 2 (CDR2) der schweren Kette von SEQ ID NO:2;
(c) eine komplementaritätsbestimmende Region 3 (CDR3) der schweren Kette von SEQ ID NO:3;
(d) eine komplementaritätsbestimmende Region 1 (CDR1) der leichten Kette von SEQ ID NO:4;
(e) eine komplementaritätsbestimmende Region 2 (CDR2) der leichten Kette von SEQ ID NO:5 und
(f) eine komplementaritätsbestimmende Region 3 (CDR3) der leichten Kette von SEQ ID NO:6

und wobei
die für FAP spezifische Antigenbindungsstelle Folgendes umfasst:

(a) eine komplementaritätsbestimmende Region 1 (CDR1) der schweren Kette von SEQ ID NO:9;
(b) eine komplementaritätsbestimmende Region 2 (CDR2) der schweren Kette von SEQ ID NO:10;
(c) eine komplementaritätsbestimmende Region 3 (CDR3) der schweren Kette von SEQ ID NO:11;
(d) eine komplementaritätsbestimmende Region 1 (CDR1) der leichten Kette von SEQ ID NO:12;
(e) eine komplementaritätsbestimmende Region 2 (CDR2) der leichten Kette von SEQ ID NO:13 und
(f) eine komplementaritätsbestimmende Region 3 (CDR3) der leichten Kette von SEQ ID NO:14;

einen zweiten Behälter, umfassend eine Zusammensetzung, die ein Chemotherapeutikum umfasst, das aus Irinotecan, Doxorubicin, Oxaliplatin, 5-FU, dem MDM2-Inhibitor RG7388, dem Bcl-2-Inhibitor ABT199, Abraxane, Paclitaxel, Gemcitabin, Bortezomib, Cyclopamin, dem PARP-Inhibitor PJ34, Ifosfamid oder einem Anti-VEGF-Antikörper ausgewählt ist.

## Revendications

1. Anticorps bispécifique qui se lie au récepteur de mort 5 (DR5) et à la protéine d'activation des fibroblastes (FAP) comprenant au moins un site de liaison à l'antigène spécifique de DR5 et au moins un site de liaison à l'antigène spécifique de FAP en combinaison avec un agent chimiothérapeutique pour une utilisation dans le traitement d'un cancer, dans lequel l'agent chimiothérapeutique est choisi parmi l'irinotécan, la doxorubicine, l'oxaliplatine, le 5-FU, l'inhibiteur de MDM2 RG7388, l'inhibiteur de Bcl-2 ABT199, l'abraxane, le paclitaxel, la gemcitabine, le bortézomib, la cyclopamine, l'inhibiteur de PARP PJ34, l'ifosfamide ou un anticorps anti-VEGF, dans lequel
le site de liaison à l'antigène spécifique de DR5 comprend :

(a) une région déterminant la complémentarité 1 (CDR1) de chaîne lourde de SEQ ID NO:1;
(b) une région déterminant la complémentarité 2 (CDR2) de chaîne lourde de SEQ ID NO:2;
(c) une région déterminant la complémentarité 3 (CDR3) de chaîne lourde de SEQ ID NO:3;
(d) une région déterminant la complémentarité 1 (CDR1) de chaîne légère de SEQ ID NO:4;
(e) une région déterminant la complémentarité 2 (CDR2) de chaîne légère de SEQ ID NO: 5 ; et
(f) une région déterminant la complémentarité 3 (CDR3) de chaîne légère de SEQ ID NO:6;

et dans lequel
le site de liaison à l'antigène spécifique de FAP comprend :

(a) une région déterminant la complémentarité 1 (CDR1) de chaîne lourde de SEQ ID NO:9;
(b) une région déterminant la complémentarité 2 (CDR2) de chaîne lourde de SEQ ID NO: 10 ;
(c) une région déterminant la complémentarité 3 (CDR3) de chaîne lourde de SEQ ID NO: 11 ;
(d) une région déterminant la complémentarité 1 (CDR1) de chaîne légère de SEQ ID NO: 12 ;
(e) une région déterminant la complémentarité 2 (CDR2) de chaîne légère de SEQ ID NO: 13 ; et
(f) une région déterminant la complémentarité 3 (CDR3) de chaîne légère de SEQ ID NO: 14.

2. Anticorps bispécifique qui se lie au récepteur de mort 5 (DR5) et à la protéine d'activation des fibroblastes (FAP) en combinaison avec un agent chimiothérapeutique pour une utilisation dans le traitement selon la revendication 1, dans lequel le cancer est un cancer colorectal, un sarcome, un cancer de la tête et du cou, un carcinome à cellules squameuses, un cancer du sein, un cancer pancréatique, un cancer gastrique, un carcinome pulmonaire non à petites cellules, un cancer du poumon à petites cellules et un mésothéliome.

3. Anticorps bispécifique qui se lie au récepteur de mort 5 (DR5) et à la protéine d'activation des fibroblastes (FAP) en combinaison avec un agent chimiothérapeutique pour une utilisation dans le traitement selon la revendication 2, dans lequel le sarcome est un chondrosarcome, un léiomyosarcome, des tumeurs stromales gastro-intestinales,

un fibrosarcome, un ostéosarcome, un liposarcome ou un histiocytome fibreux malin.

4. Anticorps bispécifique qui se lie au récepteur de mort 5 (DR5) et à la protéine d'activation des fibroblastes (FAP) en combinaison avec un agent chimiothérapeutique pour une utilisation dans le traitement selon l'une quelconque des revendications 1 à 3, dans lequel l'anticorps bispécifique et l'agent chimiothérapeutique sont administrés ensemble.

5. Anticorps bispécifique qui se lie au récepteur de mort 5 (DR5) et à la protéine d'activation des fibroblastes (FAP) en combinaison avec un agent chimiothérapeutique pour une utilisation dans le traitement selon la revendication 4, dans lequel l'anticorps bispécifique et l'agent chimiothérapeutique sont administrés sous la forme d'une formulation combinée.

6. Anticorps bispécifique qui se lie au récepteur de mort 5 (DR5) et à la protéine d'activation des fibroblastes (FAP) en combinaison avec un agent chimiothérapeutique pour une utilisation dans le traitement selon l'une quelconque des revendications 1 à 3, dans lequel l'anticorps bispécifique et l'agent chimiothérapeutique sont administrés en alternance.

7. Anticorps bispécifique qui se lie au récepteur de mort 5 (DR5) et à la protéine d'activation des fibroblastes (FAP) en combinaison avec un agent chimiothérapeutique pour une utilisation dans le traitement selon la revendication 6, dans lequel l'agent chimiothérapeutique est administré avant l'anticorps bispécifique.

8. Anticorps bispécifique qui se lie au récepteur de mort 5 (DR5) et à la protéine d'activation des fibroblastes (FAP) en combinaison avec un agent chimiothérapeutique pour une utilisation dans le traitement selon la revendication 6, dans lequel l'agent chimiothérapeutique est administré après l'anticorps bispécifique.

9. Anticorps bispécifique qui se lie au récepteur de mort 5 (DR5) et à la protéine d'activation des fibroblastes (FAP) en combinaison avec un agent chimiothérapeutique pour une utilisation dans le traitement selon l'une quelconque des revendications 1 à 8, dans lequel la combinaison est administrée à des intervalles d'environ une semaine à trois semaines.

10. Anticorps bispécifique qui se lie au récepteur de mort 5 (DR5) et à la protéine d'activation des fibroblastes (FAP) en combinaison avec un agent chimiothérapeutique pour une utilisation dans le traitement selon l'une quelconque des revendications 1 à 9, dans lequel le site de liaison à l'antigène spécifique de DR5 comprend une chaîne lourde variable comprenant une séquence d'acides aminés de SEQ ID NO: 7 et une chaîne légère variable comprenant une séquence d'acides aminés de SEQ ID NO: 8 ; et le site de liaison à l'antigène spécifique de FAP comprend une région variable de chaîne lourde comprenant une séquence d'acides aminés de SEQ ID NO : 15 et une région variable de chaîne légère comprenant une séquence d'acides aminés de SEQ ID NO: 16.

11. Anticorps bispécifique qui se lie au récepteur de mort 5 (DR5) et à la protéine d'activation des fibroblastes (FAP) en combinaison avec un agent chimiothérapeutique pour une utilisation dans le traitement selon l'une quelconque des revendications 1 à 10, dans lequel l'anticorps bispécifique comprend les séquences d'acides aminés SEQ ID NO: 18, 19 et 20 ou l'anticorps bispécifique comprend les séquences d'acides aminés SEQ ID NO: 17, 19 et 20.

12. Anticorps bispécifique qui se lie au récepteur de mort 5 (DR5) et à la protéine d'activation des fibroblastes (FAP) en combinaison avec un agent chimiothérapeutique pour une utilisation dans le traitement selon l'une quelconque des revendications 1 à 11, dans lequel l'anticorps bispécifique comprend un domaine Fc, deux fragments Fab comprenant chacun un site de liaison à l'antigène spécifique de DR5 et deux fragments Fab comprenant chacun un site de liaison à l'antigène spécifique de FAP.

13. Anticorps bispécifique qui se lie au récepteur de mort 5 (DR5) et à la protéine d'activation des fibroblastes (FAP) en combinaison avec un agent chimiothérapeutique pour une utilisation dans le traitement selon la revendication 12, dans lequel soit les régions variables, soit les régions constantes des chaînes lourde et légère du ou des fragments Fab comprenant un site de liaison à l'antigène spécifique de FAP sont échangées.

14. Anticorps bispécifique qui se lie au récepteur de mort 5 (DR5) et à la protéine d'activation des fibroblastes (FAP) en combinaison avec un agent chimiothérapeutique pour une utilisation dans le traitement selon l'une quelconque des revendications 12 ou 13, dans lequel au moins l'un des fragments Fab est connecté au domaine Fc via un lieur peptidique.

**15.** Anticorps bispécifique qui se lie au récepteur de mort 5 (DR5) et à la protéine d'activation des fibroblastes (FAP) en combinaison avec un agent chimiothérapeutique pour une utilisation dans le traitement selon l'une quelconque des revendications 12 à 14, dans lequel le domaine Fc comprend une ou plusieurs substitutions d'acides aminés qui réduisent la liaison à un récepteur Fc et/ou à une fonction effectrice, dans lequel lesdites une ou plusieurs substitutions d'acides aminés sont en une ou plusieurs positions choisies dans le groupe de L234, L235 et P329.

**16.** Anticorps bispécifique qui se lie au récepteur de mort 5 (DR5) et à la protéine d'activation des fibroblastes (FAP) en combinaison avec un agent chimiothérapeutique pour une utilisation dans le traitement selon la revendication 15, dans lequel chaque sous-motif du domaine Fc comprend trois substitutions d'acides aminés qui réduisent la liaison à un récepteur Fc activateur ou inhibiteur et/ou à une fonction effectrice dans lequel lesdites substitutions d'acides aminés sont L234A, L235A et P329G.

**17.** Kit pour une utilisation dans le traitement d'un cancer comprenant :

un premier récipient comprenant une composition qui comprend un anticorps bispécifique qui se lie au récepteur de mort 5 (DR5) et à la protéine d'activation des fibroblastes (FAP) comprenant au moins un site de liaison à l'antigène spécifique de DR5 et au moins un site de liaison à l'antigène spécifique de FAP, dans lequel le site de liaison à l'antigène spécifique de DR5 comprend :

(a) une région déterminant la complémentarité 1 (CDR1) de chaîne lourde de SEQ ID NO: 1;
(b) une région déterminant la complémentarité 2 (CDR2) de chaîne lourde de SEQ ID NO: 2;
(c) une région déterminant la complémentarité 3 (CDR3) de chaîne lourde de SEQ ID NO: 3 ;
(d) une région déterminant la complémentarité 1 (CDR1) de chaîne légère de SEQ ID NO: 4;
(e) une région déterminant la complémentarité 2 (CDR2) de chaîne légère de SEQ ID NO: 5 ; et
(f) une région déterminant la complémentarité 3 (CDR3) de chaîne légère de SEQ ID NO: 6;

et dans lequel
le site de liaison à l'antigène spécifique de FAP comprend :

(a) une région déterminant la complémentarité 1 (CDR1) de chaîne lourde de SEQ ID NO: 9;
(b) une région déterminant la complémentarité 2 (CDR2) de chaîne lourde de SEQ ID NO: 10 ;
(c) une région déterminant la complémentarité 3 (CDR3) de chaîne lourde de SEQ ID NO: 11 ;
(d) une région déterminant la complémentarité 1 (CDR1) de chaîne légère de SEQ ID NO: 12 ;
(e) une région déterminant la complémentarité 2 (CDR2) de chaîne légère de SEQ ID NO : 13 ; et
(f) une région déterminant la complémentarité 3 (CDR3) de chaîne légère de SEQ ID NO: 14 ;

un second récipient comprenant une composition comprenant un agent chimiothérapeutique choisi parmi l'irinotécan, la doxorubicine, l'oxaliplatine, le 5-FU, l'inhibiteur de MDM2 RG7388, l'inhibiteur de Bcl-2 ABT199, l'abraxane, le paclitaxel, la gemcitabine, le bortézomib, la cyclopamine, l'inhibiteur de PARP PJ34, l'ifosfamide ou un anticorps anti-VEGF.

Fig. 1

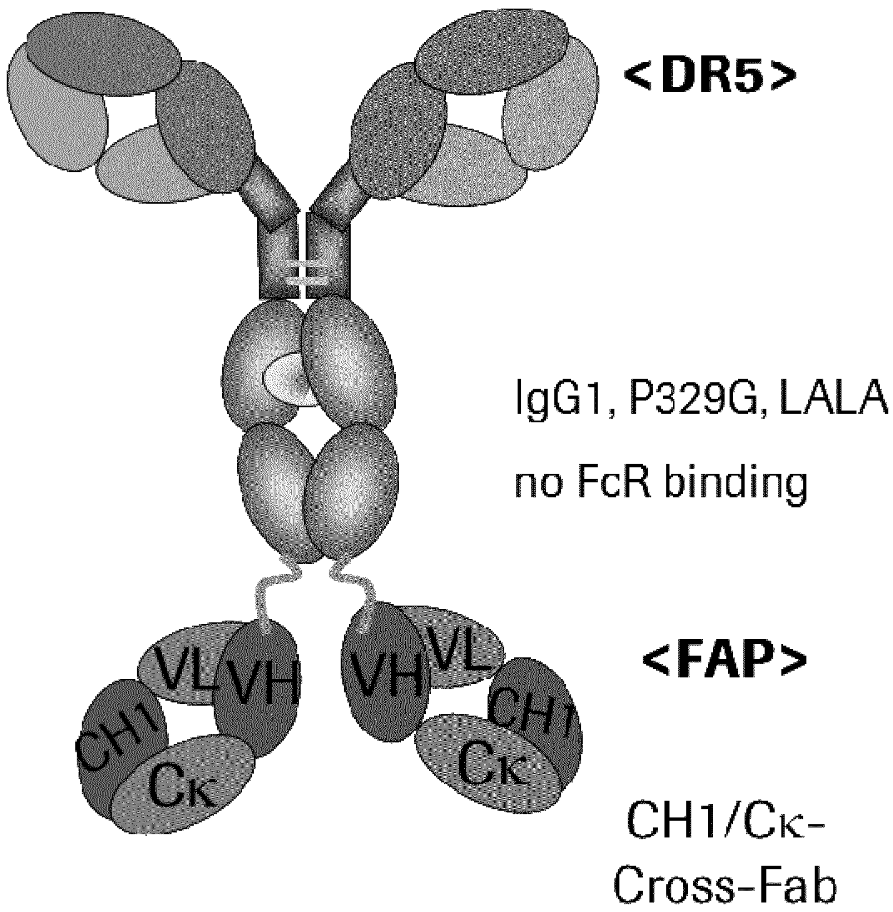
<DR5>
IgG1, P329G, LALA
no FcR binding
VL
VH
CH1
Cκ
VH
VL
CH1
Cκ
<FAP>
CH1/Cκ-
Cross-Fab

Fig. 2

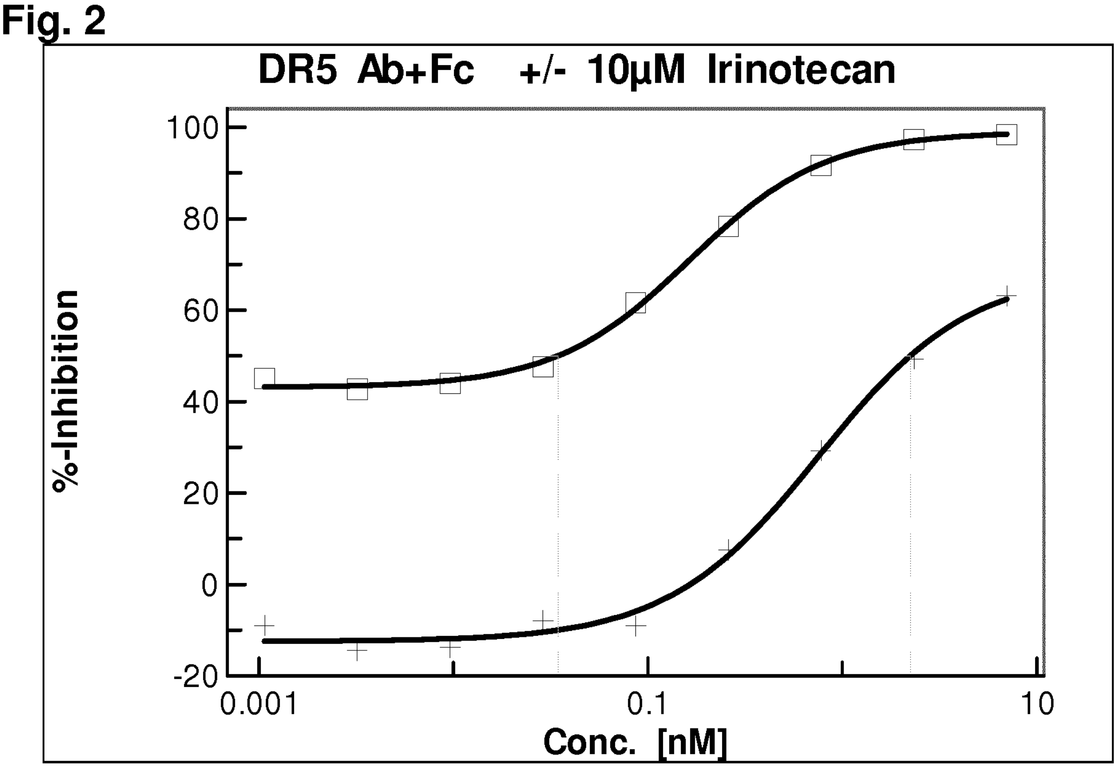


+ DR5 Ab+Fc mono

☐ Combo

Fig. 3

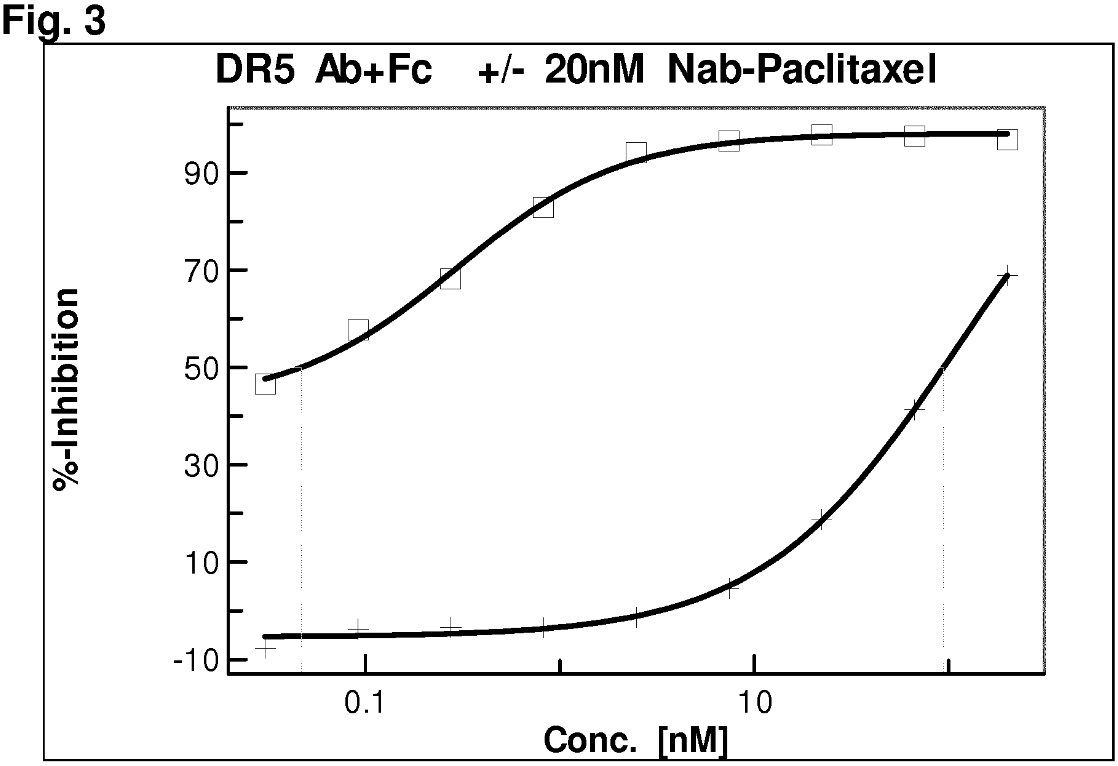


+ DR5 Ab+Fc mono

☐ Combo

Fig. 4

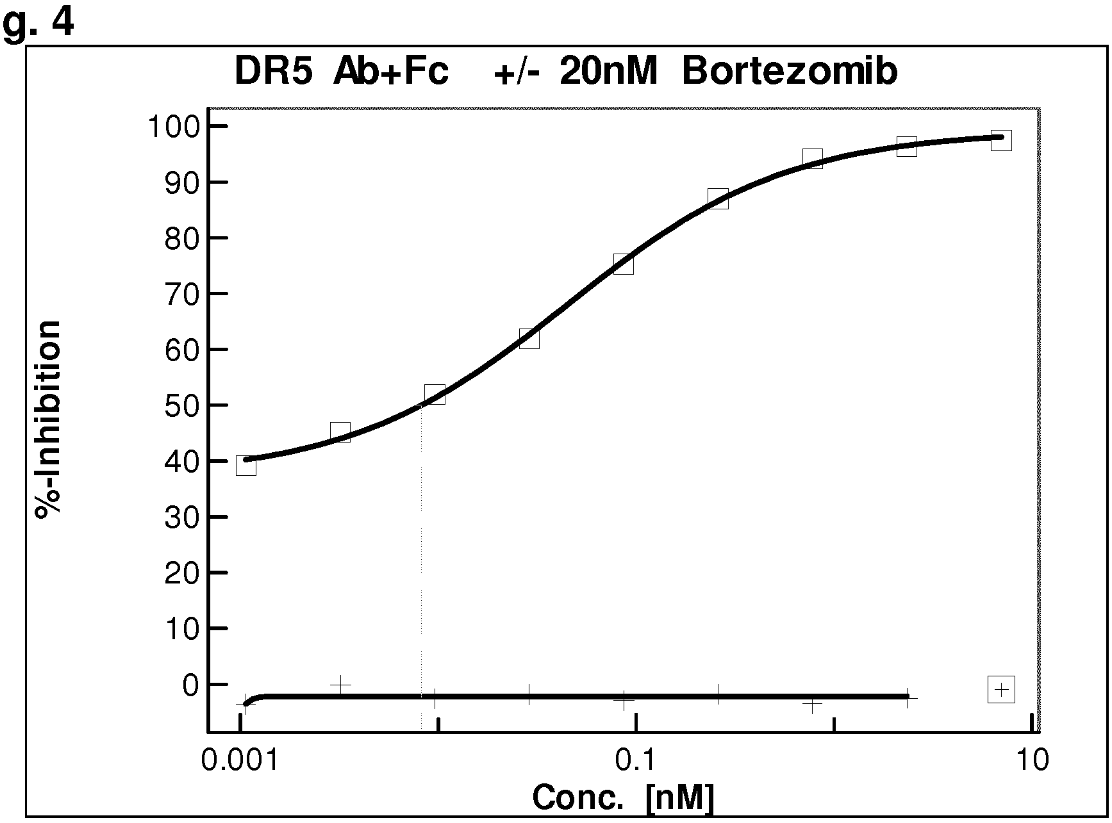


+ DR5 Ab+Fc mono
□ Combo

Fig. 5

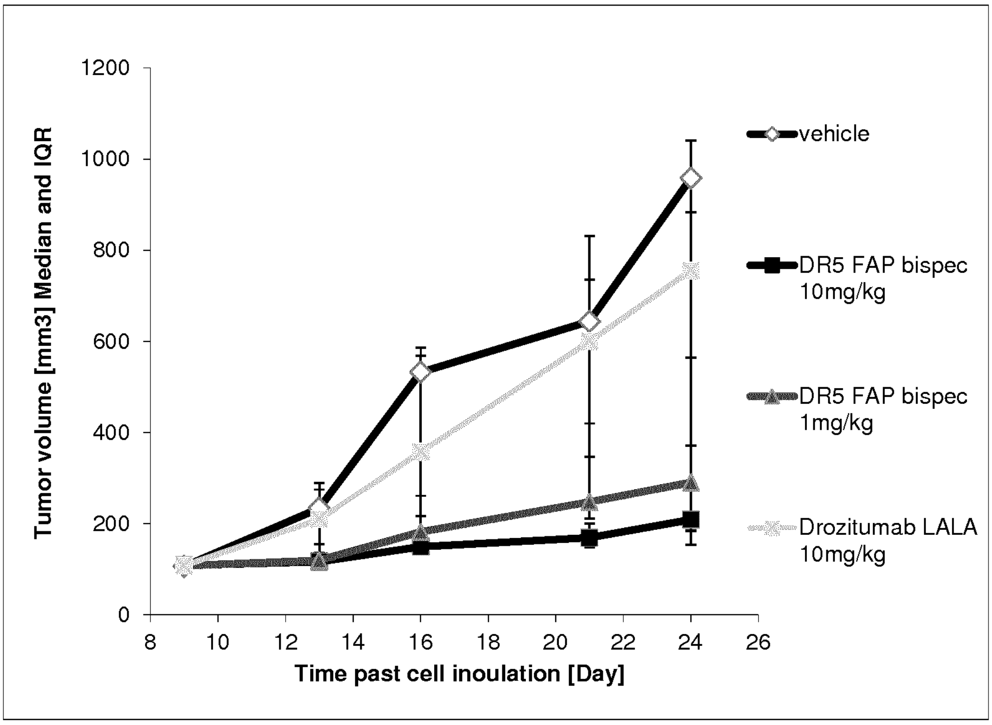
1200
1000
800
600
400
200
0
Tumor volume [mm3] Median and IQR
8
10
12
14
16
18
20
22
24
26
Time past cell inoulation [Day]
vehicle
DR5 FAP bispec 10mg/kg
DR5 FAP bispec 1mg/kg
Drozitumab LALA 10mg/kg

Fig. 6

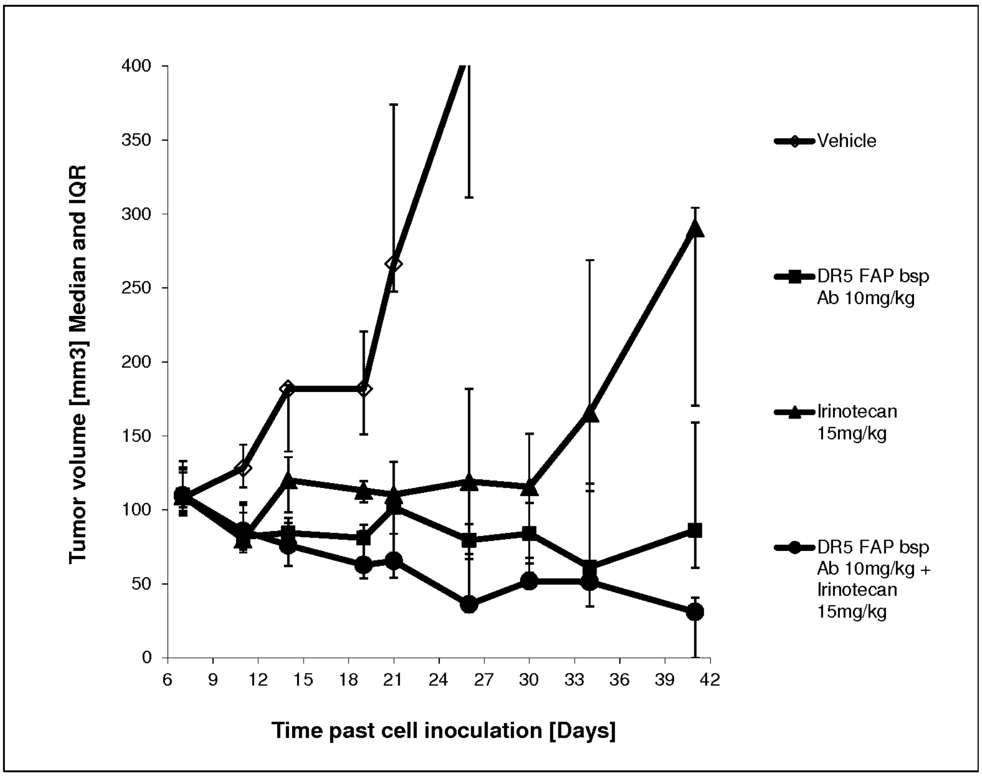
400
350
300
250
200
150
100
50
0
Tumor volume [mm3] Median and IQR
6
9
12
15
18
21
24
27
30
33
36
39
42
Time past cell inoculation [Days]
Vehicle
DR5 FAP bsp Ab 10mg/kg
Irinotecan 15mg/kg
DR5 FAP bsp Ab 10mg/kg + Irinotecan 15mg/kg

Fig. 7

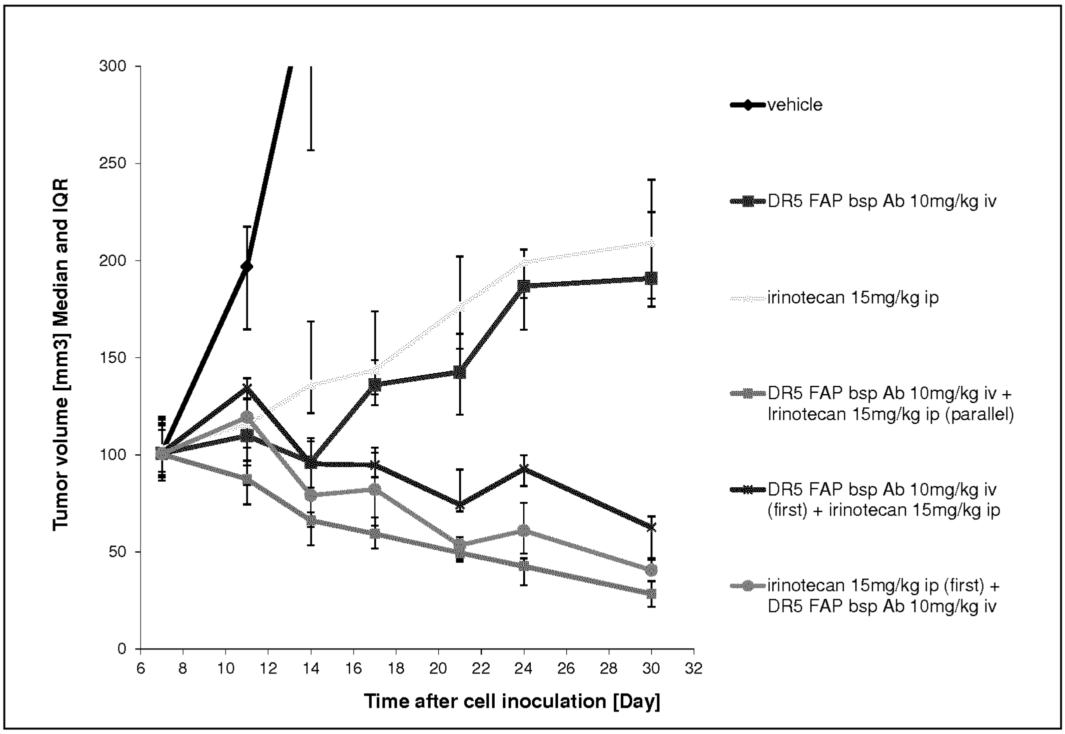
Tumor volume [mm3] Median and IQR
300
250
200
150
100
50
0
6
8
10
12
14
16
18
20
22
24
26
28
30
32
Time after cell inoculation [Day]
vehicle
DR5 FAP bsp Ab 10mg/kg iv
irinotecan 15mg/kg ip
DR5 FAP bsp Ab 10mg/kg iv + Irinotecan 15mg/kg ip (parallel)
DR5 FAP bsp Ab 10mg/kg iv (first) + irinotecan 15mg/kg ip
irinotecan 15mg/kg ip (first) + DR5 FAP bsp Ab 10mg/kg iv

Fig. 8

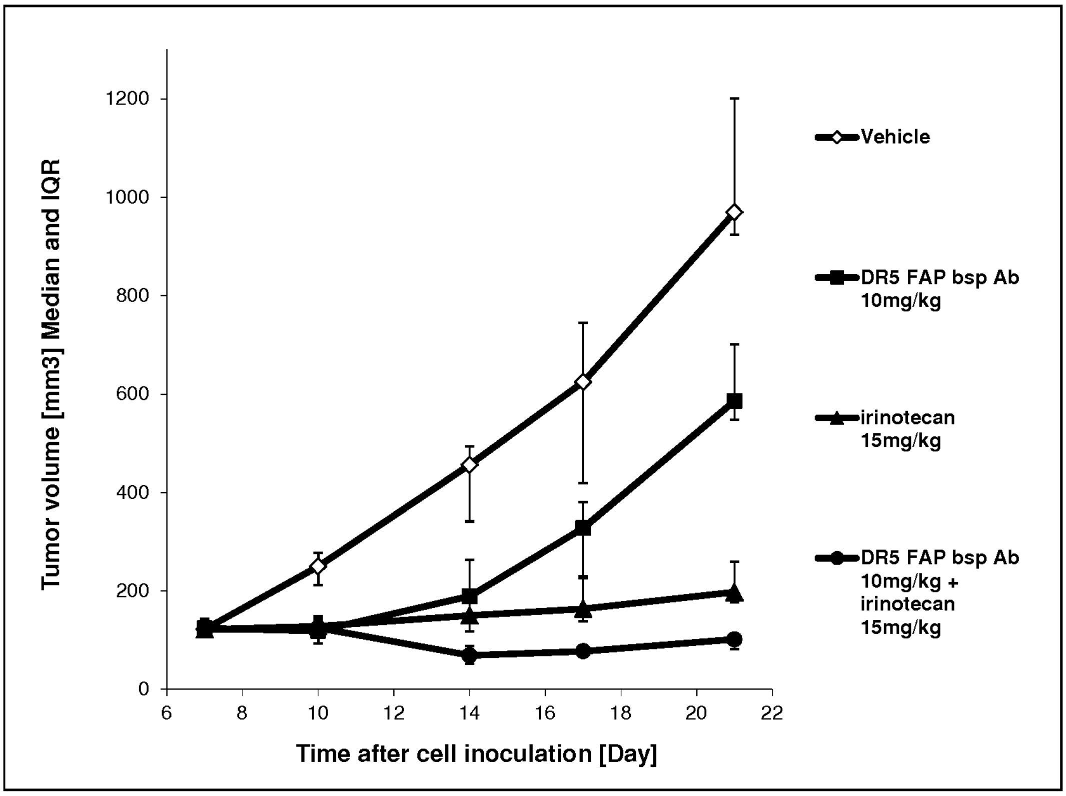
Tumor volume [mm3] Median and IQR
1200
1000
800
600
400
200
0
6
8
10
12
14
16
18
20
22
Time after cell inoculation [Day]
Vehicle
DR5 FAP bsp Ab 10mg/kg
irinotecan 15mg/kg
DR5 FAP bsp Ab 10mg/kg + irinotecan 15mg/kg

Fig. 9

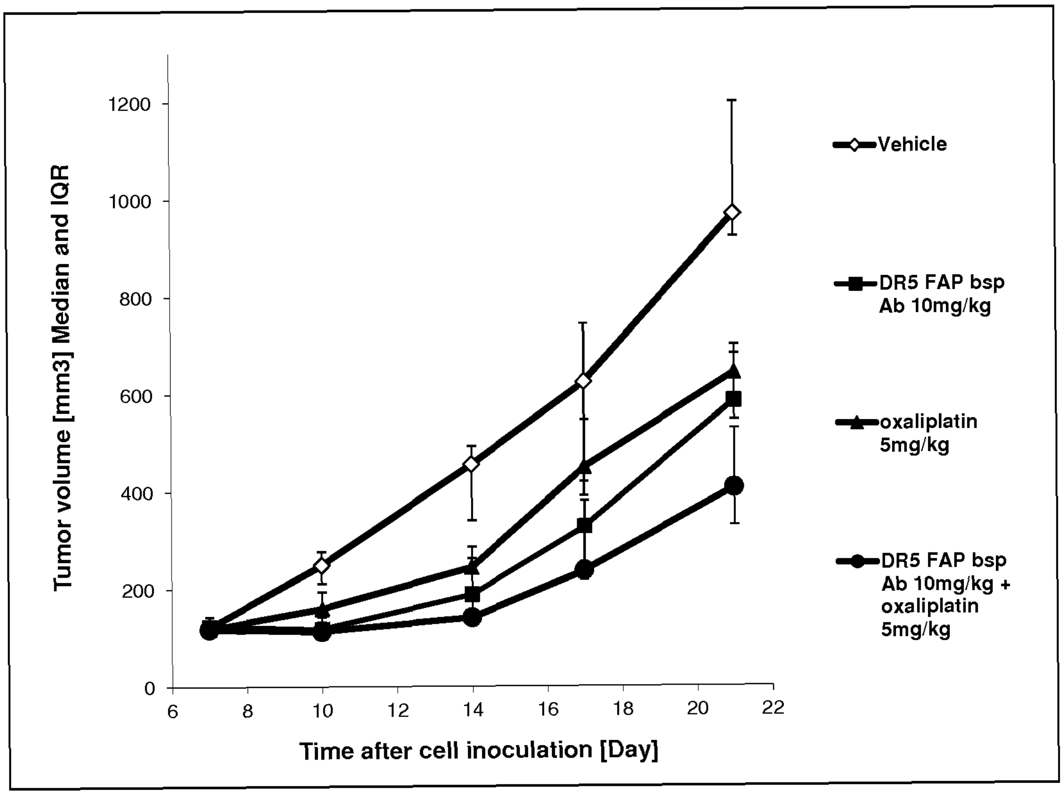
Tumor volume [mm3] Median and IQR
1200
1000
800
600
400
200
0
6
8
10
12
14
16
18
20
22
Time after cell inoculation [Day]
Vehicle
DR5 FAP bsp Ab 10mg/kg
oxaliplatin 5mg/kg
DR5 FAP bsp Ab 10mg/kg + oxaliplatin 5mg/kg

Fig. 10

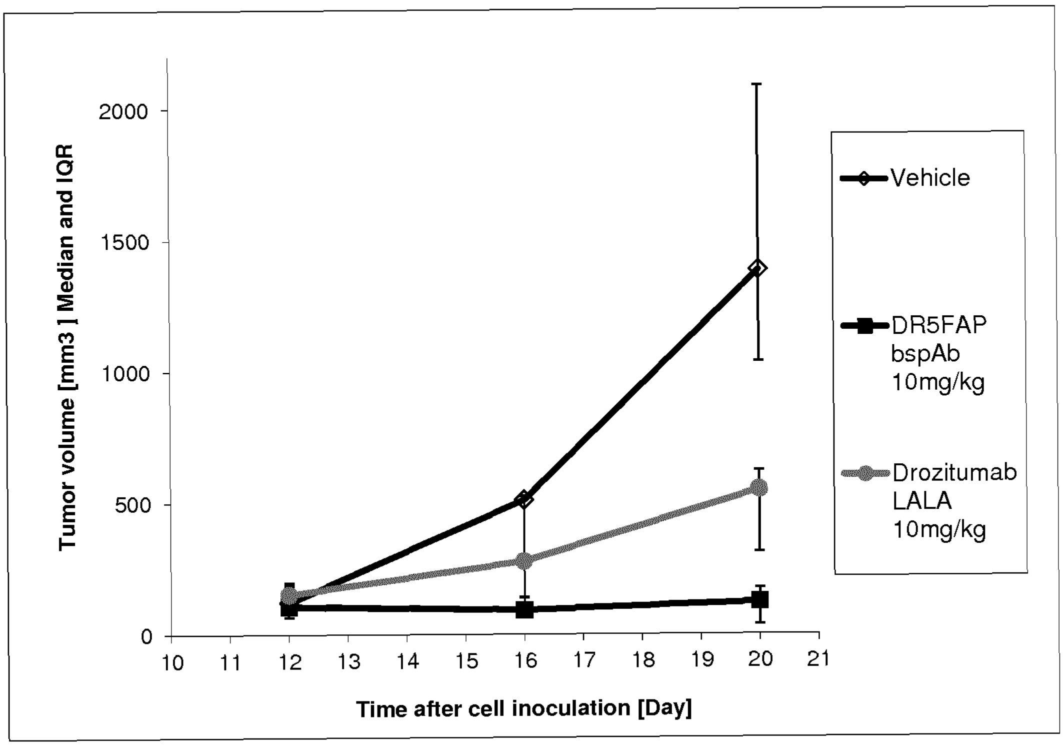
Tumor volume [mm3 ] Median and IQR
2000
1500
1000
500
0
10
11
12
13
14
15
16
17
18
19
20
21
Time after cell inoculation [Day]
Vehicle
DR5FAP bspAb 10mg/kg
Drozitumab LALA 10mg/kg

Fig. 11

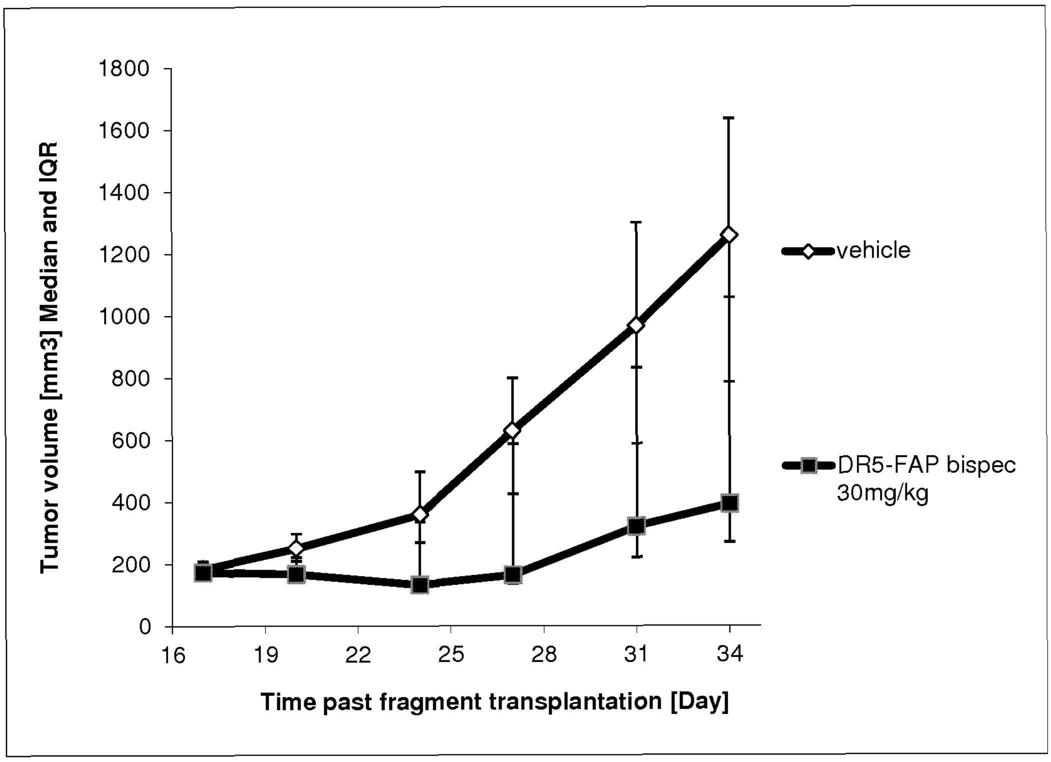
1800
1600
1400
1200
1000
800
600
400
200
0
Tumor volume [mm3] Median and IQR
16
19
22
25
28
31
34
Time past fragment transplantation [Day]
vehicle
DR5-FAP bispec 30mg/kg

Fig. 12

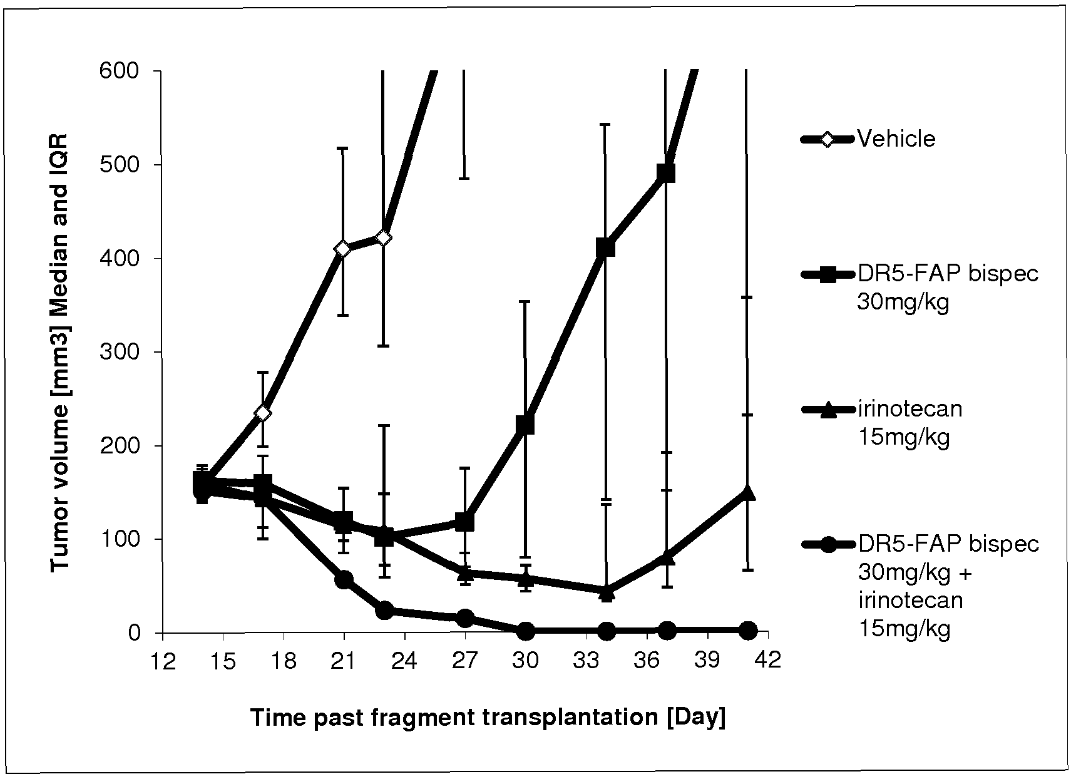
600
500
400
300
200
100
0
Tumor volume [mm3] Median and IQR
12
15
18
21
24
27
30
33
36
39
42
Time past fragment transplantation [Day]
Vehicle
DR5-FAP bispec 30mg/kg
irinotecan 15mg/kg
DR5-FAP bispec 30mg/kg + irinotecan 15mg/kg

Fig. 13

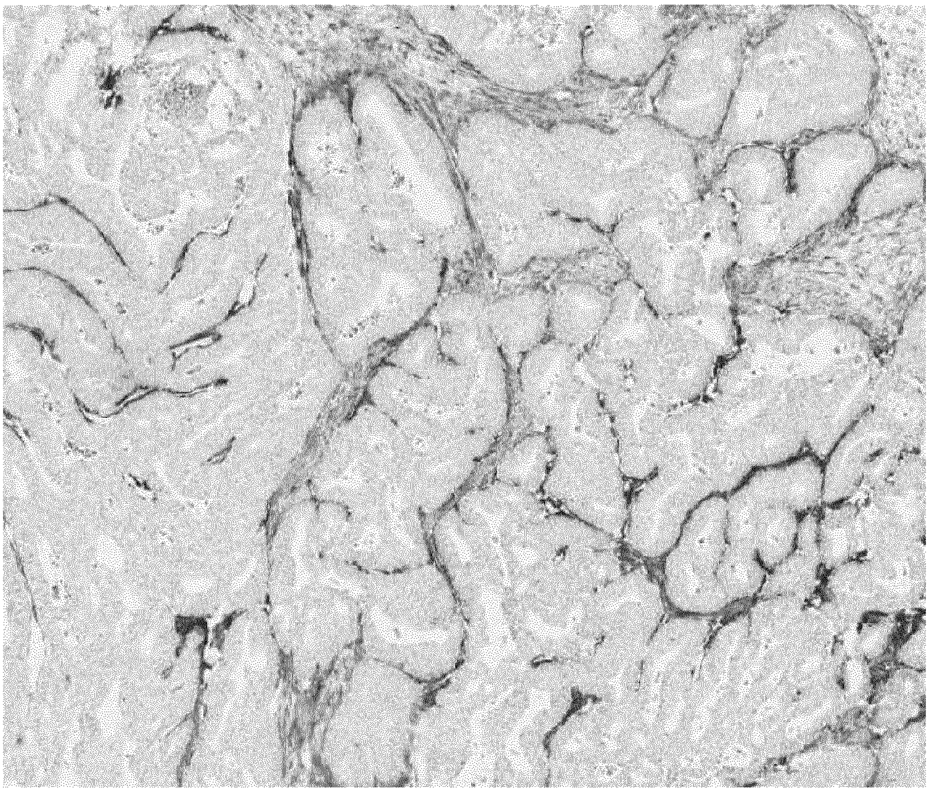

FAP IHC Co5896

Fig. 14

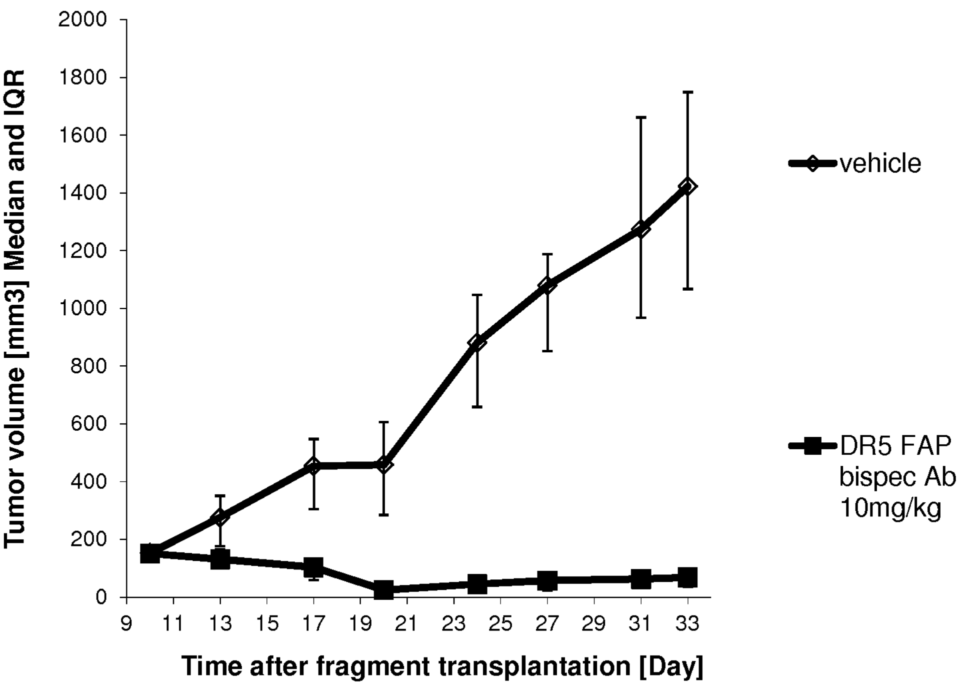

# Fig. 15

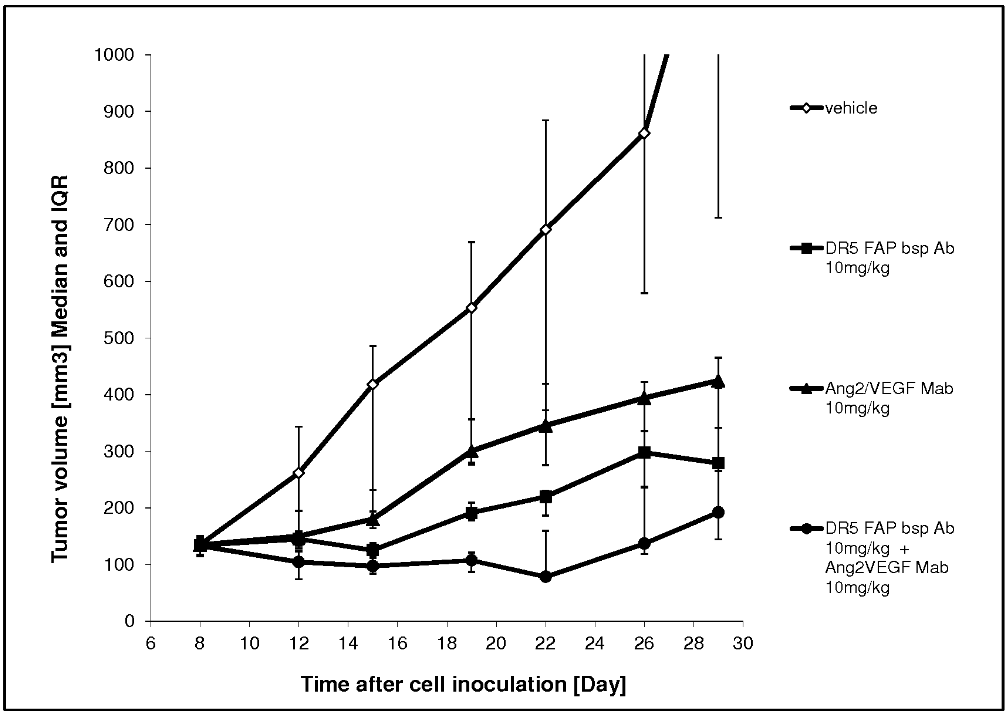

Tumor volume [mm3] Median and IQR
Time after cell inoculation [Day]
vehicle
DR5 FAP bsp Ab 10mg/kg
Ang2/VEGF Mab 10mg/kg
DR5 FAP bsp Ab 10mg/kg + Ang2VEGF Mab 10mg/kg


# Fig. 16

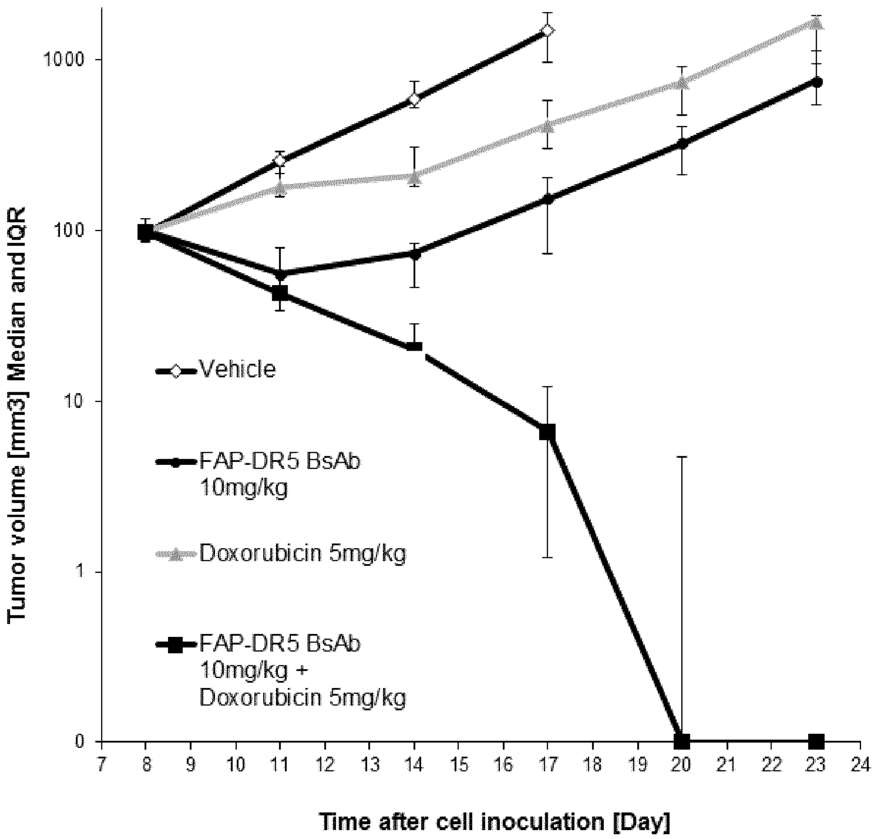

Tumor volume [mm3] Median and IQR
Time after cell inoculation [Day]
Vehicle
FAP-DR5 BsAb 10mg/kg
Doxorubicin 5mg/kg
FAP-DR5 BsAb 10mg/kg + Doxorubicin 5mg/kg

Fig. 17

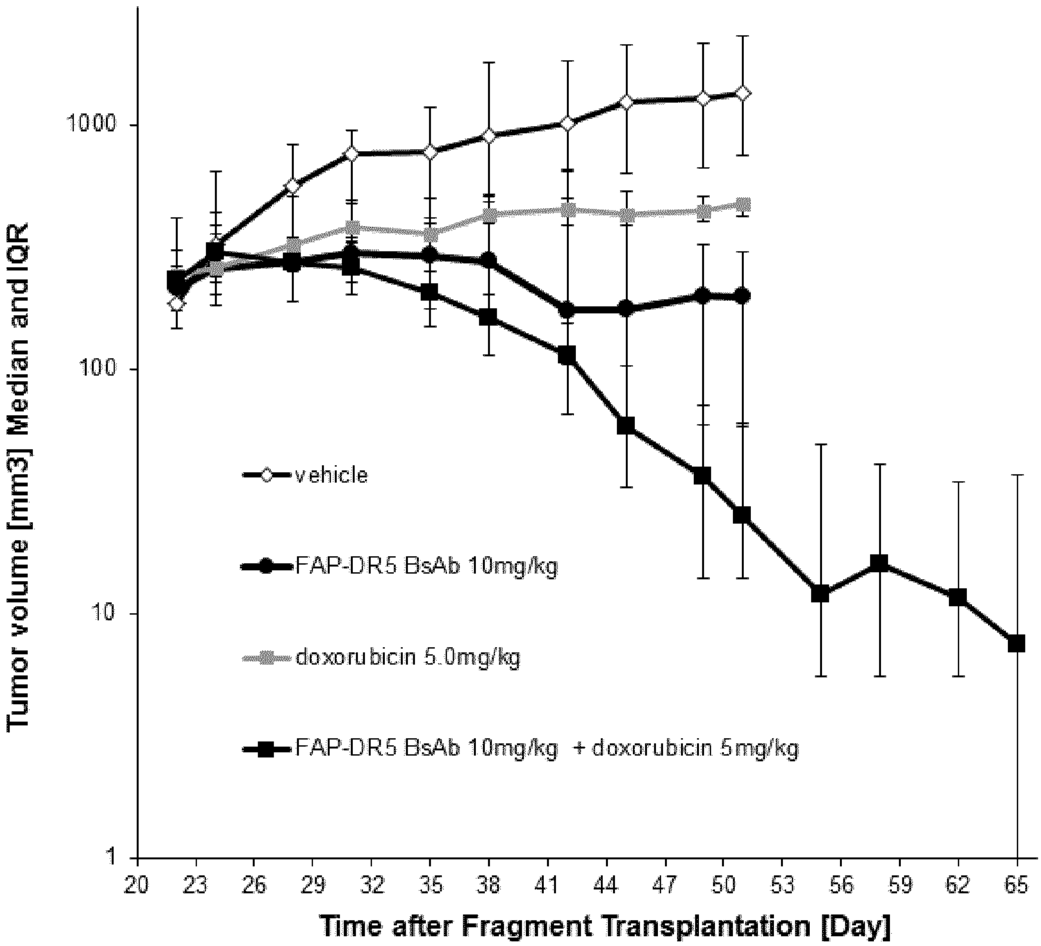
Tumor volume [mm3] Median and IQR
1000
100
10
1
20
23
26
29
32
35
38
41
44
47
50
53
56
59
62
65
Time after Fragment Transplantation [Day]
vehicle
FAP-DR5 BsAb 10mg/kg
doxorubicin 5.0mg/kg
FAP-DR5 BsAb 10mg/kg + doxorubicin 5mg/kg

Fig. 18

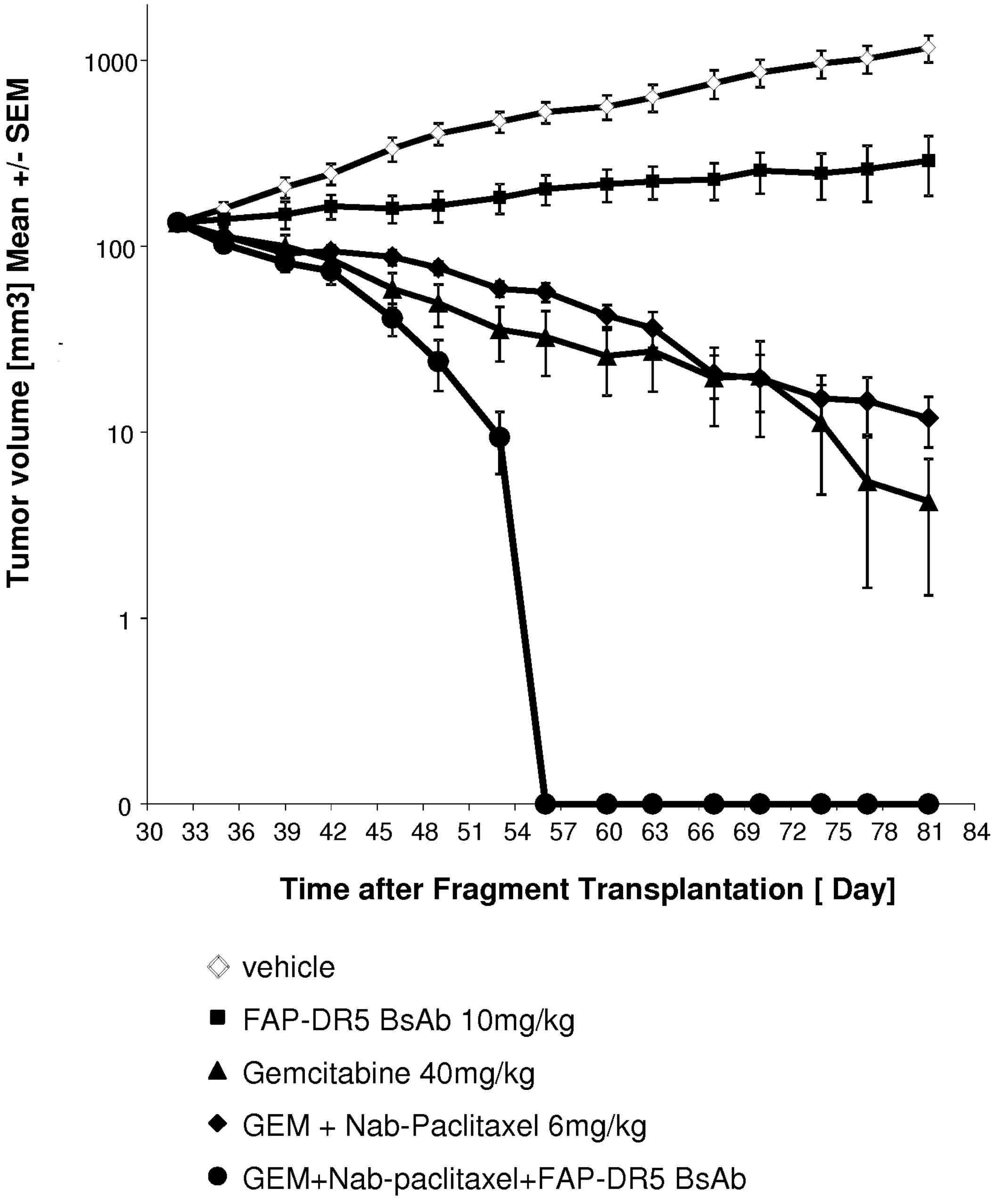
Tumor volume [mm3] Mean +/- SEM
1000
100
10
1
0
30 33 36 39 42 45 48 51 54 57 60 63 66 69 72 75 78 81 84
Time after Fragment Transplantation [ Day]
vehicle
FAP-DR5 BsAb 10mg/kg
Gemcitabine 40mg/kg
GEM + Nab-Paclitaxel 6mg/kg
GEM+Nab-paclitaxel+FAP-DR5 BsAb

**Fig. 19**

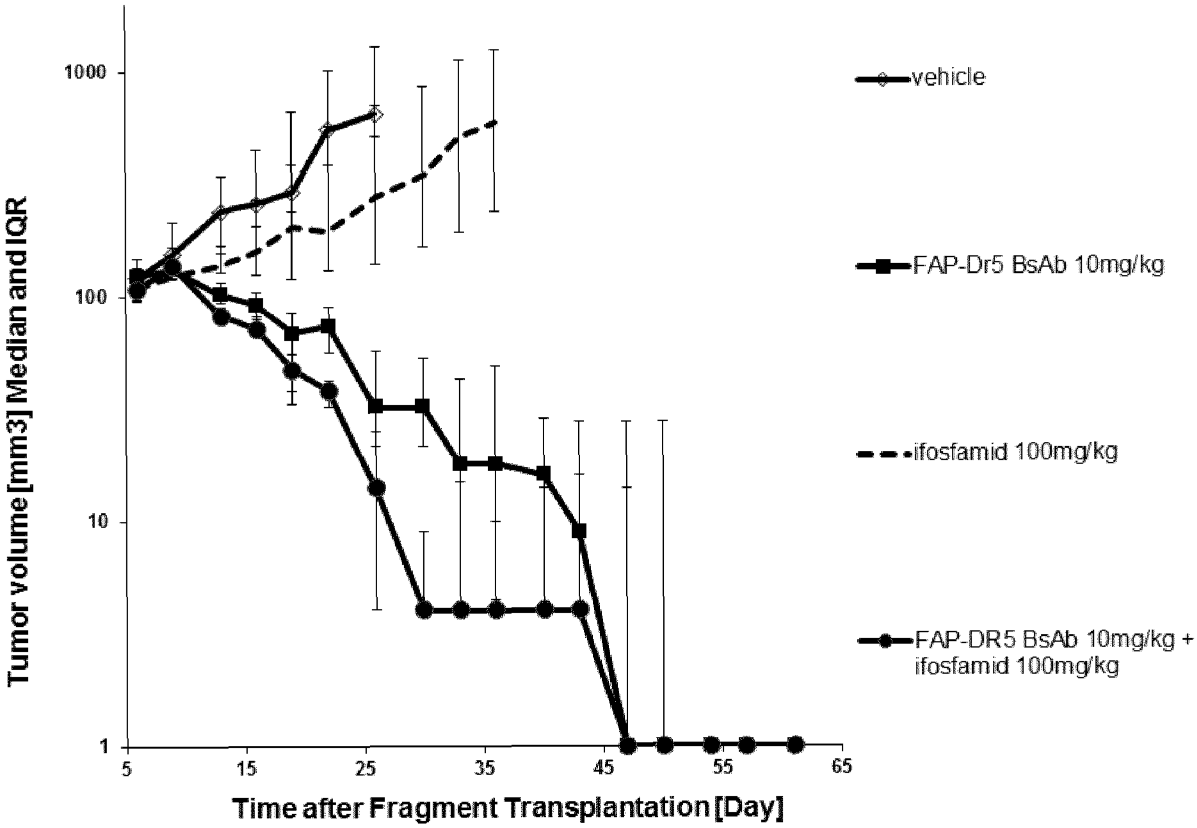
1000
100
10
1
Tumor volume [mm3] Median and IQR
5
15
25
35
45
55
65
Time after Fragment Transplantation [Day]
vehicle
FAP-Dr5 BsAb 10mg/kg
ifosfamid 100mg/kg
FAP-DR5 BsAb 10mg/kg + ifosfamid 100mg/kg

# Fig. 20

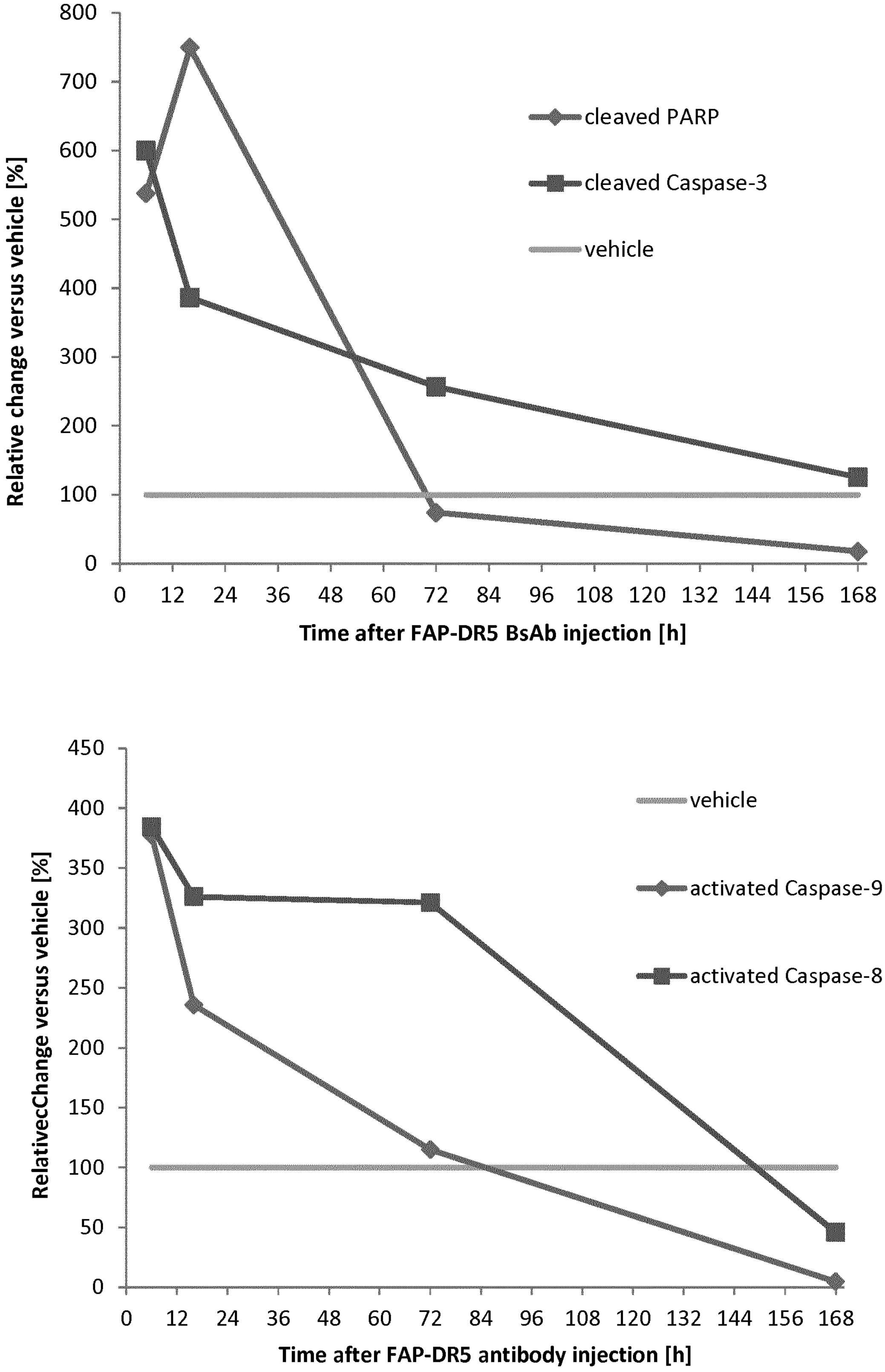
800
700
600
500
400
300
200
100
0
Relative change versus vehicle [%]
cleaved PARP
cleaved Caspase-3
vehicle
0 12 24 36 48 60 72 84 96 108 120 132 144 156 168
Time after FAP-DR5 BsAb injection [h]
450
400
350
300
250
200
150
100
50
0
RelativecChange versus vehicle [%]
vehicle
activated Caspase-9
activated Caspase-8
0 12 24 36 48 60 72 84 96 108 120 132 144 156 168
Time after FAP-DR5 antibody injection [h]

Fig. 21 a

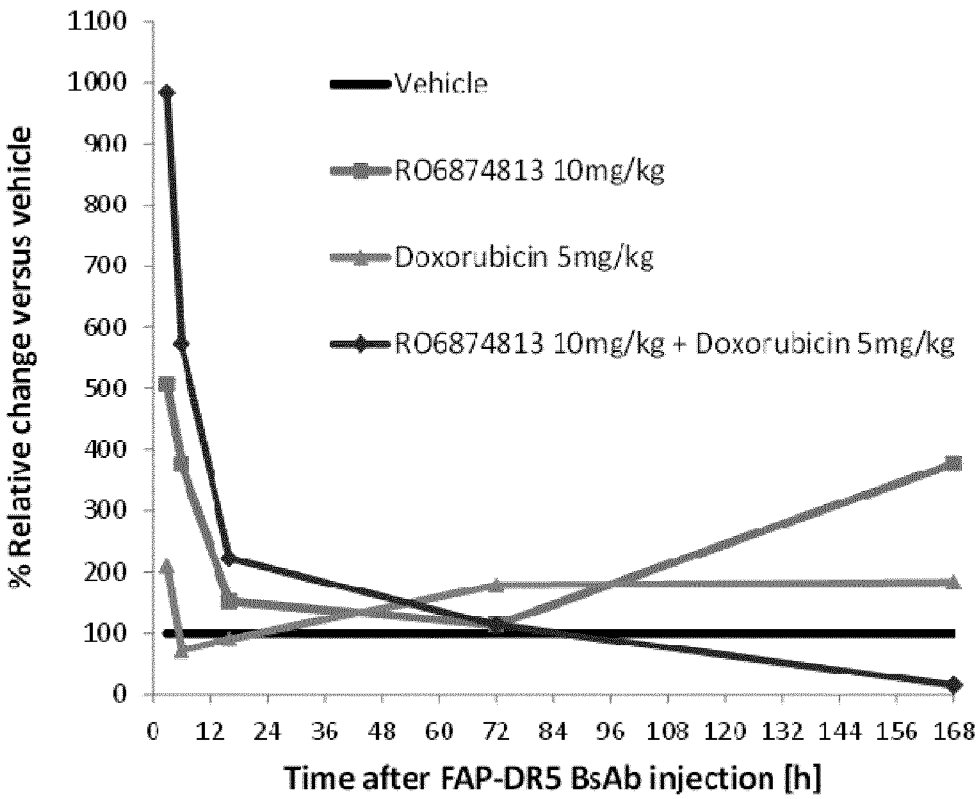

1100
1000
900
800
700
600
500
400
300
200
100
0
% Relative change versus vehicle
Vehicle
RO6874813 10mg/kg
Doxorubicin 5mg/kg
RO6874813 10mg/kg + Doxorubicin 5mg/kg
0 12 24 36 48 60 72 84 96 108 120 132 144 156 168
Time after FAP-DR5 BsAb injection [h]

Fig. 21 b

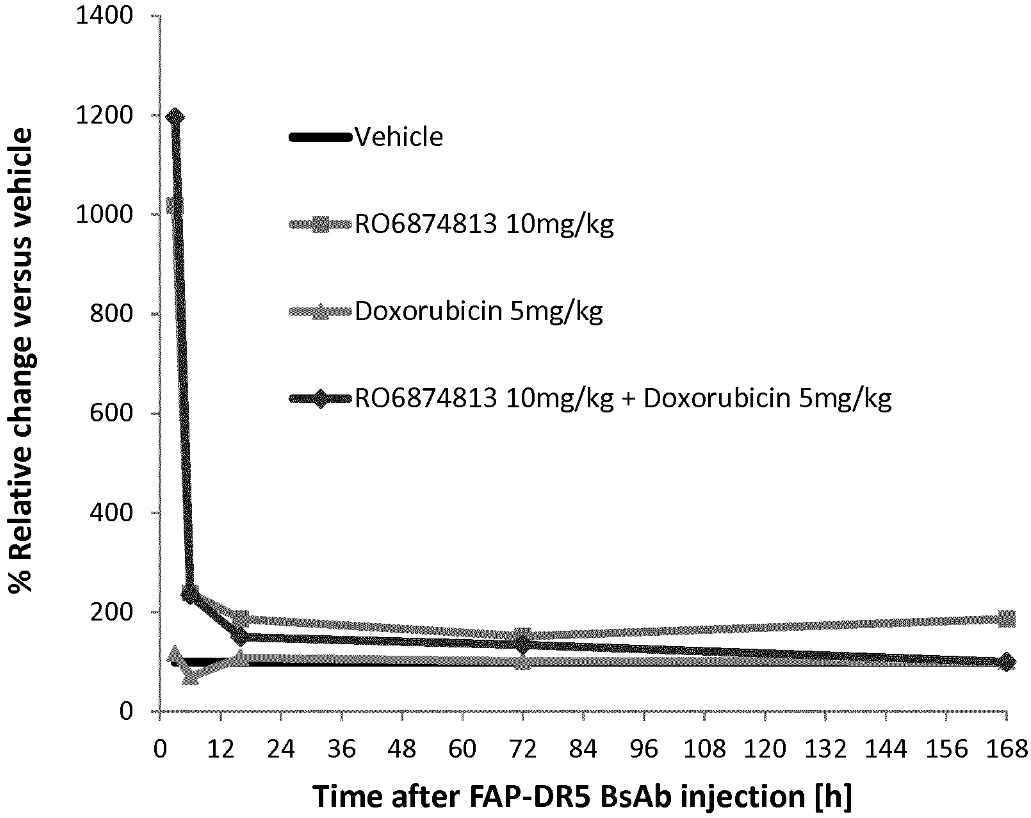

% Relative change versus vehicle
1400
1200
1000
800
600
400
200
0
Vehicle
RO6874813 10mg/kg
Doxorubicin 5mg/kg
RO6874813 10mg/kg + Doxorubicin 5mg/kg
0 12 24 36 48 60 72 84 96 108 120 132 144 156 168
Time after FAP-DR5 BsAb injection [h]

Fig. 21 c

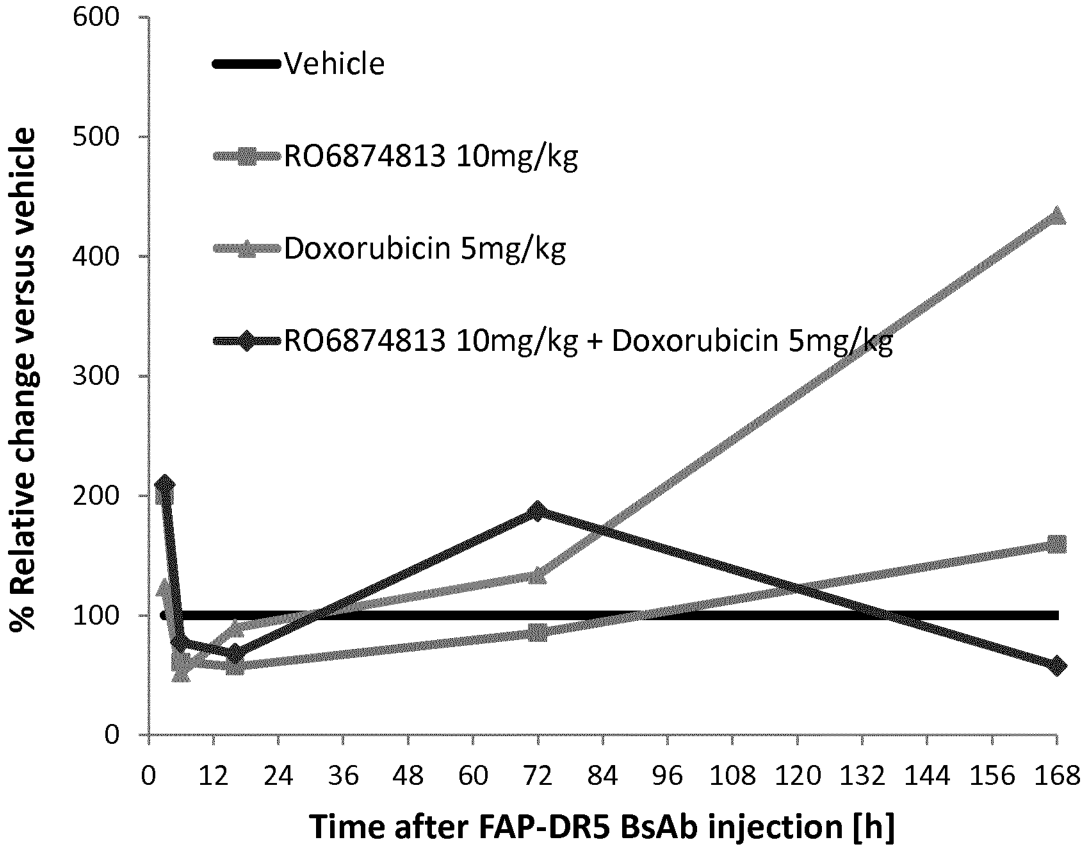
600
500
400
300
200
100
0
Vehicle
RO6874813 10mg/kg
Doxorubicin 5mg/kg
RO6874813 10mg/kg + Doxorubicin 5mg/kg
% Relative change versus vehicle
0 12 24 36 48 60 72 84 96 108 120 132 144 156 168
Time after FAP-DR5 BsAb injection [h]

Fig. 22 a

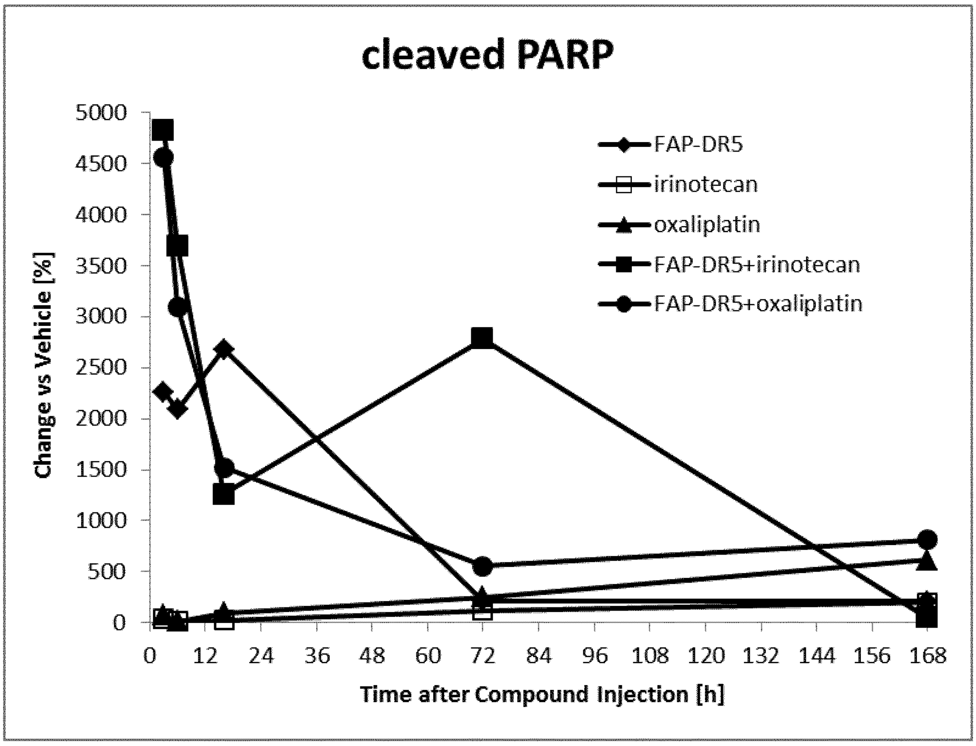


Fig. 22 b

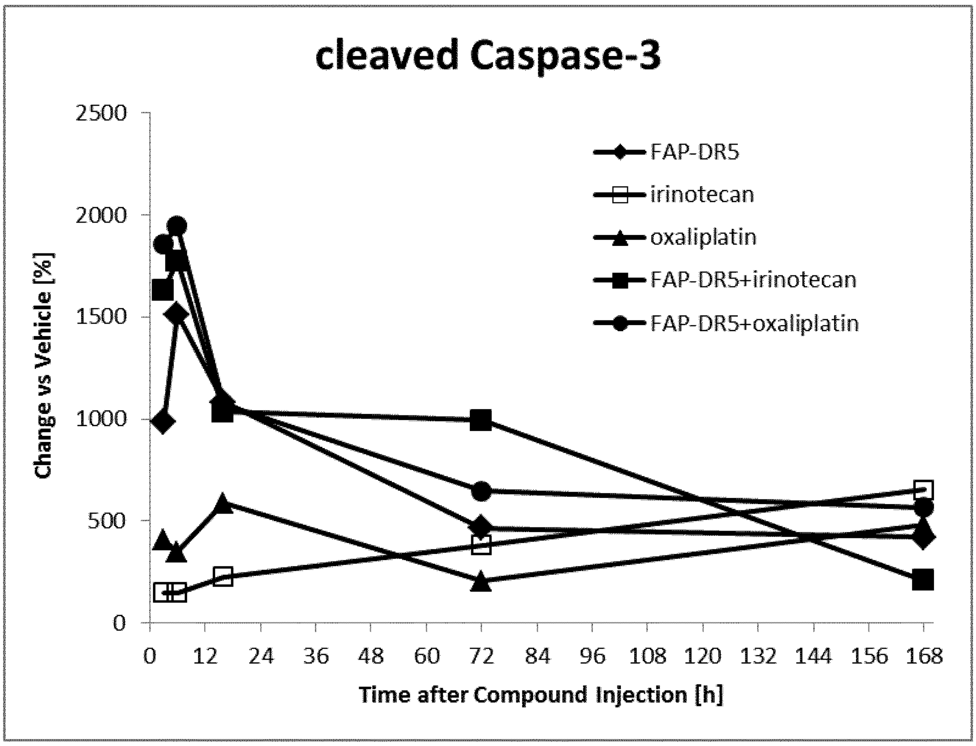

Fig. 22 c

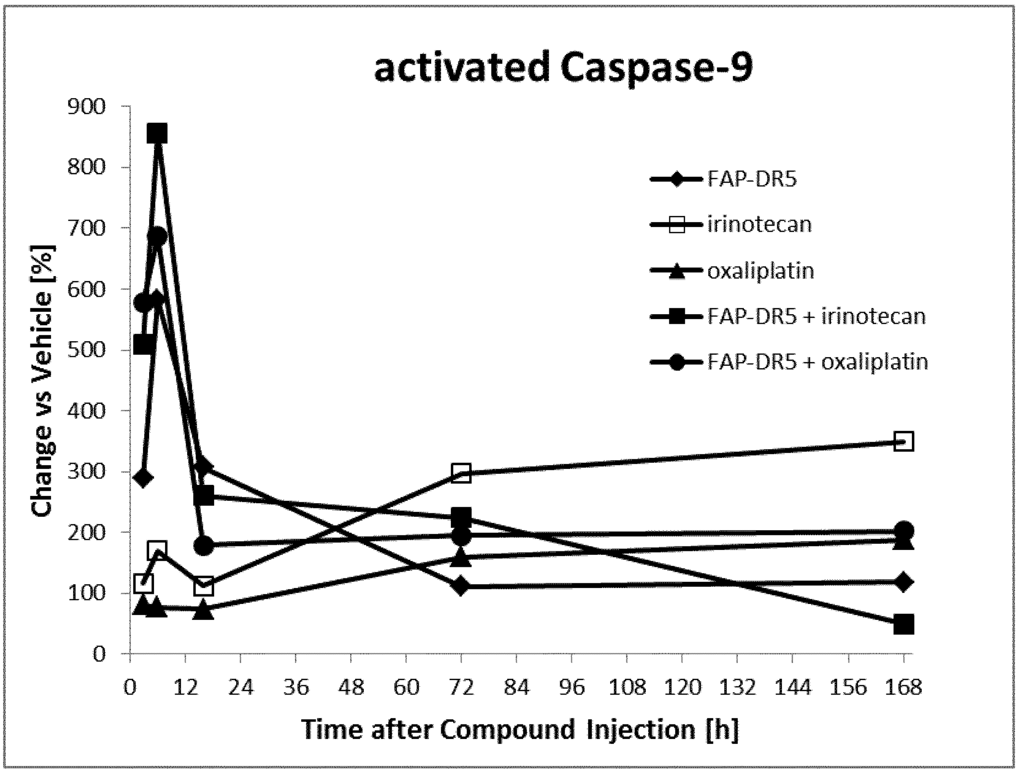


Fig. 22 d

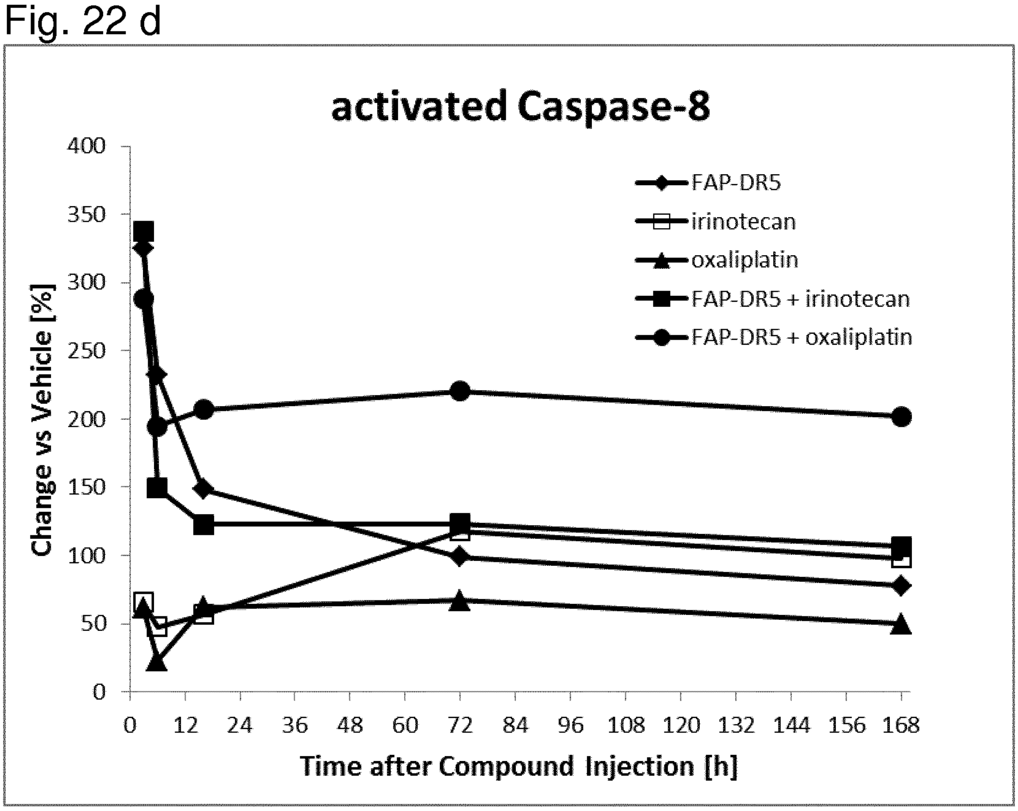

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070031414 A
- WO 2006083971 A
- EP 1922337 A
- WO 2011098520 A
- WO 2011039126 A
- WO 9305804 A
- WO 2009080252 A
- WO 2009080253 A
- WO 2009080251 A
- WO 2009080254 A
- WO 2010136172 A
- WO 2010145792 A
- WO 2013026831 A
- US 5202238 A
- US 5204244 A
- WO 2012020006 A
- WO 9308829 A
- US 5731168 A
- WO 2009089004 A
- US 4676980 A
- US 20060025576 A1
- US 20080069820 A
- WO 2010040508 A
- WO 2011117329 A
- WO 2012130831 A
- US 6737056 B
- US 7332581 B
- US 5500362 A
- US 5821337 A
- WO 2006029879 A
- WO 2005100402 A
- US 7695936 B
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1, Adams
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S
- US 7521541 B
- US 5959177 A
- US 6040498 A
- US 6420548 B
- US 7125978 B
- US 6417429 B
- US 4186567 A
- US 5969108 A, McCafferty
- US 5565332 A, Winter
- US 7527791 B
- US 6982321 B
- US 7087409 B
- US 20040132066 A
- WO 2004065540 A
- US 20050260186 A
- US 20060104968 A
- US 6267958 B
- US 6171586 B
- WO 2006044908 A


### Non-patent literature cited in the description


- **NIEDERMEYER et al.** *Int J Cancer,* 1997, vol. 71, 383-389
- **NIEDERMEYER et al.** *Eur J Biochem,* 1998, vol. 254, 650-654
- **RETTIG et al.** *Proc Natl Acad Sci USA,* 1988, vol. 85, 3110-3114
- **GARIN-CHESA et al.** *Proc Natl Acad Sci USA,* 1990, vol. 87, 7235-7239
- **JIN et al.** *Anticancer Res,* 2003, vol. 23, 3195-3198
- **LI ; RAVETCH.** *PNAS,* 2012
- **WILSON.** *Cancer Cell,* 2011

- **HOUSTON, J.S.** *Methods in Enzymol.,* 1991, vol. 203, 46-96
- **MORRISON, S.L. et al.** *Proc. Natl. Acad. Sci. USA,* 1984, vol. 81, 6851-6855
- **KABAT et al.** Sequences of Proteins of Immunological Interest. National Institutes of Health, 1991
- **KABAT et al.** Sequences of Proteins of Immunological Interest. NIH Publication, 1991, vol. 1-3
- **CHOTHIA ; LESK.** *J. Mol. Biol.,* 1987, vol. 196, 901-917
- **KABAT et al.** Sequences of Proteins of Immunological Interest. U.S. Dept. of Health and Human Services, 1983
- **CHOTHIA et al.** *J. Mol. Biol.,* 1987, vol. 196, 901-917
- **KABAT et al.** Sequence of Proteins of Immunological Interest. U.S. Dept. of Health and Human Services, 1983
- **ALMAGRO ; FRANSSON.** *Front. Biosci.,* 2008, vol. 13, 1619-1633
- **FLATMAN et al.** *J. Chromatogr. B,* 2007, vol. 848, 79-87
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **MILSTEIN ; CUELLO.** *Nature,* 1983, vol. 305, 537
- **TRAUNECKER et al.** *EMBO J.,* 1991, vol. 10, 3655
- **BRENNAN et al.** *Science,* 1985, vol. 229, 81
- **KOSTELNY et al.** *J. Immunol.,* 1992, vol. 148 (5), 1547-1553
- **HOLLINGER et al.** *Proc. Natl. Acad. Sci. USA,* 1993, vol. 90, 6444-6448
- **GRUBER et al.** *J. Immunol.,* 1994, vol. 152, 5368
- **TUTT et al.** *J. Immunol.,* 1991, vol. 147, 60
- **DING et al.** *J. Med. Chem.,* 2013, vol. 56 (14), 5979-5983
- Mutant p53 and MDM2 in Cancer. **SWATU PALIT DEB ; SUMITRA DEB.** Subcellular Biochemistry. Springer, 85
- **SOUERS et al.** *Nat. Med.,* 2013, vol. 19 (2), 202-208
- **MADISON et al.** *DNA Repair,* 10 October 2011, vol. 10 (10), 1003-13
- **SCHAEFER et al.** *Proc Natl Acad Sci USA,* 2011, vol. 108, 11187-92
- **STUBENRAUCH et al.** *Drug Metabolism and Disposition,* 2010, vol. 38, 84-91
- **HELLSTROM et al.** *Proc Natl Acad Sci USA,* 1986, vol. 83, 7059-7063
- **HELLSTROM et al.** *Proc Natl Acad Sci USA,* 1985, vol. 82, 1499-1502
- **BRUGGEMANN et al.** *J Exp Med,* 1987, vol. 166, 1351-1361
- **CLYNES et al.** *Proc Natl Acad Sci USA,* 1998, vol. 95, 652-656
- **SANTORO et al.** *J Immunol Methods,* 1996, vol. 202, 163
- **CRAGG et al.** *Blood,* 2003, vol. 101, 1045-1052
- **CRAGG ; GLENNIE.** *Blood,* 2004, vol. 103, 2738-2743
- **RIDGWAY et al.** *Prot Eng,* 1996, vol. 9, 617-621
- **CARTER.** *J Immunol Meth,* 2001, vol. 248, 7-15
- **CARTER.** *J Immunol Methods,* 2001, vol. 248, 7-15
- **CARTER P. ; RIDGWAY J.B.B. ; PRESTA L.G.** *Immunotechnology,* February 1996, vol. 2 (1), 73-73
- **CHOWDHURY.** *Methods Mol. Biol.,* 2008, vol. 207, 179-196
- **HOOGENBOOM et al.** Methods in Molecular Biology. Human Press, 2001, vol. 178, 1-37
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **MANIATIS et al.** MOLECULAR CLONING: A LABORATORY MANUAL. Cold Spring Harbor Laboratory, 1989
- **AUSUBEL et al.** CURRENT PROTOCOLS IN MOLECULAR BIOLOGY. Greene Publishing Associates, 1989
- **GERNGROSS.** *Nat Biotech,* 2004, vol. 22, 1409-1414
- **LI et al.** *Nat Biotech,* 2006, vol. 24, 210-215
- **GRAHAM et al.** *J Gen Virol,* 1977, vol. 36, 59
- **MATHER.** *Biol Reprod,* 1980, vol. 23, 243-251
- **MATHER et al.** *Annals N.Y. Acad Sci,* 1982, vol. 383, 44-68
- **URLAUB et al.** *Proc Natl Acad Sci USA,* 1980, vol. 77, 4216
- **YAZAKI ; WU.** Methods in Molecular Biology. Humana Press, 2003, vol. 248, 255-268
- **HARLOW ; LANE.** Antibodies, a laboratory manual. Cold Spring Harbor Laboratory, 1988
- **ALMAGRO ; FRANSSON.** *Front Biosci,* 2008, vol. 13, 1619-1633
- **RIECHMANN et al.** *Nature,* 1988, vol. 332, 323-329

- **QUEEN et al.** *Proc Natl Acad Sci USA,* 1989, vol. 86, 10029-10033
- **JONES et al.** *Nature,* 1986, vol. 321, 522-525
- **MORRISON et al.** *Proc Natl Acad Sci,* 1984, vol. 81, 6851-6855
- **MORRISON ; OI.** *Adv Immunol,* 1988, vol. 44, 65-92
- **VERHOEYEN et al.** *Science,* 1988, vol. 239, 1534-1536
- **PADLAN.** *Molec Immun,* 1994, vol. 31 (3), 169-217
- **KASHMIRI et al.** *Methods,* 2005, vol. 36, 25-34
- **PADLAN.** *Mol Immunol,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br J Cancer,* 2000, vol. 83, 252-260
- **VAN DIJK ; VAN DE WINKEL.** *Curr Opin Pharmacol,* 2001, vol. 5, 368-74
- **LONBERG.** *Curr Opin Immunol,* 2008, vol. 20, 450-459
- Monoclonal Antibody Production Techniques and Applications. Marcel Dekker, Inc, 1987, 51-63
- **LONBERG.** *Nat Biotech,* 2005, vol. 23, 1117-1125
- **MCCAFFERTY et al.** *Nature,* vol. 348, 552-554
- **CLACKSON et al.** *Nature,* 1991, vol. 352, 624-628
- **LILJEBLAD et al.** *Glyco J,* 2000, vol. 17, 323-329
- **HEELEY.** *Endocr Res,* 2002, vol. 28, 217-229
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- **CHEN et al.** *J Mol Biol,* 1999, vol. 293, 865-881
- Remington's Pharmaceutical Sciences. 1980